(19) Europäisches Patentamt
European Patent Office
Office européen des brevets

(11) Veröffentlichungsnummer: **0 170 926**
**B1**

(12) # EUROPÄISCHE PATENTSCHRIFT

(45) Veröffentlichungstag der Patentschrift: **28.02.90**

(51) Int. Cl.[5]: **C 07 D 461/00, A 61 K 31/475**

(21) Anmeldenummer: **85108623.1**

(22) Anmeldetag: **11.07.85**

(54) **In der 9- oder 11-Stellung eine, gegebenenfalls substituierte, Aminogruppe aufweisende Eburnancarbonsäurederivate, Verfahren zu ihrer Herstellung und diese Verbindungen enthaltende Arzneimittel.**

(30) Priorität: **11.07.84 HU 270384**
**11.07.84 HU 270284**

(43) Veröffentlichungstag der Anmeldung: **12.02.86 Patentblatt 86/07**

(45) Bekanntmachung des Hinweises auf die Patenterteilung: **28.02.90 Patentblatt 90/09**

(84) Bennante Vertragsstaaten:
**AT BE CH DE FR GB IT LI NL SE**

(56) Entgegenhaltungen:
**FR-A-2 320 302**
**FR-A-2 341 585**
**FR-A-2 342 980**
**FR-A-2 442 846**

(73) Patentinhaber: **RICHTER GEDEON VEGYESZETI GYAR R.T.**
**Gyömröi ut 19-21**
**H-1475 Budapest X (HU)**

(72) Erfinder: **Vedres, András, Dr. Dipl.-Chem.**
**Füredi u. 56, H-1144 Budapest (HU)**
Erfinder: **Szántay, Csaba, Dr. Dipl.-Ing.**
**Zsombolyai u. 8, H-1113 Budapest (HU)**
Erfinder: **Moldvai, István**
**Kossuth L. u. 107, H-1212 Budapest (HU)**
Erfinder: **Stefkž, Béla, Dr. Dipl.-Chem.**
**Orlay u. 2/b, H-1117 Budapest (HU)**
Erfinder: **Grož, Džra, Dr.**
**Napraforgž u. 27, H-1021 Budapest (HU)**
Erfinder: **Kárpáti, Egon, Dr.**
**Mihályfi E. u. 7/b, H-1022 Budapest (HU)**
Erfinder: **Kiss, Béla**
**OTP ltp. A/1, H-2220 Vecsés (HU)**
Erfinder: **Lapis, Erzsébet, Dipl.-Ing.**
**Abaliget u. 86, H-1172 Budapest (HU)**
Erfinder: **Laszlovszky, István, Dr.**
**Bartžk B. ut 16, H-1111 Budapest (HU)**
Erfinder: **Pálosi, Eva, Dr.**
**Vend u. 21, H-1025 Budapest (HU)**
Erfinder: **Riesz, Mikližs, Dr.**
**Damjanich u.52, H-1071 Budapest (HU)**
Erfinder: **Szombathelyi, Zsolt, Dr.**
**Munkácsy M. u. 12, H-1063 Budapest (HU)**
Erfinder: **Szporny, Lászlž, Dr.**
**Szabolcska M. u. 7, H-1114 Budapest (HU)**

(74) Vertreter: **Beszédes, Stephan G., Dr. Patentanwalt**
**Münchener Strasse 80a Postfach 1168**
**D-8060 Dachau (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 170 926 B1

LIBERGRAF, STOCKHOLM 1990

**Beschreibung**

Die Erfindung betrifft neue in der 9- oder 11-Stellung eine, gegebenenfalls substituierte, Aminogruppe aufweisende Eburnancarbonsäurederivate, ein Verfahren zur Herstellung derselben und von bekannten Eburnancarbonsäurederivaten und diese neuen Verbindungen und/oder eine bekannte Verbindung enthaltende Arzneimittel, insbesondere mit gefäßerweiternder, spasmolytischer, antihypoxischer und antikonvulsiver Wirkung.

9- und 11-(Amino)-apovincamin, auch 9- beziehungsweise 11-(Amino)-apovincaminsäuremethylester genannt, sind aus der französischen Patentschrift 2 342 980 bekannt, wobei auch ihre den Cerebralkreislauf regelnde Wirkung erwähnt ist. Gemäß dieser Druckschrift werden diese Verbindungen auf zweierlei Weise hergestellt. Beim einen Reaktionsweg wird Apovincamin direkt nitriert, wobei ein Gemisch von 9- und 11-(Nitro)-apovincamin anfällt. Aus diesem wird das 9-(Nitro)-apovincamin durch Kristallisieren aus Methanol abgetrennt; das 11-(Nitro) -apovincamin bleibt in der methanolischen Mutterlauge und kann aus dieser chromatographisch abgetrennt werden, jedoch nur als amorphe Substanz. Das 9- und 11-(Nitro)-apovincamin werden dann getrennt reduziert. Gemäß dem zweiten Reaktionsweg werden das 9- beziehungsweise 11-(Amino)-apovincamin aus 9- beziehungsweise 11-(Amino)-vincamin durch Dehydratisieren beziehungsweise Wasserentzug oder aus 9- beziehungsweise 11-(Nitro)-vincamin durch Dehydratisieren beziehungsweise Wasserentzug und anschließendes Reduzieren des erhaltenen 9- beziehungsweise 11-(Nitro)-apovincamines hergestellt. Als dehydratisierendes beziehungsweise wasserentziehendes Mittel wird wasserfreie Ameisensäure verwendet, die Reaktionstemperatur beträgt 100°C, und die Reaktionszeiten – 48 beziehungsweise 24 Stunden – sind recht lang.

Zur Reinigung der Endprodukte sind gegebenenfalls ebenfalls chromatographische Verfahren erforderlich.

Ferner ist in der französischen Patentschrift 2 320 302 das 11-(Acetamido)-vincamin beschrieben, von einer pharmakologischen Wirkung desselben ist jedoch keine Rede.

Weiterhin sind aus der französischen Patentschrift 2 442 846 11-(Alkoxycarbonylamino)-apovincaminsäurealkylester, die in jedem Alkylrest 1 bis 5 Kohlenstoffatom(e) aufweisen können, bekannt.

Der Erfindung liegt die Aufgabe zugrunde, überlegene pharmakologische Wirkungen aufweisende neue 9- beziehungsweise 11-(Amino)-eburnancarbonsäurederivate, ein Verfahren zur Herstellung derselben und eines bekannten 11-(Amino)-eburnancarbonsäurederivates und diese neuen und bekannte Verbindungen enthaltende Arzneimittel zu schaffen.

Das Obige wurde überraschenderweise durch die Erfindung erreicht.

Gegenstand der Erfindung sind in der 9- oder 11-Stellung eine, gegebenenfalls substituierte, Aminogruppe aufweisende Eburnancarbonsäurederivate der allgemeinen Formel

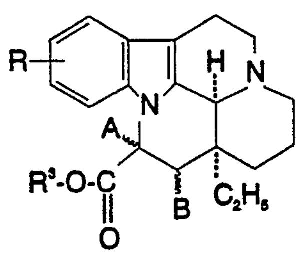

I,

worin

R für eine Aminogruppe oder einen Acylamidorest der allgemeinen Formel

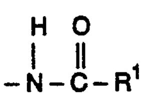

II,

in welchletzterer

$R^1$ Wasserstoff oder einen aliphatischen Kohlenwasserstoffrest mit 1 bis 10 Kohlenstoffatom(en), cycloaliphatischen Kohlenwasserstoffrest mit 3 bis 8 Kohlenstoffatomen, aromatischen Kohlenwasserstoffrest mit 6 bis 14 Kohlenstoffatomen oder einen 1 oder mehr gleiche oder verschiedene aus Stickstoff, Sauerstoff und/oder Schwefel bestehende Heteroatome aufweisenden 5- bis 7-gliedrigen Heteroarylrest, gegebenenfalls zusätzlich zu diesen Gliedern mit 4 zur Bildung eines Benzolrings ankondensierten Gliedern, vor allem einen Pyrrolyl-, Furyl-, Thienyl-Pyridyl-, Pyranyl-, Pyrazolyl-, Imidazolyl-, Pyrimidinyl-, Indolyl- und/oder Chinolylrest bedeutet, wobei die vorstehend genannten Reste mit einem oder mehreren gleichen oder verschiedenen Substituenten substituiert sein können, bestehend aus Halogenatomen, primären, sekundären und tertiären Aminogruppen, Hydroxygruppen, Arylresten mit 6 bis 14 Kohlenstoffatomen, Alkyl-, Alkoxy- und Alkylthioresten mit 1 bis 8 Kohlenstoffatomen, Trifluormethylresten, Carboxylgruppen, Alkoxycarbonylresten mit 1 bis 8 Kohlenstoffatomen im Alkylteil, Thio-, Sulfinyl-, Sulfonyl-, Nitro-, Keto-, Aldehyd- und Cyangruppen und 1 oder mehr aus Stickstoff, Sauerstoff und/oder Schwefel als Heteroatome aufweisende 5- bis 7-gliedrige Heteroarylresten, gegebenenfalls zusätzlich zu diesen Gliedern mit 4 zur Bildung eines Benzolrings ankondensierten Gliedern, vor allem Pyrrolyl-, Furyl-, Thienyl-, Pyridyl-, Pyranyl-, Pyrazolyl-, Imidazolyl-, Pyrimidinyl-, Indolyl- und Chinolylresten, die gegebenenfalls durch 1 oder mehr der aufgeführten Substituenten substituiert sein können, oder einen Sulfonamidorest der allgemeinen Formel

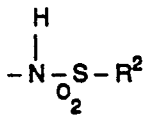

III,

in welchletzterer

$R^2$ einen aliphatischen Kohlenwasserstoffrest mit 1 bis 10 Kohlenstoffatom(en) oder einen aromatischen Kohlenwasserstoffrest mit 6 bis 14 Kohlenstoffatomen bedeutet, wobei diese Reste durch einen oder mehrere gleiche oder verschiedene Substituenten, wie sie im Zusammenhang mit $R^1$ aufgeführt sind, substituiert sein können, steht,

$R^3$ Wasserstoff oder einen aliphatischen Kohlenwasserstoffrest mit 1 bis 10 Kohlenstoffatom(en), cycloaliphatischen Kohlenwasserstoffrest mit 3 bis 8 Kohlenstoffatomen oder aromatischen Kohlenwasserstoffrest mit 6 bis 14 Kohlenstoffatomen bedeutet, wobei diese Reste mit 1 oder mehr gleichen oder verschiedenen Substituenten, wie sie im Zusammenhang mit $R^1$ definiert worden sind, substituiert sein können, und

A für eine Hydroxygruppe steht und

B Wasserstoff bedeutet oder

A und B zusammen eine chemische Bindung darstellen und

R in der 9- oder 11-Stellung ist, mit den weiteren Maßgaben, daß,

a) im Falle daß

R für eine Aminogruppe steht,

$R^3$ von einem nicht substituierten Methylrest verschieden ist, und

b) im Falle daß

R für einen in der 9- oder 11-Stellung befindlichen Acylamidorest der allgemeinen Formel II, bei welchem

$R^1$ einen Methylrest bedeutet,

steht,

A eine Hydroxygruppe bedeutet und

B Wasserstoff darstellt,

$R^3$ von einem nicht substituierten Methylrest verschieden ist,

sowie ihre optisch aktiven Isomere und Salze. Die erfindungsgemäßen Eburnancarbonsäurederivate umfassen also sowohl racemische als auch optisch aktive, von welchletzteren die rechtsdrehenden bevorzugt sind.

Von den 9- und 11-(Amino)-apovincamin der französischen Patentschrift 2 342 980 und den dort ebenfalls erwähnten 9- und 11-(Amino)-vincamin unterscheiden sich die erfindungsgemäßen Eburnancarbonsäurederivate darin, daß sie dann, wenn sie in der 9- beziehungsweise 11-Stellung eine nicht substituierte Aminogruppe aufweisen, keine Methylester sein können.

Von den in der bereits erwähnten französischen Patentschrift 2 342 980 beschriebenen Verbindungen 9-Nitro- und 9-Aminoapovincamin, die nicht in den Rahmen der Erfindung fallen, besitzt, wie Untersuchungen ergeben haben, das 9-Nitro-apovincamin keine antihypoxische Wirkung, wobei der auf eigenen Erfahrungen basierende Analogieschluß zulässig ist, daß auch das 9-Amino-apovincamin keine derartige Wirkung zeigt.

Von dem in der französischen Patentschrift 2 320 302 beschriebenen 11-Acetamido-vincamin unterscheiden sich die erfindungsgemäßen Eburnancarbonsäurederivate darin, daß sie dann, wenn sie Vincaminmethylesterderivate sind, keinen Acylamidorest in der 11-Stellung haben können. Von der bekannten Verbindung sind keine durch Untersuchungen belegte pharmakologischen Wirkungen bekannt.

Von den in der französischen Patentschrift 2 442 846 beschriebenen 11-Alkoxycarbonylamino-apovincaminsäure-alkylestern unterscheiden sich die erfindungsgemäßen Eburnancarbonsäurederivate darin, daß ihre Aminogruppe nicht durch einen Alkoxycarbonylrest substituiert sein kann. Die aus dieser französischen Patentschrift bekannten Verbindungen zeigen in einer Dosis von 1,5 mg/kg eine 58 %-ige Wirkung auf den Kreislauf, diese Wirkung erstreckt sich jedoch nur auf den vertebralen Bereich, während demgegenüber die erfindungsgemäßen Verbindungen in einer um 50 % geringeren Dosis, und zwar in einer Dosis von 1,0 mg/kg, und in einem von dem oben genannten Gefäßbereich verschiedenen Bereich, nämlich an der Carotis eine größere Wirkung, d. h. eine Wirkung von 60 – 80 %, zeigen. Daher kann mit den erfindungsgemäßen Verbindungen eine höhere Wirkung erzielt werden, wobei diese Wirkung darüber hinaus in einem anderen Gefäßbereich verwirklicht wird als im Falle der bekannten Verbindungen.

Ein weiterer Unterschied besteht darin, daß die Wirkung der erfindungsgemäßen Verbindungen selektiv ist, da ein Teil dieser Verbindungen nur im cerebralen Bereich und ein anderer Teil nur im femoralen Bereich wirkt, während die bekannten Verbindungen außer im vertebralen Bereich auch in einem gewissen Ausmaß eine Wirkung im femoralen Bereich zeigen, d. h. daß diese Verbindungen nicht selektiv sind.

Ein weiterer Unterschied zwischen den erfindungsgemäßen Verbindungen und denen aus der genannten französischen Patentschrift bekannten besteht darin, daß die bekannten Verbindungen den Blutdruck permanent beeinflussen, während die Wirkung der erfindungsgemäßen Verbindungen auf den Blutdruck nur vorübergehend ist und etwa nur 1 bis 3 Minuten dauert, was von erheblicher Bedeutung ist.

In der französischen Patentschrift 2 341 585 wird die Herstellung von 9-Nitro- und 9-Amino-vincamin, d.h. von Verbindungen, die nicht in den Rahmen der Erfindung fallen, beschrieben, wobei, wie Versuche gezeigt haben, das 9-Nitro-vincamin keine gefäßerweiternde Wirkung besitzt und demzufolge, wie die Erfahrung gezeigt

hat, auch das 9-Aminoderivat keine derartige Wirkung besitzt.

Der beziehungsweise die aliphatische(n) Kohlenwasserstoffrest(e), für welche[n] $R^1$ oder $R^2$ und/oder $R^3$ stehen kann beziehungsweise können, kann beziehungsweise können geradkettig oder verzweigt, gesättigt oder ungesättigt sein. Beispiele sind der beziehungsweise die Methyl-, Äthyl-, n-Propyl-, Isopropyl-, n-Butyl-, sek. -Butyl-, tert.-Butyl, Isobutyl-, n-Pentyl-, Isopentyl-, n-Hexyl-, Isohexyl-, n-Heptyl-, Isoheptyl-, n-Octyl-, Isooctyl-, Vinyl-, Acryl-, Methacryl- und/oder Propenylrest(e). Der beziehungsweise die aliphatische(n) Kohlenwasserstoffrest(e), für welche(n) $R^1$ oder $R^2$ und/oder $R^3$ stehen kann beziehungsweise können, kann beziehungsweise können [ein] Alkylrest(e), insbesondere [ein] solche[r] mit 1 bis 8, ganz besonders 1 bis 4, vor allem 1 bis 3, wie 2 oder 3 Kohlenstoffatom(en), oder ein Alkenyl- oder Alkinylrest, insbesondere [ein] solche[r] mit 2 bis 4, ganz besonders 2 oder 3, Kohlenstoffatomen, vor allem der Allylrest oder der Propargylrest, sein.

Beispiele für den beziehungsweise die cycloaliphatischen Kohlenwasserstoffrest(e), für welchen $R^1$ und/oder $R^3$ stehen kann beziehungsweise können, ist beziehungsweise sind der beziehungsweise die Cyclopropyl-, Cyclobutyl-, Cyclopentyl-, Cyclohexyl-, Cycloheptyl- und/oder Cyclooctylrest(e). Vorzugsweise ist beziehungsweise sind der beziehungsweise die cycloaliphatische(n) Kohlenwasserstoffrest(e), für welche[n] $R^1$ und/oder $R^3$ stehen kann beziehungsweise können, [ein] Cycloalkylrest(e), insbesondere [ein] solche[r] mit 5 bis 7, ganz besonders 5 oder 6, vor allem 6, Kohlenstoffatomen.

Der beziehungsweise die aromatische(n) Kohlenwasserstoffrest(e), für welche[n] $R^1$ oder $R^2$ und/oder $R^3$ stehen kann beziehungsweise können, kann beziehungsweise können, monocyclisch, nämlich [ein] Phenylrest(e), und/oder polycyclisch nicht kondensiert [isoliert], insbesondere [ein] Diphenylrest(e), und/oder polycyclisch kondensiert, insbesondere [ein] Naphthylrest(e), sein.

Vorzugsweise ist der Heteroarylrest, für welchen
$R^1$ stehen kann, 5- oder 6-gliedrig.

soweit er ein Thienylrest ist, ist er ganz besonders bevorzugt ein Thien-2-ylrest und, soweit er ein Pyridylrest ist, ist er ganz besonders bevorzugt ein Pyrid-3-ylrest.

Vorzugsweise ist beziehungsweise sind der beziehungsweise die Substituent(en), durch welche[n] der beziehungsweise die aliphatische(n), und/oder aromatische(n) Kohlenwasserstoffrest(e), für den $R^1$ oder $R^2$ und/oder $R^3$ stehen kann beziehungsweise können, beziehungsweise der beziehungsweise die cycloaliphatische(n) Rest(e), für den beziehungsweise die $R^1$ und/oder $R^3$ stehen kann beziehungsweise können, beziehungsweise der heteroaromatische Kohlenwasserstoffrest, für den $R^1$ stehen kenn, substituiert sein kann beziehungsweise können, soweit er, beziehungsweise sie [ein] Halogenatom(e) ist beziehungsweise sind [ein] Fluor-, Chlor- und/oder Bromatom(e), soweit er beziehungsweise sie [eine] primäre, sekundäre und/oder tertiäre Aminogruppe(n) ist beziehungsweise sind, [eine] Dialkylaminogruppe(n) mit 1 bis 10, ganz besonders 1 bis 4, vor allem 1 Kohlenstoffatom(en) in jedem Alkylteil, soweit er beziehungsweise sie [ein] Arylrest(e) mit 6 bis 14 Kohlenstoffatomen ist beziehungsweise sind, [ein] Phenyl-, Diphenyl- oder Naphthylrest(e), soweit er beziehungsweise sie [ein] Alkyl-, Alkoxy- und/oder Alkylthiorest(e) mit [je] 1 bis 8 Kohlenstoffatom(en) ist beziehungsweise sind, [ein] solche[r] mit 1 bis 4, ganz besonders 1 oder 2, Kohlenstoffatom(en), soweit er beziehungsweise sie [ein] Alkoxycarbonylrest(e) mit 1 bis 8 ein Alkylteil ist beziehungsweise sind, [ein] solche[r] 1 bis 4, ganz besonders 1 oder 2, vor allem 1, Kohlenstoffatom(en) im Alkylteil, und/oder, soweit er beziehungsweise sie [ein] 1 oder mehr gleiche oder verschiedene Heteroatom(e), bestehend aus Stickstoff, Sauerstoff und/oder Schwefel, aufweisende[r] Heteroarylrest(e) ist beziehungsweise sind, [ein] 5- bis 7-gliedrige[r], ganz besonders 5- oder 6-gliedrige[r], wie bereits gesagt, gegebenenfalls zusätzlich zu diesen Gliedern mit 4 zur Bildung eines Benzolringel ankondensierten Gliedern soweit er beziehungsweise sie [ein] Thienylrest(e) ist beziehungsweise sind, ist beziehungsweise sind er beziehungsweise sie besonders bevorzugt [ein] Thien-2-ylrest(e) und, soweit er beziehungsweise sie [ein] Pyridylrest(e) ist beziehungsweise sind, ist beziehungsweise sind er beziehungsweise sie [ein] Pyrid-3-ylrest(e). Im Falle, daß $R^1$ und/oder $R^3$ für [einen] cycloaliphatischen Rest(e) steht beziehungsweise stehen, hat beziehungsweise haben diese[r] als Substituenten besonders bevorzugt 1 oder mehr Alkylrest(e).

Die Salze der Eburnancarbonsäurederivate der allgemeinen Formel I können Säureadditionssalze oder quaternäre Salze sein. Die Säureadditionssalze können zum Beispiel solche mit den folgenden Säuren sein:

Mit anorganischen Säuren, zum Beispiel Salzsäure, Bromwasserstoffsäure, Schwefelsäure, Phosphorsäure, Überhalogensäuren, wie Überchlorsäure, und mit organischen Säuren, zum Beispiel Ameisensäure, Essigsäure, Propionsäure, Glykolsäure, Maleinsäure, Hydroxymaleinsäure, Fumarsäure, Bernsteinsäure, Weinsäure, Ascorbinsäure, Citronensäure, Apfelsäure, Salicylsäure, Milchsäure, Zimtsäure, Benzoesäure, Phenylessigsäure, p-Aminobenzoesäure, p-Hydroxybenzoesäure, p-Aminosalicylsäure, Alkylsulfonsäuren, wie Methansulfonsäure beziehungsweise Äthansulfonsäure, cycloaliphatischen Sulfonsäuren, wie Cyclohexylsulfonsäure, Arylsulfonsäuren, wie Naphthalinsulfonsäure, p-Toluolsulfonsäure beziehungsweise Sulfanilsäure, und schließlich Aminosäuren, wie Asparaginsäure, Glutaminsäure, N-Acetylasparaginsäure beziehungsweise N-Acetylglutarsäure. Als quaternäre Salze kommen zum Beispiel Methojodide, Methobromide, Methochloride, Methosulfate beziehungsweise deren Alkylhomologen mit 2 bis 4 Kohlenstoffatomen, zum Beispiel Äthojodide, Äthobromide beziehungsweise Äthochloride, in Frage.

Besonders bevorzugte erfindungsgemäße Eburnancarbonsäurederivate sind
11-Benzoylamino)-apovincaminsäure,
9-(Benzoylamino)-apovincaminsäure,
11-(Thien-2'-ylcarbonylamino)-apovincaminsäure,
{11-(2-Thiophen-carbonylamino)-apovincaminsäure},

9-(Thien-2'-ylcarbonylamino)-apovincaminsäure,
{9-(2-Thiophen-carbonylamino)-apovincaminsäure},
11-(Amino)-apovincaminsäureäthylester,
9-(Amino)-apovincaminsäureäthylester und sein Hydrochlorid,
11-(Methylsulfonylamino)-apovincaminsäureäthylester und sein Hydrochlorid,
9-(Methylsulfonylamino)-apovincaminsäureäthylester und sein Hydrochlorid,
11-(Amino)-vincaminsäureäthylester,
9-(Amino)-vincaminsäureäthylester,
11-[4'-(Nitro)-benzoylamino]-apovincaminsäuremethylester,
9-[4'-(Nitro)-benzoylamino]-apovincaminsäuremethylester,
11-[2'-(Brom)-benzoylamino]-apovincaminsäuremethylester und sein Hydrochlorid,
9-[2'-(Brom)-benzoylamino]-apovincaminsäuremethylester und sein Hydrochlorid,
11-[2'-(Fluor)-benzoylamino]-vincamin,
9-[2'-(Fluor)-Benzoylamino]-vincamin,
11-[3',4',5'-Tri-(methoxy)-benzoylamino]-vincamin,
9-[3',4',5'-Tri-(methoxy)-benzoylamino]-vincamin,
11-(Pivaloylamino)-vincamin,
9-(Pivaloylamino)-vincamin,
9-(Benzoylamino)-vincamin,
11-(Benzoylamino)-vincamin,
11-(Thien-2'-ylcarbonylamino)-vincamin,
{11-(2-Thiophen-carbonylamino)-vincamin},
9-(Thien-2'-ylcarbonylamino)-vincamin,
{9-(2-Thiophen-carbonylamino)-vincamin},
11-(Thien-2'-ylacetylamino)-vincamin,
{11-(2-Thiophen-acetylamino)-vincamin},
9-(Thien-2'-ylacetylamino)-vincamin,
{9-(2-Thiophen-acetylamino)-vincamin},
9-(Acetamido)-apovincamin,
11-(Acetamido)-apovincamin,
9-(Acetamido)-apovincaminsäureäthylester und sein Hydrochlorid,
11-(Acetamido)-apovincaminsäureäthylester,
9-(Acetamido)-apovincaminsäureisobutylester,
11-(Acetamido)-apovincaminsäureisobutylester und sein Hydrochlorid,
11-[2',3',4'-Tri-(methoxy)-benzoylamino]-apovincamin,
11-[3',4',5'-Tri-(methoxy)-benzoylamino]-apovincamin,
9-[3',4',5'-Tri-(methoxy)-benzoylamino]-apovincamin,
11-(Pivaloylamino)-apovincamin,
9-(Benzoylamino)-vincaminsäureäthylester,
11-(Benzoylamino)-vincaminsäureäthylester,
9-(Benzoylamino)-apovincaminsäureäthylester,
11-(Benzoylamino)-apovincaminsäureäthylester und sein Hydrochlorid,
11-[Acetamido]-vincaminsäure-[2'-(hydroxy)-äthyl]-ester,
9-[Acetamido]-vincaminsäure-[2'-(hydroxy)-äthyl]-ester,
11-(Nicotinoylamino)-apovincamin,
9-(Nicotinoylamino)-apovincamin,
11-[2'-(Fluor)-benzoylamino]-apovincamin,
9-[2'-(Fluor)-benzoylamino]-apovincamin,
11-(Thien-2'-ylcarbonylamino)-apovincamin,
{11-(2-Thiophen-carbonylamino)-apovincamin},
9-(Thien-2'-ylcarbonylamino)-apovincamin,
{9-(2-Thiophen-carbonylamino)-apovincamin},
9-(Thien-2'-ylcarbonylamino)-apovincaminsäureäthylester,
11-(Thien-2'-ylcarbonylamino)-apovincaminsäureäthylester und sein Hydrochlorid,
11-(Benzoylamino)-apovincamin,
9-(Benzoylamino)-apovincamin,
11-[Benzoylamino]-apovincaminsäure-[2'-(hydroxy)-äthyl]-ester,
9-[Benzoylamino]-apovincaminsäure-[2'-(hydroxy)-äthyl]-ester,
11-[Benzoylamino]-apovincaminsäure-[2'-(methyl)-propyl]-ester und sein Hydrochlorid,
9-[Benzoylamino]-apovincaminsäure-[2'-(methyl)-propyl]-ester und sein Hydrochlorid,
11-(Amino)-vincaminsäure,
9-(Amino)-vincaminsäure,
11-[2'-(Fluor)-benzoylamino]-vincaminsäure,
9-[2'-(Fluor)-benzoylamino]-vincaminsäure,
11-(Cyclohexylcarbonylamino)-vincamin,

11-[3'-(Brom)-propionylamino]-apovincamin,
11-(Cyclohexylcarbonylamino)-apovincamin,
9-(Pivaloylamino)-apovincamin,
9-(Cyclohexylcarbonylamino)-vincamin,
9-[3'-(Brom)-propionylamino]-apovincamin und
9-(Cyclohexylcarbonylamino)-apovincamin
sowie ihre optisch aktiven Isomere.

Gegenstand der Erfindung ist auch ein Verfahren zur Herstellung der erfindungsgemäßen Eburnancarbonsäurederivate beziehungsweise von 11-(Acetamido)-vincamin beziehungsweise 9- beziehungsweise 11-(Amino)-apovincamin welches dadurch gekennzeichnet ist, daß

a) racemische oder optisch aktive Eburnancarbonsäurederivate der allgemeinen Formel

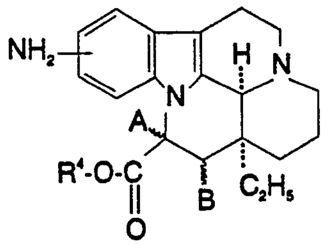

IV,

worin
$R^4$ für einen Methylrest steht oder, falls A und B zusammen eine chemische Bindung darstellen, auch Wasserstoff bedeuten kann, und die Aminogruppe sich in der 9- oder 11-Stellung befindet,

oder deren Salze, gegebenenfalls nach einer Hydrolyse, acyliert werden ausgenommen das Acetylieren, im Falle das $R^4$ für einen Methylrest steht, A eine Hydroxygruppe bedeutet und B Wasserstoff darstellt, und

α) gegebenenfalls die erhaltenen Eburnancarbonsäurederivate der allgemeinen Formel I, bei welchen R für einen Acylamidorest der allgemeinen Formel II oder einen Sulfonylamidorest der allgemeinen Formel III, wobei $R^1$ und $R^2$ die oben angegebenen Bedeutungen haben, steht, A und B die oben angegebenen Bedeutungen haben und $R^3$ die Bedeutung von $R^4$ hat, beziehungsweise ihre Salze verestert beziehungsweise umgeestert werden und/oder

β) gegebenenfalls die erhaltenen Eburnancarbonsäurederivate der allgemeinen Formel I, bei welchen R für einen Acylamidorest der allgemeinen Formel II oder einen Sulfonylamidorest der allgemeinen Formel III, wobei $R^1$ und $R^2$ die oben angegebenen Bedeutungen haben, steht, A für eine Hydroxygruppe steht, B Wasserstoff bedeutet und $R^3$ von Wasserstoff verschieden ist, beziehungsweise ihre Salze hydrolysiert werden und/oder

γ) gegebenenfalls die erhaltenen Eburnancarbonsäurederivate der allgemeinen Formel I, bei welchen R und $R^3$ die oben angegebenen Bedeutungen, ausgenommen die Bedeutung nicht substituierter Methylrest für $R^3$, haben, A für eine Hydroxygruppe steht und B Wasserstoff bedeutet, beziehungsweise ihre Salze dehydratisiert werden oder

b) racemische oder optisch aktive Eburnancarbonsäurederivate der allgemeinen Formel

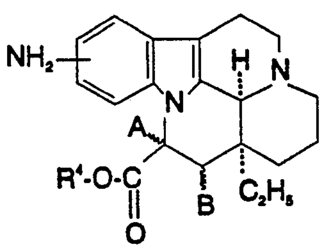

IV,

worin
$R^4$ für einen Methylrest steht oder, falls A und B zusammen eine chemische Bindung darstellen, auch Wasserstoff bedeuten kann, und

die Aminogruppe sich in der 9- oder 11-Stellung befindet, oder deren Salze verestert beziehungsweise umgeestert werden und

α) gegebenenfalls die erhaltenen Eburnancarbonsäurederivate der allgemeinen Formel I, bei welchen R für eine Aminogruppe steht, A eine Hydroxygruppe bedeutet, B ein Wasserstoffatom darstellt und $R^3$ von Wasserstoff verschieden ist, beziehungsweise ihre Salze hydrolysiert werden und/oder

β) gegebenenfalls die erhaltenen Eburnancarbonsäurederivate der allgemeinen Formel I, bei welchen R und $R^3$ die oben angegebenen Bedeutungen, ausgenommen die Bedeutung nicht substituierter Methylrest für $R^3$, haben, A für eine Hydroxygruppe steht und B Wasserstoff bedeutet, beziehungsweise ihre Salze dehydratisiert werden und/oder

γ) gegebenenfalls die erhaltenen Eburnancarbonsäurederivate der allgemeinen Formel I, bei welchen R für eine Aminogruppe steht und $R^3$, A und B die oben angegebenen Bedeutungen haben, beziehungsweise ihre Salze acyliert werden oderr

c) racemische oder optisch aktive 9- oder 11-(Nitro)-eburnancarbonsäurederivate der allgemeinen Formel

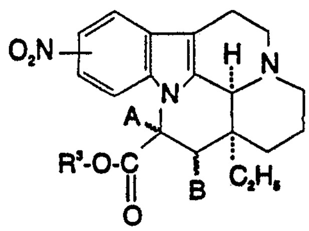

V,

worin $R^3$, A und B die oben angegebenen Bedeutungen haben und die Nitrogruppe in der 9- oder 11-Stellung ist, reduziert werden und gegebenenfalls die Umsetzungen nach a) α) und/oder α) β) beziehungsweise b) α) und/oder a) γ) beziehungsweise b) β) und/oder b) γ) durchgeführt werden

sowie gegebenenfalls die erhaltenen Eburnancarbonsäurederivate der allgemeinen Formel I in Säureadditionssalze oder quaternäre Salze überführt werden und/oder gegebenenfalls die erhaltenen Salze der Eburnancarbonsäurederivate der allgemeinen Formel I in die freien Eburnancarbonsäurederivate der allgemeinen Formel I oder in andere Säureadditionssalze beziehungsweise quaternäre Salze überführt werden und/oder gegebenenfalls eine Spaltung der erhaltenen racemischen Eburnancarbonsäurederivate der allgemeinen Formel I beziehungsweise von deren Säureadditionssalzen beziehungsweise quaternären Salzen beziehungsweise eine Racemisierung der entsprechenden optisch aktiven Verbindungen in Stereoisomere vorgenommen wird.

Nach dem erfindungsgemäßen Verfahren lassen sich gegenüber der französischen Patentschrift 2 342 980 sowohl 9- als auch 11-(Amino)-apovincamin in einfacher Weise auch durch Verestern der entsprechenden neuen 9- beziehungsweise 11-(Amino)-apovincaminsäuren herstellen. Die Herstellung der dazu als Ausgangsstoffe verwendeten neuen Aminoapovincaminsäuren kann nach der Variante c) des erfindungsgemäßen Verfahrens beziehungsweise nach der der ungarischen Patentanmeldung vom 11. Juli 1984 mit dem Aktenzeichen No. 2702/84 mit entsprechenden gleichzeitig eingereichten europäischen Patentanmeldung EP-A-173 824 derselben Anmelderin erfolgen. Das Wesen dieser Herstellung besteht darin, daß die entsprechenden (Nitro)-apovincaminsäuren reduziert werden. II,

Diejenigen der im erfindungsgemäßen Verfahren als Ausgangsstoffe verwendeten Eburnancarbonsäurederivate der allgemeinen Formel IV, bei welchen $R^4$ für einen Methylrest steht, können auf die in den französischen Patentschriften 2 341 585 und 2 342 980 beschriebene Weise hergestellt worden sein.

Diejenigen der im erfindungsgemäßen Verfahren als Ausgangsstoffe verwendeten Eburnancarbonsäurederivate der allgemeinen Formel IV, bei welchen $R^4$ für Wasserstoff steht und A und B zusammen eine chemische Bindung darstellen, das heißt 9- und 11-(Amino)-apovincaminsäure, können wie bereits gesagt nach der Variante c) des erfindungsgemäßen Verfahrens beziehungsweise nach dem in der EP-A-173 824 beschriebenen Verfahren durch Reduktion der entsprechenden Nitroverbindungen hergestellt werden.

Die im erfindungsgemäßen Verfahren als Ausgangsstoffe verwendeten Eburnancarbonsäurederivate der allgemeinen Formel II und die Eburnancarbonsäurederivate der allgemeinen Formel I, bei welchen R für eine Aminogruppe steht, können mit jeder beliebigen, den gewünschten Acylrest innerhalb der obigen Festlegungen aufweisenden Carbon- oder Sulfonsäure oder deren funktionellen Derivaten, zum Beispiel Anhydriden beziehungsweise Halogeniden, acyliert werden. Von den Säurehalogeniden sind besonders die Säurechloride geeignet.

Soll eine Verbindung der allgemeinen Formel IV acyliert werden, in der $R^4$ für Wasserstoff oder einen Methylrest und A zusammen mit B für eine chemische Bindung stehen, so kann man die Acylierung in dem der Herstellung der Verbindung der allgemeinen Formel IV dienenden Reaktionsgemisch vornehmen. In diesem Falle wird nach Reduktion der entsprechenden Nitroverbindung der Katalysator beziehungsweise das Reduktionsmittel aus dem Reaktionsgemisch entfernt und das Acylierungsmittel direkt zu dem Gemisch gegeben. Die entsprechende Aminovincaminsäure beziehungsweise das entsprechende Apovincamin braucht demnach nicht zwischenzeitlich abgetrennt zu werden. Auf diese Weise ist es möglich, die Acylaminoapovincaminsäurederivate in einem Schritt direkt aus den Nitroapovincaminsäuren beziehungsweise ihren Estern herzustellen, was für technische beziehungsweise industrielle Synthesen von Interesse ist.

Wird zum Acylieren eine Carbon- oder Sulfonsäure eingesetzt, so führt man die Umsetzung zweckmäßig in einem inerten organischen dipolaren aprotischen Lösungsmittel aus. Als Lösungsmittel sind zum Beispiel Dimethylformamid, Dimethylsulfoxyd und Acetonitril geeignet. Man kann in Gegenwart eines Kondensationsmittels, zum Beispiel eines Carbodiimids, wie Dicyclohexylcarbodiimid, arbeiten. Die Reaktionstemperatur liegt zweckmäßig zwischen 0°C und 40°C, insbesondere bei etwa Raumtemperatur.

Wird als Acylierungsmittel ein Säureanhydrid eingesetzt, so sind als Lösungsmittel eher apolare aprotische Lösungsmittel, wie, gegebenenfalls halogenierte, aliphatische oder aromatische Kohlenwasserstoffe, zum Beispiel Benzol, Chloroform und Dichlormethan, geeignet, jedoch kann auch in polar-protischen Lösungsmitteln, zweckmäßig in aliphatischen Alkoholen mit 1 – 6 Kohlenstoffatomen, zum Beispiel in Methanol, oder aber im Überschuß des Säureanhydrids gearbeitet werden. Die Reaktionstemperaturen liegen zwischen 0°C und 150°C, insbesondere wird bei etwa Raumtemperatur gearbeitet.

Wird zum Acylieren ein Säurehalogenid, zweckmäßig ein Säurechlorid, verwendet, so wird die Umsetzung zweckmäßig in einem neutralen organischen Lösungsmittel vorgenommen. Geeignet sind zum Beispiel aliphatische Äther, wie Diäthyläther, cyclische Äther, wie Tetrahydrofuran, gegebenenfalls halogensubstituierte, aliphatische oder aromatische Kohlenwasserstoffe, zum Beispiel Chloroform und Benzol, und schließlich organische Basen, wie zum Beispiel Pyridin. Zweckmäßig arbeitet man in Gegenwart eines Säurebindemittels. Als solches können anorganische Basen, zum Beispiel Alkalihydroxide, Alkalicarbonate, wie Kaliumcarbonat, ferner Alkalihydride, zum Beispiel Natriumhydrid, ferner organische tertiäre Basen, zum Beispiel Pyridin, verwendet werden. Die organischen Basen können in gewissen Fällen gleichzeitig als Lösungsmittel und als Säurebindemittel wirken. Die Reaktionstemperatur kann innerhalb eines weiten Bereiches variiert werden. Zweckmäßig arbeitet man bei 0 – 100°C. Ist die Ausgangsverbindung kein Ester, sondern eine Säure ($R^4$ = H), d.h. selbst von saurem Charakter, so acyliert man zweckmäßig mit einem Säurehalogenid oder Säureanhydrid in einem wäßrigen Lösungsmittelmedium, das zusätzlich ein mit Wasser mischbares organisches Lösungsmittel, zum Beispiel einen niederen aliphatischen Alkohol, enthält. Die Reaktionstemperatur sollte zwischen 0°C und 100°C liegen, bevorzugt sind Temperaturen um Raumtemperatur. Bevorzugt wird auch ein Säurebindemittel verwendet, zum Beispiel ein Alkalihydroxyd, wie Kalium- oder Natriumhydroxyd, oder ein Alkalicarbonat, wie zum Beispiel Natriumcarbonat oder Kaliumcarbonat.

Die Verbindungen der allgemeinen Formel IV, in denen $R^4$ für Wasserstoff steht, sowie die Verbindungen der allgemeinen Formel I, in denen $R^3$ für Wasserstoff steht, können direkt oder indirekt verestert werden.

Zur direkten Veresterung werden die entsprechenden Verbindungen der allgemeinen Formel IV beziehungsweise I mit einer Hydroxyverbindung der allgemeinen Formel $R^3$–OH (worin die Bedeutung von $R^3$ mit Ausnahme von Wasserstoff die gleiche wie oben ist) umgesetzt. Die Hydroxyverbindung wird im Überschuß eingesetzt, vorzugsweise wird unter Ausschluß der Luftfeuchtigkeit gearbeitet, zweckmäßig wird auch ein Katalysator beziehungsweise ein Dehydratisiermittel bzw. wasserentziehendes Mittel zugesetzt. Als Katalysator kommen insbesondere starke organische und anorganische Säuren, zum Beispiel Salzsäure, vorzugsweise in Gasform, ferner Schwefelsäure und p-Toluolsulfonsäure, in Frage, als Dehydratisiermittel bzw. wasserentziehendes Mittel kann zum Beispiel ein Molekularsieb verwendet werden.

Bei der indirekten Veresterung wird aus der Verbindung der allgemeinen Formel IV beziehungsweise I zunächst mit einer Base, zum Beispiel einem Alkalihydroxyd, wie Lithium-, Natrium- oder Kaliumhydroxyd, oder einem Alkalicarbonat, zum Beispiel Natrium- oder Kaliumcarbonat, oder Ammoniak, gegebenenfalls im Reaktionsgemisch selbst, ein Salz gebildet und das abgetrennte oder im Reaktionsgemisch in Lösung befindliche Salz dann mit einer Verbindung der allgemeinen Formel $R^3$–Y umgesetzt, worin die Bedeutung von $R^3$ mit Ausnahme von Wasserstoff die gleiche wie oben ist und Y einen Säurerest (im Falle, daß es sich um einen organischen Säurerest handelt, ist darunter ein Acyloxyrest zu verstehen) bedeutet. Im Falle von Verbindungen $R^3$–Y, in denen Y für ein Halogenid, zum Beispiel Chlorid, Bromid oder Jodid, oder aber für Sulfat steht, verwendet man zweckmäßig ein aprotisches dipolares Lösungsmittel, wie Acetonitril oder Dimethylformamid. Das Metall- oder Ammoniumsalz der Verbindung der allgemeinen Formel IV oder I wird in dem Lösungsmittel suspendiert, und das entsprechende Säurehalogenid wird der Suspension in geringem Überschuß zugesetzt. Die Reaktionstemperaturen liegen zwischen 50°C und 120°C. Als Lösungsmittel kann auch ein Gemisch aus einem der aufgeführten Lösungsmittel und Wasser verwendet werden. In diesem Fall führt man die Umsetzung in Gegenwart eines zweckmäßig gewählten Phasentransfer-Katalysators aus. Geeignete Phasentransferkatalysatoren sind zum Beispiel die Tetraalkylammoniumhalogenide.

Die Verbindungen der allgemeinen Formel I, in denen die Bedeutung von R die gleiche wie oben ist, A für Hydroxygruppe und B für Wasserstoff steht und die Bedeutung von $R^3$ mit Ausnahme von Wasserstoff die gleiche wie oben ist, werden zweckmäßig in alkalischem Medium hydrolysiert. Als Lösungsmittel sind mit Wasser mischbare organische Lösungsmittel, wie die aliphatischen Alkohole, zum Beispiel Methanol und Äthanol, aliphatische Ketone, zum Beispiel Aceton, mit einem Wassergehalt von 5 bis 50 Gew.-% geeignet. Die alkalischen Bedingungen werden durch den Zusatz eines Alkalihydroxyds, zum Beispiel von Natrium- oder Kaliumhydroxyd, eingestellt. Die Reaktionstemperaturen liegen zwischen 20°C und 120°C.

Das Reduzieren nach der Variante c) des erfindungsgemäßen Verfahrens kann durch katalytisches Hydrieren, vorteilhaft in Gegenwart von Palladium auf Aktivkohle oder Raney-Nickel, vorzugsweise bei annähernd neutralem pH-Wert, oder mit chemischen Reduktionsmitteln, vorteilhaft Zinn in salzsaurem Medium, durchgeführt werden.

Diejenigen Verbindungen der allgemeinen Formel I, in denen die Bedeutung von R und $R^3$ die gleiche wie oben ist und A für Hydroxygruppe und B für Wasserstoffatom stehen, können auf jede für das Dehydratisieren von Vincaminalkaloiden geeignete Weise dehydratiert werden. Vorzugsweise wird das Dehydratisieren in Gegenwart von p-Toluolsulfonsäure unter azeotropem Abdestillieren des gebildeten Wassers vorgenommen. Gemäß einer bevorzugten Ausführungsform wird die Verbindung der allgemeinen Formel I in einem inerten organischen Lösungsmittel, zum Beispiel in einem aromatischen Kohlenwasserstoff, gelöst und die Lösung in Gegenwart von p-Toluolsulfonsäure gekocht, wobei dafür – zum Beispiel durch Benutzen eines Wasserabscheiders – gesorgt wird, daß das gebildete Wasser kontinuierlich aus dem System austritt.

Aus den erfindungsgemäßen Verbindungen der allgemeinen Formel I können gewünschtenfalls quaternäre Salze gebildet werden. Bevorzugt werden zur Quaternisierung Alkylhalogenide oder Alkylsulfate in einem geringen Überschuß eingesetzt. Die Quaternisierung wird zweckmäßig in einem inerten, organischen wasserfreien Lösungsmittel vorgenommen. Geeignet sind zum Beispiel die aliphatischen Alkohole mit 1 – 6 Kohlenstoffatomen, d.h. zum Beispiel wasserfreies Methanol oder Äthanol, Ketone, wie Aceton oder Methyläthylketon, ferner Acetonitril. Die Umsetzung wird zweckmäßig bei erhöhter Temperatur vorgenommen.

Die erfindungsgemäßen Verbindungen können auch zu Säureadditionssalzen umgesetzt werden. Die dafür geeigneten Säuren wurden bereits genannt. Zur Herstellung der Säureadditionssalze werden die Verbindungen der allgemeinen Formel I in einem inerten organischen Lösungsmittel, zum Beispiel einem aliphatischen Alkohol mit 1 – 6 Kohlenstoffatomen, gelöst, und die Lösung wird bis zur schwach sauren Reaktion (pH 5 – 6) mit der entsprechenden Säure oder deren mit dem gleichen Lösungsmittel bereiteten Lösung versetzt. Das ausgefallene Säureadditionssalz wird auf eine geeignete Weise, zum Beispiel durch Filtrieren, abgetrennt.

Die Erfindung betrifft sowohl die racemischen Verbindungen der allgemeinen Formel I als auch ihre optisch aktiven Isomeren. Geht man von racemischen Ausgangsstoffen aus, so wird auch das Endprodukt als Racemat erhalten; optisch aktive Ausgangsverbindungen führen zu optisch aktiven Endprodukten. Liegt das Endprodukt als Racemat vor, so kann dieses in jeder bekannten Weise in die optisch aktiven Antipoden aufgetrennt werden. Die Trennung in optisch aktive Isomere kann in jedem Schritt des erfindungsgemäßen Verfahrens erfolgen.

Ferner sind erfindungsgemäß Arzneimittel, welche 1 oder mehr erfindungsgemäße Verbindung(en) und/oder 11-(Acetamido)-vincamin (Formel I mit der Ergänzung, daß R für einen Acylamidorest der allgemeinen Formel II mit $R^1$ = Acetylrest in der 11-Stellung steht, A eine Hydroxygruppe bedeutet, B Wasserstoff darstellt und $R^3$ für einen Methylrest steht), zweckmäßigerweise zusammen mit 1 oder mehr üblichen Träger- und/oder Hilfsstoff(en), enthalten, vorgesehen.

Die erfindungsgemäßen Verbindungen der allgemeinen Formel I sowie ihre physiologisch verträglichen quaternären Salze und Säureadditionssalze weisen nämlich pharmakologische, insbesondere gefäßerweiternde, spasmolytische, antihypoxische und antikonvulsive, Wirkungen auf.

Die gefäßerweiternde Wirkung wurde an mit "Chloralose-Pentobarbital"-Gemisch narkotisierten Hunden untersucht. Die Durchblutung der Extremitäten wurde an der Arteria femoralis gemessen, über die Durchblutung des Gehirns ergaben Messungen an der Arteria carotis interna Aufschluß. Zu den Messungen wurden elektromagnetische Strömungsmesser verwendet. Die zu untersuchenden Substanzen wurden in einer Dosis von 1 mg/kg Körpergewicht sechs Hunden appliziert, und die in den gemessenen Parametern eintretenden Veränderungen wurden in Prozent umgerechnet. Als Referenzsubstanz diente der in der Medizin als gefäßerweiternde Substanz allgemein angewendete (+)-Apovincaminsäureäthylester. Die Ergebnisse sind in der Tabelle I zusammengefaßt.

Die spasmolytische Aktivität der Verbindungen wurde am isolierten Meerschweinchendarm auf klassische Weise bestimmt (Magnus, R., Pflügers Arch., *102*, 123/1904/). Als Referenzsubstanz wurden das bekannte Spasmolytikum Papaverin, ferner (+)-Apovincaminsäureäthylester verwendet. Die Ergebnisse sind in der Tabelle II zusammengefaßt.

**Tabelle I**

Wirkung einer Dosis von 1 mg/kg intravenös verabreichtem Wirkstoff auf den Kreislauf narkotisierter Hunde (Veränderungen in Prozent)

| Bezeichnung | Substanz Beispiel | A. carotis | A. femoralis |
|---|---|---|---|
| Apovincaminsäureäthylester (Referenz) | – | + 30 | + 15 |
| 11-(Thien-2'-ylcarbonylamino)-apovincaminsäureäthylesterhydrochlorid | 37 | + 65 | 0 |
| 11-(Benzoylamino)-apovincaminsäureäthylesterhydrochlorid | 38 | + 80 | 0 |
| 11-(Acetamido)-apovincamin | 21 | + 50 | 0 |
| 11-(Acetamido)-apovincaminsäureisobutylesterhydrochlorid | 39 | + 60 | 0 |
| 9-(Methylsulfonylamino)-apovincaminsäureäthylesterhydrochlorid | 7 | 0 | + 70 |
| 9-(Acetamido)-apovincaminsäureäthylesterhydrochlorid | 40 | 0 | + 50 |
| 9-(Benzoylamino)-apovincaminsäureäthylester | 23 | + 35 | + 140 |

**Tabelle II**

Hemmung der durch Bariumchlorid ausgelösten Kontraktion am isolierten Meerschweinchenileum

| Bezeichnung | Substanz Beispiel | $ED_{50}$ µg/ml |
|---|---|---|
| Papaverin (Referenz) | – | 5,4 |
| Apovincaminsäureäthylester (Referenz) | – | 2,0 |
| 11-(Benzoylamino)-apovincamin | 28 | 2,5 |
| 11-[2'-(Fluor)-benzoylamino]-apovincamin | 26 | 4,0 |

Die antihypoxische Wirkung wurde an nicht narkotisierten Mäusen in normobarer Hypoxie bestimmt. Fünf männliche Mäuse wurden in einen Glaszylinder von 3 Liter Volumen gesetzt, und in den Zylinder wurde ein aus 96 % Stickstoff und 4 % Sauerstoff bestehendes Gasgemisch eingeleitet. Gemessen wurde die Zeit zwischen dem Einbringen der Tiere in den Zylinder und ihrem Verenden. Diejenigen Tiere, die die unbehandelte Kontrolle um mehr als die doppelte Zeitspanne überlebten, können als erfolgreich behandelt gelten. Die zu untersuchenden Substanzen wurden je 10 Tieren in einer Dosis von 50 mg/kg intraperitoneal 30 Minuten vor Versuchsbeginn verabreicht.

**Tabelle III**

Antihypoxische Wirkung der Verbindungen auf den normobarhypoxischen Zustand nicht narkotisierter Mäuse

| Bezeichnung | Substanz Beispiel | Überlebensdauer*) (min) | Überlebensdauer*) (%) | Schutz (%) |
|---|---|---|---|---|
| Kontrolle | – | 6,0 ± 1,04 | – | – |
| 9-(Amino)-apovincaminsäureäthylesterhydrochlorid | 6 | 7,3 ± 2,31 | 22 | 10 |
| Apovincaminsäureäthylester (Referenz) | – | 6,4 ± 1,58 | 7 | – |
| Vincamin | – | 6,1 ± 1,30 | 2 | – |

*) durchschnittliche Lebensdauer ± Streuung (min. bzw. % Verlängerung)

Die antihypoxische Wirkung wurde ferner noch an asphyxischer Anoxie (Caillard C. et al.: Life Sci. *16*, 1607 /1975/) und an hypobarer Hypoxie (Baumel J. et al.: Proc. Soc. Biol. N.Y. *132*, 629 /1969/) gemessen. Dabei wurden die Tiere 16 Stunden lang ausgehungert, dann p.o. behandelt und eine Stunde später in gut schließende Glasgefäße von 100 ml Volumen gesetzt. Als Überlebenszeit wurde die Zeit vom Verschließen des Gefäßes bis zur letzten Atembewegung registriert. Als erfolgreich behandelt können diejenigen Tiere gelten, die eine um 30 % längere Zeit an Leben blieben als der Durchschnitt der Kontrollgruppe.

Die Wirkung auf die hypobare Hypoxie wurde an 16 Stunden lang ausgehungerten Tieren untersucht, die eine Stunde vor Versuchsbeginn p.o. mit den Verbindungen behandelt wurden. Die Tiere wurden in einen Vakumexsikkator gesetzt, und der Druck wurde innerhalb von 20 Sekunden auf 221 mbar [170 Torr] gesenkt. Von diesem Zeitpunkt an wurde als Überlebensdauer die Zeit bis zur letzten Atembewegung gemessen. Als erfolgreich behandelt wurden diejenigen Tiere betrachtet, deren Überlebensdauer um 100 % länger war als die durchschnittliche Überlebensdauer der Kontrolle. Aus dem prozentualen Anteil der geschützten Tiere wurden mittels Probit-Analyse die $ED_{50}$-Werte berechnet.

**Tabelle IV**

| Bezeichnung | Substanz Beispiel | $ED_{50}$ (mg/kg, p.o.) Asphyxische Anoxie | $ED_{50}$ (mg/kg, p.o.) Hypobare Hypoxie |
|---|---|---|---|
| Mephenitoin (Referenz) | – | > 80,0 | > 80,0 |
| Apovincaminsäureäthylester (Referenz) | – | > 50,0 | > 50,0 |
| 11-[4'-(Nitro)-benzoylamino]-apovincamin | 9 | 40,9 | 42,1 |
| 11-(Benzoylamino)-apovincamin | 28 | 25,0 | > 50,0 |

Die antikonvulsive Wirkung wurde nach folgenden Methoden untersucht.

Maximaler Elektroschock an Mäusen (Swinyard E.A. et al.: J. Pharmacol. Exp. Ther. *106*, 319 /1959/). Der Schock wurde an 22 – 24 g schweren Tieren durch eine corneale Elektrode ausgelöst (20 mA, 0,2 s, HSE-Schockgerät Typ 207). Als geschützt wurden diejenigen Tiere betrachtet, bei denen durch die Behandlung die tonische Extension der hinteren Gliedmaßen ausblieb.

Metrazolkrampf an Mäusen (Everett, G.M. and Richards, R.K.: J. Pharmacol. Exp. Ther. *81*, 402 /1944/). Die Tiere erhielten nach der Vorbehandlung subkutan 125 mg/kg Pentylentetrazol. Registriert wurde das Ausbleiben des persistierenden clonischen Krampfes und des tonischen Extensorkrampfes.

Strychninkrampf (Kerley, T.L. et al.: J. Pharmacol. Exp. Ther. *132*, 360 /1961/). Durch i.p. Applikation von 2,5 mg/kg Strychninnitrat wurde ein tonischer Extensorkrampf ausgelöst. Als geschützt wurden diejenigen überlebenden Tiere betrachtet, bei denen durch die Behandlung der Krampf ausblieb.

Die neurotoxischen Nebenwirkungen wurden auf folgende Weise untersucht.

Messung der Muskelinkoordination (Rotarod) (H. Kwibara et al.: Japan J. Pharmacol. *27*, 117 /1977/). Nach vorherigem Training wurden Tiere ausgewählt, die im Stande sind, 120 Sekunden lang auf dem horizontalen Stab (Durchmesser 20 mm, Drehzahl 12 $min^{-1}$) oben zu bleiben. Als Muskelinkoordination wurde gewertet, wenn die Tiere innerhalb der Limitzeit herabfielen.

Muskelrelaxierende Wirkung (Traction). Männliche Mäuse eines Gewichtes von 18 – 22 g wurden mit den Vorderpfoten an einen horizontalen Draht von 5 mm Durchmesser aufgehängt. Verminderter Muskeltonus galt als bei denjenigen Tieren vorhanden, die ihre Hinterpfoten nicht innerhalb von 5 Sekunden nach oben neben die Vorderpfoten zogen.

**Tabelle V**

| $ED_{50}$ (mg/kg, p.o.) | Mephenitoin (Referenz) | A | B |
|---|---|---|---|
| Antikonvulsive Wirkung | | | |
| max. Elektroschock | 26,3 | 16,0 | 16,7 |
| Metrazolkrampf | 24,5 | 15,9 | 9,7 |
| Strychninkrampf | – | | |
| Neurotoxische Wirkung | | | |
| Rotarod | 78,6 | 180 mg/kg wirkungslos | |
| Traction | 154,3 | 180 mg/kg wirkungslos | |

A = 11-(Benzoylamino)-apovincamin [Beispiel 28]
B = 11-(Benzoylamino)-apovincaminsäureäthylesterhydrochlorid [Beispiel 38]

Aus diesen Experimenten ist ersichtlich, daß die Substitution im Ring A die biologische Wirkung sehr vorteilhaft beeinflußt. Das manifestiert sich vor allem in einer selektiven gefäßerweiternden Wirkung. Ein Teil der Verbindungen fördert spezifisch die Durchblutung des Gehirns, die der Extremitäten jedoch gar nicht oder nur in geringem Maße. Die gefäßerweiternde Wirkung im Gehirn ist besonders im Falle der in 11-Stellung substituierten Verbindungen stärker als die der Referenzverbindung ähnlicher Struktur, während die Substitution in 9-Stellung in unerwarteter Weise die periphere gefäßerweiternde Wirkung erhöht. Besondere Bedeutung kommt der Benzoylaminogruppe zu; das 11-Benzoylaminoderivat zeigt eine bedeutende gefäßerweiternde Wirkung im Gehirn, das 9-Benzoylaminoderivat hingegen hauptsächlich periphere gefäßerweiternde Wirkung.

Außer den Wirkungen auf den Kreislauf haben die Verbindungen auch eine spasmolytische Wirkung auf die glatte Muskulatur, die die Wirkung das als Referenz verwendeten Papaverines erreicht oder übertrifft.

Wie die Versuche zeigten, verlängern die Verbindungen bei Gehirnhypoxie mit tödlichem Ausgang die Überlebensdauer. Das kann bei der Behandlung von Störungen in der Blut- und Sauerstoffversorgung des Gehirns von therapeutischem Nutzen sein.

Die Verbindungen zeigen eine überragend gute antikonvulsive Wirkung, sie sind in dieser Richtung wirksamer als die Referenzsubstanz Mephenitoin und zeigen nicht einmal in der zehnfachen Menge der antikonvulsiven Dosis neurotoxische Nebenwirkungen, während die Referenzsubstanz in der 3 – 6-fachen Menge der antikonvulsiven Dosis bereits neurotoxisch wirkt. Die therapeutische Breite der Referenzsubstanz ist demnach wesentlich geringer als die der erfindungsgemäßen Verbindungen.

Die antikonvulsive und antihypoxische Wirkung der Verbindungen ist zusammen mit der durchblutungsfördernden Wirkung insbesondere zur Behandlung von Krankheitsformen vorteilhaft, bei denen die Versorgung des Gehirns mit Sauerstoff vorübergehend oder dauerhaft gestört ist.

Die Verbindungen der allgemeinen Formel (I) können mit den in der Arzneimittelherstellung üblichen, zur parenteralen oder enteralen Applikation geeigneten, nichttoxischen festen oder flüssigen Trägerstoffen und/oder Hilfsstoffen vermischt und zu Arzneimittelpräparaten formuliert werden. Als Trägerstoffe finden zum Beispiel Wasser, Gelatine, Lactose, Stärke, Pektin, Magnesiumstearat, Stearinsäure, Talk, Pflanzenöle (wie Erdnußöl und Olivenöl) Verwendung. Die Wirkstoffe werden zu den üblichen Darreichungsformen formuliert, zum Beispiel festen Präparaten (wie abgerundeten oder eckigen Tabletten, Dragees, Kapseln, z. B. Hartgelatinekapseln, Pillen und Zäpfchen) oder flüssigen Präparaten (mit Öl oder Wasser bereiteten injizierbaren Lösungen oder Suspensionen). Die Menge des festen Trägerstoffes kann in einem weiten Bereich variieren, eine Darreichungseinheit enthält vorzugsweise etwa 25 mg – 1 g Trägerstoff. Die Präparate können gegebenenfalls die üblichen Hilfsstoffe, zum

Beispiel Konservierungs-, Stabilisierungs-, Netz- und Emulgiermittel, Salze zum Einstellen des osmotischen Druckes, Puffer und Geruchs- und Geschmacksstoffe, enthalten. Die Präparate können ferner weitere pharmazeutisch wertvolle, bekannte Verbindungen enthalten, ohne daß dadurch eine synergistische Wirkung einträte. Die Präparate werden vorzugsweise in Darreichungseinheiten formuliert, die der gewünschten Applikationsform entsprechen. Die Arzneimittelpräparate werden mittels der dafür üblichen Verfahren hergestellt, zum Beispiel durch Sieben, Mischen, Granulieren und Pressen der Komponenten oder durch Auflösen. Die Präparate können ferner weiteren, in der Arzneimittelindustrie üblichen Arbeitsgängen unterzogen, zum Beispiel sterilisiert werden.

Die Erfindung wird an Hand der folgenden Beispiele näher erläutert.

**Beispiel 1**

**11-Benzoylamino-apovincaminsäure**

3,37 g (0,01 Mol) (+)-11-Aminoapovincaminsäure werden in 100 ml n Natronlauge gelöst und zu der Lösung bei Raumtemperarur 1,7 ml (0,015 Mol) Benzoylchlorid gegeben. Das Gemisch wird zwei Stunden lang gerührt, dann noch einmal mit der gleichen Menge Benzoylchlorid versetzt und weitere zwei Stunden lang gerührt. Das Gemisch wird mit 10 %-iger Salzsäure neutralisiert, der Niederschlag abfiltriert und zunächst mit 50 ml Wasser der Temperatur von 50 – 60°C, dann mit 50 ml kaltem Wasser gewaschen und schließlich getrocknet. Man erhält 3,30 g (75 %) des Titelproduktes, das bei 244 – 248°C schmilzt.

Elementaranalyse für $C_{27}H_{27}N_3O_3$ (M = 441,52)

| | | | |
|---|---|---|---|
| berechnet, %: | C 73,45 | H 6,16 | N 9,52 |
| gefunden. %: | C 73,62 | H 6,82 | N 9,48. |

$^1$H-NMR (DMSO-$d_6$) δ: Et 0,94 t (3), H-3 4,10 s (1), H-15 6,00 s (1), NH 7,95 s (1), Ar-H 7,3 – 7,6 m (6), $H_{ortho}$ 7,09 – 8,05 m (2), COOH 10,10 s (1), Gerüstprotonen 1,10 – 3,4(m).

**Beispiel 2**

**11-(Thien-2'-ylcarbonylamino)-apovincaminsäure**

3,37 g (0,01 Mol) (+)-11-Aminovincaminsäure werden in 100 ml n Natronlauge gelöst und zu der Lösung unter Rühren bei Raumtemperatur 3 ml (0,03 Mol) Thiophen-2-carbonsäurechlorid gegeben. Das Gemisch wird eine halbe Stunde lang gerührt und dann durch Zusatz von 10 %-iger Salzsaure neutralisiert. Die ausgefallenen Kristalle werden abfiltriert und dreimal mit je 40 ml Wasser gewaschen. Nach dem Trocknen erhält man 3,26 g (73 %) der Titelverbindung, die bei 246 – 248°C schmilzt.

Elementaranalyse für $C_{25}H_{25}N_3O_3S$ (M = 447,5)

| | | | |
|---|---|---|---|
| berechnet, %: | C 67,10 | H 5,63 | N 9,39 |
| gefunden, %: | C 67,35 | H 6,00 | N 9,40. |

**Beispiel 3**

**11-Aminoapovincaminsäureäthylester**

a) 3,95 g (0,01 Mol) 11-Nitroapovincaminsäureäthylester werden in einem Gemisch aus 50 ml Äthanol und 10 ml konzentrierter Salzsäure mit 6 g Zinnstaub unter Rühren eine Stunde lang bei 100°C gehalten. Das Gemisch wird warm filtriert und die erhaltene Lösung im Vakuum zur Trockne eingedampft. Der Rückstand wird in 100 ml Wasser aufgenommen und mit n Natronlauge auf pH 10 alkalisch gestellt. Der ausgefallene Niederschlag wird abfiltriert, dreimal mit je 40 ml 1 %-iger Natronlauge und dreimal mit je 40 ml Wasser gewaschen. Nach dem Trocknen erhält man 2,80 g (77 %) der Titelverbindung, die bei 86 – 88°C schmilzt.

$^1$H-NMR ($CDCl_3$) δ: Et 1,00 t (3), 1,90 q (2), EtO 1,40 t (3), 4,40 q (2), $NH_2$ 3,23 (2), H-3 4,10 s (1), H-15 6,07 s (1). Gerüstprotonen 1,5 – 3,2 m.

b) Ein Gemisch aus 3,83 g (0,01 Mol) wie im Beispiel 5 beschrieben hergestelltem 11-Aminovincaminsäureäthylester, 7,0 g p-Toluolsulfonsäure und 150 ml Benzol wird unter Rückfluß unter einem Wasserabscheider 8 Stunden lang gekocht. Dann wird das Gemisch auf Raumtemperarur abgekühlt, mit 100 ml Wasser versetzt und mit konzentriertem wäßrigem Ammoniak auf pH 8 alkalisch gestellt. Die wäßrige Phase wird abgetrennt, die organische Phase wird mit je 100 ml Wasser dreimal gewaschen, über geglühtem Natriumsulfat getrocknet und nach dem Filtrieren im Vakuum zur Trockne eingedampft. Der Rückstand wird mit Äther verrieben, die entstehenden Kristalle werden abfiltriert, mit Äther gewaschen und dann getrocknet. Man erhalt 3,0 g (82 %) der Titelverbindung.

**Beispiel 4**

**11-(Methylsulfonylamino)-apovincaminsäureäthylester · HCl**

1,62 g (0,0044 Mol) 11-Aminoapovincaminsäureäthylester werden in 10 ml wasserfreiem Pyridin gelöst und zu der Lösung unter Kühlung 0,6 ml (0,008 Mol) Mesylchlorid in 10 ml Chloroform gegeben. Das Gemisch wird bei 0°C drei Stunden lang gerührt und dann im Vakuum zur Trockne eingedampft. Zu dem Rückstand werden 50 ml Äthylacetat, 25 ml gesättigte wäßrige Natriumbicarbonatlösung und 50 ml Wasser gegeben. Die Phasen werden voneinander getrennt, und die organische Phase wird zweimal mit je 30 ml Wasser gewaschen, dann getrocknet und im Vakuum zur Trockne eingedampft. Der Rückstand (1,87 g = 96 %) ist die Titelverbindung in Basenform. Sie wird in 15 ml Aceton gelöst und die Lösung mit salzsaurem Äthanol auf pH 3 angesäuert. Nach Zusatz von 45 ml Diisopropyläther fallen Kristalle aus, die abfiltriert, mit Äther gewaschen und dann getrocknet werden. Man erhält 1,56 g (74 %) der Titelverbindung, die bei 230 – 234°C schmilzt.

Elementaranalyse für $C_{23}H_{29}N_3O_4S \cdot HCl$ (M = 480,11)

| | | | | | |
|---|---|---|---|---|---|
| berechnet, %: | C 57,53 | H 6,29 | N 8,75 | S 6,67 | Cl 7,38 |
| gefunden, %: | C 57,42 | H 6,87 | N 8,56 | S 6,43 | Cl 7,40. |

**Beispiel 5**

**11-Aminovincaminsäureäthylester**

7,4 g (0,02 Mol) 11-Aminovincamin werden in 500 ml Äthanol in Gegenwart von 0,5 g Kalium-tert.-butylat zwei Stunden lang unter Rückfluß gekocht. Das Gemisch wird im Vakuum zur Trockne eingedampft und der Rückstand mit einem Gemisch aus 200 ml Wasser und 40 ml Äthanol verrieben. Die ausgefallenen Kristalle werden abfiltriert, und mit einem Gemisch aus 100 ml Wasser und 20 ml Äthanol in drei Portionen gewaschen. Man erhält 5,43 g (71 %) der Titelverbindung, die, bei 184 – 186°C schmilzt.

$^1$H-NMR ($CDCl_3$) δ: Et 0,85 t (3), $CH_2$ überdeckt, $H_2$-15 2,08 d (1), 2,16 d (1) ($J_{gem}$ = 14 Hz), $NH_2$ 3,80, $CH_2$-O 4,25 q (2), H-3 3,84 s (1), H-9 7,30 d (1), H-10 6,60 dd (1), H-12 6,41 d (1).

**Beispiel 6**

**9-Aminoapovincaminsäureäthylester-monohydrochlorid**

a) Ausgehend von 3,83 g (0,01 Mol) 9-Aminovincaminsäureäthylester werden auf die im Beispiel 3 beschriebene Weise 2,84 g (78 %) der Base der Titelverbindung hergestellt. Die Base wird in 20 ml Aceton gelöst, die Lösung mit salzsaurem Äthanol auf pH 3 angesäuert, und nach Zugabe von 20 ml Isopropyläther werden die Kristalle abfiltriert, mit Äther gewaschen und dann getrocknet. Man erhält 2,7 g (69 %) der Titelverbindung, die bei 218 – 222°C schmilzt.

Elementaranalyse für $C_{22}H_{27}N_3O_3 \cdot HCl$ (M = 401,96)

| | | | | |
|---|---|---|---|---|
| berechnet, %: | C 65,68 | H 6,71 | N 10,44 | Cl 8,83 |
| gefunden. %: | C 65,86 | H 6,90 | N 10,28 | Cl 8,78. |

b) 3,37 g (0,01 Mol) (-)-9-Aminoapovincaminsäure werden in 100 ml wasserfreiem Äthanol in Gegenwart eines Grammes Molekülsieb mit einer Porenweite von 0,3 nm (3 Å) (Aldrich 20,859-2) mit trockenem Salzsäuregas behandelt. Das Gemisch wird zuerst mit Salzsäuregas gesättigt, dann wird unter fortgesetztem Durchleiten von Salzsäuregas das Gemisch 3 Stunden lang am Rückfluß gekocht. Die Lösung wird zur Trockne eingedampft und der Rückstand mit 20 ml Äthylacetat versetzt. Die ausgefallenen Kristalle werden abfiltriert, mit Äthylacetat gewaschen und dann getrocknet. Man erhält 3,13 g (86 %) der Titelverbindung, die bei 218 – 222°C schmilzt.

**Beispiel 7**

**9-(Methylsulfonylamino)-apovincaminsäureäthylester · HCl**

Zu der Lösung von 1,20 g (0,0027 Mol) 9-Aminoapovincaminsäureäthylester · HCl in 15 ml wasserfreiem Pyridin werden unter Eiskühlung bei 0°C 0,37 ml (0,005 Mol) Mesylchlorid gegeben. Das Gemisch wird bei 0 – 5°C zwei Stunden lang gerührt und dann im Vakuum zur Trockne eingedampft. Der Rückstand wird in 50 ml Chloroform gelöst, und die Lösung wird zuerst mit 25 ml gesättigter wäßriger Natriumcarbonatlösung, dann dreimal mit je 30 ml Wasser gewaschen, über Natriumsulfat getrocknet und nach dem Filtrieren eingedampft. Man erhält 1,09 g (91 %) einer öligen Substanz, die die Basenform der Titelverbindung ist. Die Base wird in 40 ml Aceton gelöst und die Lösung mit salzsaurem Äthanol auf pH 4 angesäuert. Durch Zugabe von Äther fallen Kristalle aus, die abfiltriert, mit Äther gewaschen und dann getrocknet

werden. Ausbeute: 1,13 g (86 %), Schmp.: 189 – 192°C.

| | | | | | |
|---|---|---|---|---|---|
| Elementaranalyse für $C_{23}H_{29}N_3O_4S \cdot HCl$ (M = 480,11) | | | | | |
| berechnet, %: | C 57,53 | H 6,29 | N 8,75 | S 6,67 | Cl 7,38 |
| gefunden, %: | C 57,70 | H 6,80 | N 8,72 | S 6,40 | Cl 7,51 |

**Beispiel 8**

**9-Aminovincaminsäureäthylester**

Man geht von 7,4 g (0,02 Mol) 9-Aminovincamin aus und arbeitet auf die im Beispiel 5 beschriebene Weise. Ausbeute: 5,82 g (76 %), Schmelzpunkt: 232 – 234°C.

**Beispiel 9**

**11-(4-Nitrobenzoylamino)-apovincamin**

Zu der Lösung von 1,3 g (0,0037 Mol) 11-Aminoapovincamin in 10 ml wasserfreiem Pyridin werden bei Raumtemperatur 1,3 g (0,007 Mol) p-Nitrobenzoylchlorid gegeben. Das Gemisch wird bei Raumtemperatur drei Stunden lang gerührt und dann mit 100 ml Eiswasser und 150 ml Äthylacetat versetzt. Der pH-Wert wird mit n Natronlauge auf 8 eingestellt. Die organische Phase wird abgetrennt, viermal mit je 25 ml Wasser gewaschen, über geglühtem Natriumsulfat getrocknet und nach dem Filtrieren im Vakuum zur Trockne eingedampft. Der Rückstand wird mit 100 ml Äther verrieben. Die entstehenden Kristalle werden abfiltriert, mit Äther gewaschen und dann getrocknet. Man erhält 1,68 g (91 %) der Titelverbindung, die bei 206 – 208°C schmilzt.

$^1$H-NMR ($CDCl_3$) δ: Et 1,02 t (3), $CH_3O$ 3,98 s (1), NH 8,58 s (1), Ar-H 7,32 – 8,08 m (7), Gerüstprotonen 1,4 – 3,26.

**Beispiel 10**

**11-(2-Brombenzoylamino)-apovincaminsäuremethylester · HCl**

Zu einer Lösung von 3,51 g (0,01 Mol) 11-Aminoapovincamin in 40 ml wasserfreiem Benzol werden in Gegenwart von 2 ml Triäthylamin bei Raumtemperatur unter Rühren 5,12 g (0,023 Mol) o-Brombenzoylchlorid in 10 ml Benzol gegeben. Das Gemisch wird bei Raumtemperatur vier Stunden lang gerührt und dann im Vakuum zur Trockne eingedampft. Der Rückstand wird in einem Gemisch aus 100 ml Wasser und 50 ml Äthylacetat gelöst und die Lösung mit konzentriertem Ammoniak auf pH 8 gestellt. Die organische Phase wird abgetrennt und die wäßrige Phase zweimal mit je 50 ml Äthylacetat extrahiert. Die vereinigten organischen Phasen werden dreimal mit je 50 ml Wasser gewaschen, über geglühtem Natriumsulfat getrocknet und nach dem Filtrieren eingedampft. Man erhält 4,16 g (78 %) einer amorphen Substanz, die die Basenform der Titelverbindung darstellt.

$^1$H-NMR ($CDCl_3$) δ: Et 1,01 t (3), 1,90 q (2), H-3 4,14 s (1), $CH_3O$ 4,00 s (3), H-15 6,19 s (1), Ar-H+NH 7,05 – 7,90 m (8), Gerüstprotonen 0,8 – 3,4 m (10).

Die Base wird in 50 ml Aceton gelöst und der pH-Wert der Lösung mit salzsaurem Äthanol auf 3 eingestellt. Die durch Zugabe von Äther ausfallenden Kristalle werden abfiltriert, mit Äther gewaschen und dann getrocknet. Man erhält 3,93 g (69 %) der Titelverbindung, die bei 208 – 212°C schmilzt.

**Beispiel 11**

**11-(2-Fluorbenzoylamino)-vincamin**

Zu der Lösung von 2,1 g (0,015 Mol) o-Fluorbenzoesäure in 100 ml wasserfreiem Dimethylformamid werden bei 0°C unter Rühren 4,0 g (0,02 Mol) Dicyclohexylcarbodiimid gegeben. Das Gemisch wird 10 Minuten lang gerührt und dann mit 3,7 g (0,01 Mol) 11-Aminovincamin versetzt. Dann wird bei 0°C noch eine Stunde lang gerührt. Das Reaktionsgemisch wird über Nacht stehengelassen, der ausgeschiedene Dicyclohexylharnstoff abfiltriert und das Filtrat im Vakuum zur Trockne eingedampft. Der Rückstand wird in 400 ml Äthylacetat gelöst und die Lösung über Nacht in den Kühlschrank gestellt. Die auskristallisierten Reste des Dicyclohexylharnstoffes werden erneut abfiltriert. Das Filtrat wird viermal mit je 50 ml n Salzsäure, zweimal mit je 50 ml Wasser, dann dreimal mit je 50 ml gesättigter Natriumbicarbonatlösung und schließlich erneut zweimal mit je 50 ml Wasser gewaschen. Die organische Phase wird über geglühtem Natriumsulfat getrocknet und nach dem Filtrieren zur Trockne eingedampft. Der Rückstand wird in einem Gemisch aus

50 ml Äthylacetat und 250 ml Petroläther warm gelöst. Die bei Kühlung ausfallenden Kristalle werden abfiltriert, mit Petroläther gewaschen und dann getrocknet. Man erhält 3,8 g (78 %) der Titelverbindung, die bei 202 – 205°C schmilzt.

$^1$H-NMR ($CDCl_3$) δ: Et 0,90 t (3), $CH_3O$ 3,90 s (3), OH 4,76 s (1), Ar-H 7,05 – 8,70 m (7), Gerüstprotonen: 1,00 – 3,4 m (12).

**Beispiel 12**

**11-(3',4',5'-Trimethoxybenzoylamino)-vincamin**

Zu der Lösung von 1,85 g (0,05 Mol) 11-Aminovincamin in 75 ml wasserfreiem Pyridin werden bei Raumtemperatur unter Rühren 2,3 g (0,01 Mol) 3,4,5-Trimethoxybenzoylchlorid gegeben. Das Gemisch wird bei Raumtemperatur zwei Stunden lang gerührt und dann im Vakuum zur Trockne-eingedampft. Der Rückstand wird in einem Gemisch aus 50 ml Äthylacetat und 50 ml Wasser gelöst und der pH-Wert der Lösung mit konzentriertem Ammoniak auf 8 eingestellt. Die Phasen werden voneinander getrennt, die organische Phase wird dreimal mit je 25 ml Wasser gewaschen, getrocknet und zur Trockne eingedampft. Der Rückstand wird in 25 ml Methanol warm gelöst. Die Lösung wird mit 100 ml Diisopropyläther versetzt und über Nacht in den Kühlschrank gestellt. Die ausgefallenen Kristalle werden abfiltriert, mit Diisopropyläther gewaschen und dann getrocknet. Man erhält 2,13 g (76 %) der Titelverbindung, die bei 154 – 158°C schmilzt.

**Beispiel 13**

**11-Pivaloylaminovincamin**

Zu der Lösung von 0,74 g (0,002 Mol) 11-Aminovincamin in 30 ml wasserfreiem Pyridin werden bei 0°C unter Rühren 0,5 ml (0,004 Mol) Pivaloylchlorid gegeben. Das Gemisch wird bei der angegebenen Temperatur zwei Stunden lang gerührt, dann mit 20 ml Wasser verdünnt und mit konzentriertem Ammoniak auf pH 8 alkalisch gestellt. Die ausfallenden Kristalle werden abfiltriert, mit Wasser gewaschen und dann getrocknet. Man erhält 0,71 g (78 %) der Titelverbindung, die bei 140 – 144°C schmilzt.

$^1$H-NMR ($CDCl_3$) δ: Et 0,88 t (3), $CH_3$ 1,30 s (9), $CH_3O$ 3,87 s (1), H-3 3,90 s (1), OH 4,20 s (1), Ar-H+NH 6,97 – 7,65 m (4), Gerüstprotonen: 1,45 – 3,5 m.

**Beispiel 14**

**9-Benzoylaminovincamin**

Zu der Suspension von 5,52 g (0,015 Mol) 9-Aminovincamin, 150 ml wasserfreiem Benzol und 5 ml wasserfreiem Pyridin werden bei Raumtemperatur unter Rühren 3,5 ml (0,03 Mol) Benzoylchlorid in 20 ml Benzol gegeben. Das Gemisch wird bei Raumtemperatur drei Stunden lang gerührt und dann mit 60 ml Wasser sowie bis pH 8 mit konzentriertem Ammoniak versetzt. Die organische Phase wird abgetrennt, fünfmal mit je 20 ml Wasser gewaschen, über geglühtem Natriumsulfat getrocknet und dann zur Trockne eingedampft. Der Rückstand wird mit 50 ml Diisopropyläther verrieben. Die entstehenden Kristalle werden abfiltriert und mit Äther gewaschen. Man erhält 4,92 g (69 %) der Titelverbindung, die bei 152 – 156°C schmilzt.

$^1$H-NMR ($CDCl_3$) δ: Et 0,90 t (3), 1,65 q (2), $CH_3O$ 3,85 s (3), H-3 3,88 s (1), OH 4,75 s (1), NH 8,46 s (1), Ar-H 6,8 – 8,0 m (8).

**Beispiel 15**

**9-Acetamidovincamin**

Zu der Lösung von 6,6 g (0,018 Mol) 9-Aminovincamin in 600 ml Methanol werden bei 0°C 66 ml Essigsäureanhydrid gegeben. Das Reaktionsgemisch wird bei 0°C eine halbe Stunde lang gerührt und dann unter Rühren mit 800 g Eiswasser und 120 g Kaliumcarbonat versetzt. Das Methanol wird im Vakuum abdestilliert, und die zurückbleibende wäßrige Phase wird fünfmal mit je 100 ml Dichlormethan extrahiert. Die vereinigten organischen Phasen werden dreimal mit je 100 ml Wasser gewaschen, über geglühtem Natriumsulfat getrocknet und nach dem Filtrieren zur Trockne eingedampft. Der Rückstand wird mit 50 ml Äther versetzt. Die Kristalle werden mit 50 ml Äther gewaschen und dann getrocknet. Man erhält 5,92 g (80 %) der Titelverbindung, die bei 144 – 147°C schmilzt.

**Beispiel 16**

**11-(Thien-2'-ylcarbonylamino)-vincamin**

Zu der Lösung von 19,9 g (0,054 Mol) 11-Aminovincamin in 100 ml Pyridin werden unter Rühren bei 20°C 7,2 ml (0,067 Mol) Thiophen-2-carbonsäurechlorid in 20 ml Benzol gegeben. Das Gemisch wird bei der angegebenen Temperatur zwei Stunden lang gerührt und dann mit 50 ml gesättigter wäßriger Natriumbicarbonatlösung, mit 50 ml Wasser und schließlich mit 200 ml Äthylacetat versetzt. Dann werden die Phasen voneinander getrennt, die organische Phase wird zweimal mit je 30 ml Wasser gewaschen, über geglühtem Natriumsulfat getrocknet und nach dem Filtrieren, im Vakuum zur Trockne eingedampft. Der Rückstand wird mit 200 ml Äther versetzt, die ausgefallenen Kristalle werden abfiltriert und viermal mit je 50 ml Äther gewaschen. Man erhält 18,4 g (71 %) der Titelverbindung, die bei 229 – 231°C schmilzt.

**Beispiel 17**

**11-(Thien-2'-ylacetylamino)-vincamin**

Zu der Lösung von 0,3 g (0,0022 Mol) Thiophen-2-essigsäure in 10 ml wasserfreiem Dimethylformamid werden 0,45 g Dicyclohexylcarbodiimid gegeben. Die Lösung wird bei Raumtemperatur 15 Minuten lang gerührt und dann mit 0,74 g (0,002 Mol) 11-Aminovincamin versetzt. Das Gemisch wird bei Raumtemperatur 24 Stunden lang gerührt und dann über Nacht in den Kühlschrank gestellt. Der auskristallisierte Dicyclohexylharnstoff wird abfiltriert und das Filtrat im Vakuum zur Trockne eingedampft. Der Rückstand wird in 30 ml Äthylacetat gelöst und die Lösung zuerst mit 10 ml gesättigter wäßriger Natriumbicarbonatlösung, dann dreimal mit je 10 ml Wasser gewaschen. Die organische Phase wird über geglühtem Natriumsulfat getrocknet und nach dem Filtrieren im Vakuum zur Trockne eingedampft. Der Rückstand wird mit 20 ml Äther versetzt, die ausgefallenen Kristalle werden abfiltriert, mit Äther gewaschen und dann getrocknet. Man erhält 0,51 g (52 %) der Titelverbindung, die bei 153 – 155°C schmilzt.

$^1$H-NMR ($CDCl_3$) δ: Et 0,90 t (3), 1,7 q (2), $CH_3O$ 3,88 s (3), $CH_2CO$ 3,94 s (2), H-3 4,00 s (1), OH 4,70(1), Ar-H 6,80 – 7,65 m (6), CONH 7,48 (1).

**Beispiel 18**

**9-Acetamidoapovincamin**

Die Lösung von 5,6 g (0,014 Mol) 9-Acetamidovincamin in 300 ml Benzol wird in Gegenwart von 6,8 g p-Toluolsulfonsäure unter einem Wasserabscheider 4 Stunden lang unter Rückfluß gekocht. Das Gemisch wird gekühlt, mit 200 ml Wasser versetzt, der pH-Wert der wäßrigen Phase mit konzentriertem Ammoniak auf 8 eingestellt und dann extrahiert. Die organische Phase wird viermal mit je 100 ml Wasser gewaschen, über geglühtem Natriumsulfat getrocknet und dann im Vakuum zur Trockne eingedampft. Der Rückstand wird in 50 ml Äthylacetat heiß gelöst, die Lösung gekühlt, und die ausgefallenen Kristalle werden filtriert und mit Äthylacetat gewaschen. Man erhält 5,0 g (91 %) der Titelverbindung, die bei 138 – 140°C schmilzt.

$^1$H-NMR ($CDCl_3$) δ: Et 0,99 t (3), $CH_3CO$ 2,16 s (3), $CH_3O$ 2,16 s (3), $CH_3O$ 3,80 s (3), H-3 4,05 s (1), H-15 6,18 s (1), Ar-H 7,0 – 7,65 m (3).

**Beispiel 19**

**11-(3',4',5'-Trimethoxybenzoylamino)apovincamin**

1,3 g (0,0023 Mol) 11-(3',4',5'-Trimethoxybenzoylamino)-vincamin und 1,0 p-Toluolsulfonsäure werden in 50 ml Benzol unter einem Wasserabscheider zwei Stunden lang unter Rückfluß gekocht. Nach dem Abkühlen wird das Gemisch mit 20 ml Wasser versetzt und mit konzentriertem Ammoniak auf pH 8 alkalisch gestellt. Die Phasen werden voneinander getrennt, die organische Phase wird dreimal mit je 20 ml Wasser gewaschen, über geglühtem Natriumsulfat getrocknet und nach dem Filtrieren im Vakuum zur Trockne eingedampft. Der Rückstand wird in 3 ml Aceton heiß gelöst, die Lösung mit 15 ml Petroläther versetzt und dann abgekühlt. Die ausgefallenen Kristalle werden abfiltriert, mit Äther gewaschen und dann getrocknet. Man erhält 1,03 g (83 %) der Titelverbindung, die bei 150 – 152°C schmilzt.

$^1$H-NMR ($CDCl_3$) δ: Et 1,00 t (3), $CH_3OCO$ 3,98 s (3), $CH_3O$ 3,90 s (9), H-3 4,01 s (1), H-15 6,28 s (1), Ar-H 7,1 – 7,8 m (5), NHCO 8,30 s (1).

**Beispiel 20**

**11-Pivaloylaminoapovincamin**

0,68 g (0,0015 Mol) 11-Pivaloylaminovincamin werden in 20 ml Benzol in Gegenwart von 0,70 g wasserfreier p-Toluolsulfonsäure unter einem Wasserabscheider drei Stunden lang unter Rückfluß gekocht. Das Gemisch wird gekühlt, mit 20 ml Wasser versetzt und dann mit konzentriertem Ammoniak auf pH 8 alkalisch gestellt. Die organische Phase wird abgetrennt, mit je 10 ml Wasser viermal gewaschen, über geglühtem Natriumsulfat getrocknet und nach dem Filtrieren im Vakuum zur Trockne eingedampft. Der Rückstand wird in 25 ml heißem Hexan unter Zugabe einiger Tropfen Aceton gelöst. Die Lösung wird gekühlt, die ausfallenden Kristalle werden abfiltriert, mit Hexan gewaschen und dann getrocknet. Man erhält 0,44 g (68 %) der Titelverbindung, die bei 110 – 114°C schmilzt.

$^1$H-NMR ($CDCl_3$) δ: Et 0,97 t (3), 1,92 q (2), $CH_3$ 1,35 s (9), $CH_3O$ 3,93 s (1), H-3 4,07 s (1), Ar-H + NH 7,0 – 7,68 m (4).

**Beispiel 21**

**11-Acetamidoapovincamin**

18,1 g (0,044 Mol) 11-Acetamidovincamin in 900 ml Benzol werden in Gegenwart von 22 g p-Toluolsulfonsäure unter einem Wasserabscheider fünf Stunden lang unter Rückfluß gekocht. Das Gemisch wird gekühlt, mit 400 ml Wasser versetzt und mit konzentriertem Ammoniak auf pH 8 alkalisch gestellt. Die benzolische Phase wird dreimal mit je 300 ml Wasser gewaschen, über geglühtem Natriumsulfat getrocknet und nach dem Filtrieren im Vakuum zur Trockne eingedampft. Der Rückstand wird mit 50 ml Diisopropyläther gewaschen und dann getrocknet. Man erhält 14,35 g (83 %) der Titelverbindung, die bei 136 – 140°C schmilzt.

$^1$H-NMR ($CDCl_3$) δ: Et 0,98 t (3), 1,83 q (2), $CH_3CO$ 2,08 s (3), $CH_3O$ 3,90 s (3), H-3 4,05 s (1), H-15 6,06 s (1), Ar-H + NH 6,97 – 7,58 m (4).

**Beispiel 22**

**(+)-9-(Benzoylamino)-vincaminsäureäthylester**

Ein Gemisch aus 1,89 (0,004 Mol) (+)-9-(Benzoylamino)-vincamin, 30 ml wasserfreiem Äthanol und 0,1 g Kalium-tert.-butylat wird eine Stunde lang unter Rückfluß gekocht und dann zur Trockne eingedampft. Der Rückstand wird in 50 ml Äthylacetat gelöst und die Lösung dreimal mit je 15 ml Wasser gewaschen. Die organische Phase wird über geglühtem Natriumsulfat getrocknet und nach dem Filtrieren im Vakuum zur Trockne eingedampft. Der Rückstand wird mit Äther verrieben, die entstehenden Kristalle werden abfiltriert, mit Äther gewaschen und dann getrocknet. Man erhält 1,32 g (68 %) der Titelverbindung, die bei 135 – 137°C schmilzt.

$[\alpha]_D$ = +44,82° (c = 1, Dichlormethan).

**Beispiel 23**

**(+)-9-(Benzoylamino)-apovincaminsäureäthylester**

Ein Gemisch aus 1,22 g (0,0025 Mol) (+)-9-(Benzoylamino)-vincaminsäureäthylester, 1,3 g p-Toluolsulfonsäure und 50 ml Benzol wird unter einem Wasserabscheider zwei Stunden lang unter Rückfluß gekocht. Dann wird das Gemisch gekühlt, mit 50 ml Wasser versetzt und mit konzentriertem Ammoniak auf pH 8 alkalisch gestellt. Die organische Phase wird abgetrennt, viermal mit je 20 ml Wasser gewaschen, über geglühtem Natriumsulfat getrocknet und nach dem Filtrieren im Vakuum zur Trockne eingedampft. Der Rückstand kristallisiert bei Verreiben mit 10 ml Hexan. Die Kristalle werden abfiltriert, mit Hexan gewaschen und dann getrocknet. Man erhält 0,93 g (79 %) der Titelverbindung, die bei 112 – 116°C schmilzt. $[\alpha]_D$ = +186,96° (c = 1, Dichlormethan)

$^1$H-NMR ($CDCl_3$) δ: Et 1,00 t (3), 1,90 q (2), EtO 1,39(3), 4,30 q (2), H-3 4,0 s (1), H-15 6,17 s (1), Ar-H 7,0 – 7,98 m (8), NH 7,30 s (1).

**Beispiel 24**

**11-Acetamidoapovincaminsäure-(2'-hydroxyäthyl)-ester**

Ein Gemisch aus 2,13 g (0,0054 Mol) 11-Acetamidoapovincamin, 70 ml Äthylenglykol und 0,1 g Kalium-tert.-butylat wird bei 120°C 90 Minuten lang gerührt. Dann wird der Überschuß des Äthylenglykols im Vakuum abdestilliert. Der Rückstand wird in 50 ml Äthylacetat gelöst und die Lösung dreimal mit je 20 ml Wasser gewaschen. Die organische Phase wird über geglühtem Natriumsulfat getrocknet und nach dem Filtrieren im Vakuum zur Trockne eingedampft. Der Rückstand wird mit 30 ml Äther verrieben, die erhaltenen Kristalle werden abfiltriert, mit Äther gewaschen und dann getrocknet. Man erhält 1,47 g (64 %) der Titelverbindung, die bei 146 – 150°C schmilzt.

$^1$H-NMR ($CDCl_3$) δ: Et 0,98 t (3), 1,82 q (2), $CH_3CO$ 2,16 s (3), H-3 und $CH_2OCO$ 3,85 m (3), $OCH_2$ 4,52 m (2), H-15 6,04, Ar-H 6,7 – 7,8 m (3), NH 7,36 s (1).

**Beispiel 25**

**(+)-11-(Nicotinoylamino)-apovincamin**

Zu der Lösung von 4,0 g (0,011 Mol) (+)-11-Aminoapovincamin in 80 ml Pyridin wird bei Raumtemperatur unter Rühren die Lösung von 11,3 g Nicotinsäurechlorid in 100 ml wasserfreiem Benzol gegeben. Das Gemisch wird bei Raumtemperatur 8 Stunden lang gerührt und über Nacht stehengelassen. Anderntags wird das Gemisch im Vakuum zur Trockne eingedampft, der Rückstand in 100 ml Wasser gelöst, die Lösung mit konzentriertem Ammoniak auf pH 8 gestellt, die ausgefallene Substanz abfiltriert, mit Wasser gewaschen und dann getrocknet. Man erhält 4,0 g (80 %) der Titelverbindung, die bei 148 – 152°C schmilzt.

$^1$H-NMR ($CDCl_3$) δ: Et 1,00(3), 1,90 q (2), $CH_3O$ 3,90 s (1), H-15 6,06 s (1), Ar-H + NH 7,2 – 8,93 m (8).
$[\alpha]_D$ = +17,00° (c = 1, Dichlormethan).

**Beispiel 26**

**(-)-11-(2-Fluorbenzoylamino)-apovincamin**

Ein Gemisch aus 9,5 g (0,019 Mol) (-)-11-(2-Fluorbenzoylamino)-vincamin, 20 g p-Toluolsulfonsäure und 500 ml Benzol wird unter einem Wasserabscheider zwei Stunden lang unter Rückfluß gekocht. Das Gemisch wird gekühlt, mit 200 ml Wasser versetzt und mit konzentriertem Ammoniak auf pH 8 alkalisch gestellt. Die organische Phase wird viermal mit je 100 ml Wasser gewaschen, über geglühtem Natriumsulfat getrocknet und nach dem Filtrieren im Vakuum zur Trockne eingedampft. Der Rückstand wird aus 300 ml Diisopropyläther kristallisiert. Man erhält 7,14 g (86 %) der Titelverbindung, die bei 102 – 105°C schmilzt.

$[\alpha]_D$ = –2,40° (c = 1, Dichlormethan)
$^1$H-NMR ($CDCl_3$) δ: Et 1,02 t (3), 1,95 q (2), $CH_3O$ 4,01 s (1), H-3 4,30 s (1), H-15 6,18 s (1), Ar-H 6,95 – 8,00 m (6), H-12 7,92 d (1), 7,14 dd und 8,22 dd (1), NH 8,45 und 8,62 s (1), Gerüstprotonen: 1,00 – 3,4 m (10).

**Beispiel 27**

**11-(Thien-2'-ylcarbonylamino)-apovincamin**

a) Ein Gemisch aus 15,1 g (0,031 Mol) 11-(Thien-2'-ylcarbonylamino)-vincamin, 400 ml Benzol und 17 g p-Toluolsulfonsäure wird unter einem Wasserabscheider zwei Stunden lang unter Rückfluß gekocht. Nach dem Abkühlen wird das Gemisch mit 100 ml Wasser versetzt und mit konzentriertem Ammoniak auf pH 8 alkalisch gestellt. Die organische Phase wird abgetrennt, fünfmal mit je 50 ml Wasser gewaschen, über geglühtem Natriumsulfat getrocknet und nach dem Filtrieren im Vakuum zur Trockne eingedampft. Der Rückstand wird aus 100 ml Diisopropyläther kristallisiert. Man erhält 14,0 g (96 %) der Titelverbindung, die bei 138 – 140°C schmilzt.

$^1$H-NMR ($CDCl_3$) δ: Et 1,02 t (3), $CH_3O$ 3,98 s (3), H-3 4,03 s (1), H-15 6,10 s (1), Ar-H 7,0 – 7,74 m (6), NH 8,05 s (1).

b) 6,7 g (0,015 Mol) 11,-(Thien-2 '-ylcarbonylamino)-apovincaminsäure werden in einem Gemisch aus 20 ml Aceton und 15 ml n Natronlauge gelöst und zu der Lösung bei Raumtemperatur unter Rühren 1,5 ml Dimethylsulfat gegeben. Das Gemisch wird 90 Minuten lang gerührt, dann mit 0,5 ml konzentriertem Ammoniak versetzt und im Vakuum auf die Hälfte seines Volumens eingeengt. Die ausgefallene Substanz wird abfiltriert, zweimal mit je 10 ml Wasser gewaschen

und dann getrocknet. 6,5 g (95 %) beträgt die Ausbeute an der Titelverbindung, deren physikalische Daten mit denen des gemäß a) hergestellten Produktes übereinstimmen.

**Beispiel 28**

**11-(Benzoylamino)-apovincamin**

Eine Lösung von 4,4 g (0,01 Mol) 11-Benzoylaminoapovincaminsäure in 100 ml wasserfreiem Methanol wird in Gegenwart eines Grammes Molekülsieb mit einer Porengröße von 3 Å (Aldrich 20,859-2) mit trockenem Salzsäuregas gesättigt. Unter fortgesetztem Durchleiten von Salzsäuregas wird das Gemisch unter Rückfluß fünf Stunden lang gekocht. Dann wird die Lösung zur Trockne eingedampft. Der Rückstand wird in einem Gemisch aus 100 ml Äthylacetat und 50 ml Wasser gelöst und der pH-Wert der Lösung mit konzentriertem Ammoniak auf 8 eingestellt. Die organische Phase wird abgetrennt, zweimal mit je 30 ml Wasser gewaschen und über geglühtem Natriumsulfat getrocknet. Die Lösung wird im Vakuum zur Trockne eingedampft und der Rückstand aus einem im Volumverhältnis 1 : 1 bereiteten Gemisch von Diisopropyläther und Hexan (insgesamt 40 ml) umkristallisiert. Man erhält 3,9 g (86 %) der Titelverbindung, die bei 129 – 132°C schmilzt.

$^1$H-NMR ($CDCl_3$) δ: Et 1,00 t (3), $CH_3O$ 4,00 s (3), H-3 4,10 s (1), H-15 6,12 s (1), Ar-H + NH 7,08 – 8,15 m (9).

**Beispiel 29**

**11-(Benzoylamino)-apovincaminsäure-(2'-hydroxyäthyl)-ester**

Ein Gemisch aus 0,45 g (0,001 Mol) 11-(Benzoylamino)-apovincamin, 10 ml Äthylenglykol und 0,01 g Kalium-tert.-butylat wird zwei Stunden lang auf 120°C gehalten und dann im Vakuum zur Trockne eingedampft. Der Rückstand wird in 25 ml Wasser gelöst und die Lösung zweimal mit je 30 ml Äthylacetat extrahiert. Die organische Phase wird zweimal mit je 10 ml Wasser gewaschen, über geglühtem Natriumsulfat getrocknet und nach dem Filtrieren im Vakuum zur Trockne eingedampft. Der Rückstand wird aus 8 ml Diisopropyläther kristallisiert. Man erhält 0,38 g (78 %) der Titelverbindung, die bei 136 – 138°C schmilzt.

$^1$H NMR ($CDCl_3$) δ: Et 0,98 t (3), H-3 3,68 s (1), $CH_2OCO$ 4,55 m (2), HO-$CH_2$ 3,80 m (2), H-15 6,03 s (1), Ar-H 6,98 – 7,96 m (8), NH 8,32.

**Beispiel 30**

**11-(Benzoylamino)-apovincaminsäure-(2-methylpropyl)-ester-monohydrochlorid**

Ein Gemisch aus 0,8 g (0,0018 Mol) 11-(Benzoylamino)-apovincamin, 20 ml wasserfreiem Isobutanol und 0,01 g Kalium-tert.-butylat wird zwei Stunden lang unter Rückfluß gekocht und dann im Vakuum zur Trockne eingedampft. Der Rückstand wird in 40 ml Dichloräthan gelöst und die Lösung viermal mit je 5 ml Wasser extrahiert. Die organische Phase wird über geglühtem Natriumsulfat getrocknet und nach dem Filtrieren zur Trockne eingedampft. Man erhält 0,83 g (93 %) der Titelverbindung in Form der Base. Die Base wird in 15 ml Äther gelöst, die Lösung mit salzsaurem Äthanol auf pH 3 angesäuert, das kristalline Hydrochlorid abfiltriert, mit Äther gewaschen und dann getrocknet. Man erhält 0,69 g (72 %) der Titelverbindung, die bei 185 – 186°C schmilzt.

Elementaranalyse für $C_{31}H_{35}N_3O_3 \cdot HCl$ (M = 534,07)

| | | | | |
|---|---|---|---|---|
| berechnet, %: | C 69,71 | H 6,79 | N 7,86 | Cl 6,63 |
| gefunden, %: | C 69,86 | H 7,00 | N 7,84 | Cl 6,70. |

**Beispiel 31**

**(+)-11-Aminovincaminsäure**

Zu der Lösung von 2,5 g (0,007 Mol) (+)-11-Aminovincamin in 100 ml Äthanol wird die Lösung von 2,5 g Kaliumhydroxid in 20 ml Wasser gegeben. Das Gemisch wird eine halbe Stunde lang unter Rückfluß gekocht, dann auf Raumtemperatur gekühlt und mit 10 %-ger Salzsäure auf pH 7 gestellt. Die ausgefallenen Kristalle werden abfiltriert, zweimal mit je 10 ml kaltem, 50 %-igem Äthanol gewaschen und dann getrocknet. Man erhält 2,0 g (83 %) der Titelverbindung, die bei 255 – 256°C schmilzt.

$[\alpha]_D$ = +32,4° (c = 1, Pyridin)

**Beispiel 32**

**(+)-11-(2-Fluorbenzoylamino)-vincaminsäure**

Ein Gemisch aus 0,30 g (0,0006 Mol) (+)-11-(2-Fluorbenzoylamino)-vincamin. 5 ml Äthanol und 3 ml 10 %-iger Kalilauge wird bei 35°C eine halbe Stunde lang gerührt. Dann wird das Gemisch mit 10 %-iger Salzsäure auf pH 7 neutralisiert. Die ausgefallenen Kristalle werden abfiltriert, dreimal mit je 5 ml Wasser gewaschen und dann getrocknet. Man erhält 0,25 g (85 %) der Titelverbindung, die bei 247 – 249°C schmilzt.

$[\alpha]_D$ = +18,6° (c = 1, Pyridin)

**Beispiel 33**

**11-Benzoylaminoapovincaminsäure**

3,67 g (0,001 Mol) 11-Nitroapovincaminsäure werden in einem Gemisch aus 5 ml Äthanol und 10 ml n Natronlauge gelöst. Die Lösung wird mit 0,04 g 10 %-iger Palladiumaktivkohle versetzt und bei Raumtemperatur in einer Wasserstoffatmosphäre gerührt. Als 0,03 Mol (720 ml) Wasserstoff aufgenommen waren, wird der Katalysator abfiltriert und dreimal mit je 2 ml 50 %-igem wäßrigem Äthanol gewaschen. Zu dem mit der Waschflüssigkeit vereinigten Filtrat wird bei Raumtemperatur tropfenweise die Lösung von 3 ml Benzoylchlorid in 30 ml Aceton gegeben. Der pH-Wert des Reaktionsgemisches wird durch Zugabe von n Natronlauge zwischen 7 und 8 gehalten. Das Gemisch wird bei Raumtemperatur zwei Stunden lang gerührt und dann auf pH 7 neutralisiert. Die ausgefallenen Kristalle werden abfiltriert, mit Wasser gewaschen und dann getrocknet. Man erhält 3,88 g (88 %) der Titelverbindung, deren physikalische Konstanten mit denen der gemäß Beispiel 1 hergestellten Verbindung übereinstimmen.

**Beispiel 34**

**11-(Cyclohexylcarbonylamino)-vincamin**

Zu der Lösung von 1,85 g (0,005 Mol) 11-Aminovincamin in 80 ml wasserfreiem Pyridin wird bei Raumtemperatur innerhalb einer Stunde tropfenweise die Lösung von 1,46 g (0,01 Mol) Cyclohexancarbonsäurechlorid in 20 ml wasserfreiem Benzol gegeben. Das Gemisch wird bei Raumtemperatur zwei Stunden lang gerührt und dann im Vakuum zur Trockne eingedampft. Der Rückstand wird in einem Gemisch aus 50 ml Äthylacetat und 50 ml Wasser gelöst, der pH-Wert der Lösung mit konzentriertem Ammoniak auf 8 eingestellt, und die Phasen werden voneinander getrennt. Die organische Phase wird dreimal mit je 25 ml Wasser gewaschen, getrocknet und auf 1/3 ihres Volumens eingedampft und über Nacht in den Kühlschrank gestellt. Die Kristalle werden abfiltriert, mit kaltem Äthylacetat gewaschen und dann getrocknet. Man erhält 1,67 g (70 %) der Titelverbindung, die bei 170 – 174°C schmilzt.

**Beispiel 35**

**11-(3'-Brompropionylamino)-apovincamin**

Zu 2,0 g (5,5 mMol) 11-Aminoapovincamin in 50 ml wasserfreiem Pyridin wird unter Rühren und Eiskühlung innerhalb einer Stunde die Lösung von 1,71 g (0,01 Mol) 3-Brompropionylchlorid in 30 ml wasserfreiem Benzol gegeben. Das Gemisch wird bei Raumtemperatur zwei Stunden lang gerührt und dann im Vakuum eingedampft. Der Rückstand wird in einem Gemisch aus 50 ml Äthylacetat und 50 ml Wasser gelöst, der pH-Wert mit konzentriertem Ammoniak auf 8 eingestellt und die organische Phase abgetrennt. Die Lösung wird getrocknet und anschließend eingedampft. Der Rückstand wird in 10 ml Methanol gelöst und mit Äther ausgefällt. Der Niederschlag wird abfiltriert, mit Äther gewaschen und dann getrocknet. Man erhält 1,5 g (62 %) der Titelverbindung, die bei 175 – 180°C schmilzt.

**Beispiel 36**

**11-Cyclohexylcarbonylaminoapovincamin**

Ein Gemisch aus 4,79 g (0,01-Mol) 11-Cyclohexylcarbonylaminovincamin (Beispiel 34), 220 ml Benzol und 5,5 g p-Toluolsulfonsäure wird unter einem Wasserabscheider unter Rückfluß 6 Stunden lang gekocht. Das Gemisch wird gekühlt, mit 200 ml Wasser versetzt und sein pH-Wert mit konzentriertem Ammoniak auf 8 eingestellt. Die organische Phase wird viermal mit je 100 ml Wasser gewaschen, über geglühtem Natriumsulfat getrocknet und nach dem Filtrieren im Vakuum eingedampft. Der Rückstand wird mit Äther verrieben, die entstandenen Kristalle werden abfiltriert, mit Äther gewaschen und dann getrocknet. Man erhält 4,0 g (87 %) der Titelverbindung, die bei 130 – 135°C schmilzt.

**Beispiel 37**

**11-(Thien-2'-ylcarbonylamino)-apovincaminsäureäthylesterhydrochlorid**

Es wurde analog wie im Beispiel 27, Verfahrensweisen a) und b) beschrieben vorgegangen, jedoch von 11-(Thien-2'-ylcarbonylamino)-vincaminsäureäthylester beziehungsweise 11-(Thien-2'-ylcarbonylamino)-apovincaminsäure und Diäthylsulfat ausgegangen. Die erhaltene Base wurde in der im Beispiel 7 angegebenen Weise in ihr Hydrochlorid (Titelverbindung) überführt. Schmelzpunkt des letzteren: 197 bis 202°C.

**Beispiel 38**

**11-(Benzoylamino)-apovincaminsäureäthylesterhydrochlorid**

Es wurde analog wie im Beispiel 28 beschrieben vorgegangen, jedoch von 11-(Benzoylamino)-apovincaminsäure und Äthanol ausgegangen. Die erhaltene Base wurde in der im Beispiel 7 angegebenen Weise in ihr Hydrochlorid (Titelverbindung) überführt. Schmelzpunkt des letzteren: 196 bis 200°C.

**Beispiel 39**

**11-(Acetamido)-apovincaminsäureisobutylesterhydrochlorid**

Es wurde analog wie im Beispiel 30 beschrieben vorgegangen, jedoch von 11-(Acetamido)-apovincamin ausgegangen. Die erhaltene Base wurde in der im Beispiel 7 angegebenen Weise in ihr Hydrochlorid (Titelverbindung) überführt. Schmelzpunkt des letzteren: 185 bis 189°C.

**Beispiel 40**

**9-(Acetamido)-apovincaminsäureäthylesterhydrochlorid**

Es wurde analog wie im Beispiel 18 beschrieben vorgegangen, jedoch von 9-(Acetamido)-vincaminsäureäthylester ausgegangen. Die erhaltene Base wurde in der im Beispiel 7 angegebenen Weise in ihr Hydrochlorid (Titelverbindung) überführt. Schmelzpunkt des letzteren: 198 bis 201°C.

**Patentansprüche** für die Vertragsstaaten: BE, CH, DE, FR, GB, IT, LI, NL, SE

1. In der 9- oder 11-Stellung eine, gegebenenfalls substituierte, Aminogruppe aufweisende Eburnancarbonsäurederivate der allgemeinen Formel

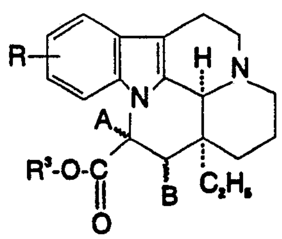

I,

worin

R für eine Aminogruppe

oder einen Acylamidorest der allgemeinen Formel

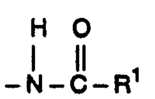

II,

in welchletzterer

$R^1$ Wasserstoff oder einen aliphatischen Kohlenwasserstoffrest mit 1 bis 10 Kohlenstoffatom(en), cycloaliphatischen Kohlenwasserstoffrest mit 3 bis 8 Kohlenstoffatomen, aromatischen Kohlenwasserstoffrest mit 6 bis 14 Kohlenstoffatomen oder einen 1 oder mehr gleiche oder verschiedene aus Stickstoff, Sauerstoff und/oder Schwefel bestehende Heteroatome aufweisenden 5- bis 7-gliedrigen Heteroarylrest, gegebenenfalls zusätzlich zu diesen Gliedern mit 4 zur Bildung eines Benzolrings ankondensierten Gliedern bedeutet, wobei die vorstehend genannten Reste mit einem oder mehreren gleichen oder verschiedenen Substituenten substituiert sein können, bestehend aus Halogenatomen, primären, sekundären und tertiären Aminogruppen, Hydroxygruppen, Arylresten mit 6 bis 14 Kohlenstoffatomen, Alkyl-, Alkoxy- und Alkylthioresten mit 1 bis 8 Kohlenstoffatomen, Trifluormethylresten, Carboxylgruppen, Alkoxycarbonylresten mit 1 bis 8 Kohlenstoffatomen im Alkylteil, Thio-, Sulfinyl-, Sulfonyl-, Nitro-, Keto-, Aldehyd- und Cyangruppen und 1 oder mehr aus Stickstoff, Sauerstoff und/oder Schwefel als Heteroatome aufweisende 5- bis 7-gliedrige Heteroarylresten, gegebenenfalls zusätzlich zu diesen Gliedern mit 4 zur Bildung eines Benzolrings ankondensierten Gliedern, vor allem Pyrrolyl-, Furyl-, Thienyl-, Pyridyl-, Pyranyl-, Pyrazolyl-, Imidazolyl-, Pyrimidinyl-, Indolyl- und Chinolylresten, die gegebenenfalls durch 1 oder mehr der aufgeführten Substituenten substituiert sein können,

oder

einen Sulfonamidorest der allgemeinen Formel

$$-\overset{\overset{\displaystyle H}{|}}{N}-\underset{O_2}{S}-R^2 \qquad \text{III,}$$

in welchletzterer

$R^2$ einen aliphatischen Kohlenwasserstoffrest mit 1 bis 10 Kohlenstoffatom(en) oder einen aromatischen Kohlenwasserstoffrest mit 6 bis 14 Kohlenstoffatomen bedeutet, wobei diese Reste durch einen oder mehrere gleiche oder verschiedene Substituenten, wie sie im Zusammenhang mit $R^1$ aufgeführt sind, substituiert sein können, steht,

$R^3$ Wasserstoff oder einen aliphatischen Kohlenwasserstoffrest mit 1 bis 10 Kohlenstoffatom(en), cycloaliphatischen Kohlenwasserstoffrest mit 3 bis 8 Kohlenstoffatomen oder aromatischen Kohlenwasserstoffrest mit 6 bis 14 Kohlenstoffatomen bedeutet, wobei diese Reste mit 1 oder mehr gleichen oder verschiedenen Substituenten, wie sie im Zusammenhang mit $R^1$ definiert worden sind, substituiert sein können, und

A für eine Hydroxygruppe steht und

B Wasserstoff bedeutet oder

A und B zusammen eine chemische Bindung darstellen und

R in der 9- oder 11-Stellung ist, mit den weiteren Maßgaben, daß,

a) im Falle daß

R für eine Aminogruppe steht,

$R^3$ von einem nicht substituierten Methylrest verschieden ist, und

b) im Falle daß

R für einen in der 9- oder 11-Stellung befindlichen Acylamidorest der allgemeinen Formel II,

bei welchem

$R^1$ einen Methylrest bedeutet,

steht,

A eine Hydroxygruppe bedeutet und

B Wasserstoff darstellt,

$R^3$ von einem nicht substituierten Methylrest verschieden ist,

sowie ihre optisch aktiven Isomere und Salze.

2. Eburnancarbonsäurederivate nach Anspruch 1, dadurch gekennzeichnet, daß der beziehungsweise die aliphatische(n) Kohlenwasserstoffrest(e), für welche[n] $R^1$ oder $R^2$ und/oder $R^3$ stehen kann beziehungsweise können, [ein] Alkylrest(e), und/oder [ein] Alkenyl- und/oder Alkinylrest(e) ist beziehungsweise sind.

3. Eburnancarbonsäurederivate nach Anspruch 1 oder 2, dadurch gekennzeichnet, daß der beziehungsweise die aliphatische(n) Kohlenwasserstoffrest(e), für welche[n] $R^1$ oder $R^2$ und/oder $R^3$ stehen kann beziehungsweise können, [ein] solche[r] mit 1 bis 8 Kohlenstoffatomen und der beziehungsweise die Alkenyl- und/oder Alkinylrest(e) [ein] solche[r] mit 2 bis 4 Kohlenstoffatomen ist beziehungsweise sind.

4. Eburnancarbonsäurederivate nach Anspruch 1 bis 3, dadurch gekennzeichnet, daß der beziehungsweise die cycloaliphatische(n) Kohlenwasserstoffrest(e), für welche[n] $R^1$ und/oder $R^3$ stehen kann beziehungsweise können, [ein] Cycloalkylrest(e) ist beziehungsweise sind.

5. Eburnancarbonsäurederivate nach Anspruch 1 bis 3, dadurch gekennzeichnet, daß der beziehungsweise die Cycloalkylrest(e) für welche[n] $R^1$ und/oder $R^3$ stehen kann beziehungsweise können, [ein] solche[r] mit 5 bis 7 Kohlenstoffatomen ist beziehungsweise sind.

6. Eburnancarbonsäurederivate nach Anspruch 1 bis 5, dadurch gekennzeichnet, daß der beziehungsweise die aromatische(n) Kohlenwasserstoffrest(e), für welche[n] $R^1$ oder $R^2$ und/oder $R^3$ stehen kann beziehungsweise können, monocyclisch, nämlich [ein] Phenylrest(e), und/oder polycyclisch nicht kondensiert und/oder polycyclisch kondensiert ist beziehungsweise sind.

7. Eburnancarbonsäurederivate nach Anspruch 1 bis 6, dadurch gekennzeichnet, daß der beziehungsweise die aromatischen Kohlenwasserstoffrest(e), für welche[n] $R^1$ oder $R^2$ und/oder $R^3$ stehen kann beziehungsweise können, soweit er beziehungsweise sie polycyclisch ist beziehungsweise sind, [ein] Diphenylrest(e) und/oder Naphthylrest(e) ist beziehungsweise sind.

8. Eburnancarbonsäurederivate nach Anspruch 1 bis 7, dadurch gekennzeichnet, daß der Heteroarylrest, für welchen $R^1$ stehen kann, ein Pyrrolyl-, Furyl-, Thienyl-, Pyridyl-, Pyranyl-, Pyrazolyl-, Imidazolyl-, Pyrimidinyl-, Indolyl- oder Chinolylrest ist.

9. Eburnancarbonsäurederivate nach Anspruch 1 bis 8, dadurch gekennzeichnet, daß der beziehungsweise die Substituent(en), durch welche[n] der beziehungsweise die aliphatische(n) und/oder aromatische(n) Kohlenwasserstoffrest(e), für den beziehungsweise die $R^1$ oder $R^2$ und/oder $R^3$ stehen kann beziehungsweise können, beziehungsweise der beziehungsweise die cycloaliphatische(n) Rest(e), für den beziehungsweise die $R^1$ und/oder $R^3$ stehen kann beziehungsweise können, beziehungsweise der Heteroarylrest, für den $R^1$ stehen kann, substituiert sein kann beziehungsweise können, soweit er beziehungsweise sie [ein] Halogenatom(e) ist beziehungsweise sind, [ein] Fluor-, Chlor- und/oder Bromatom(e), soweit er beziehungsweise sie [eine] primäre, sekundäre und/oder tertiäre Aminogruppe(n) ist beziehungsweise sind, [eine] Dialkylaminogruppe(n) mit 1 bis 10 Kohlenstoffatom(en) in jedem Alkylteil, soweit er beziehungsweise sie [ein] Arylrest(e) mit 6 bis 14 Kohlenstoffatomen ist beziehungsweise sind, [ein] Phenyl-, Diphenyl- oder Naphthylrest(e), soweit er beziehungsweise sie [ein] Alkyl-, Alkoxy- und/oder Alkylthiorest(e) mit [je] 1 bis 8 Kohlenstoffatom(en), ist beziehungsweise sind [ein] solche[r] mit 1 bis 4 Kohlenstoffatom(en), soweit er beziehungsweise sie [ein] Alkoxycarbonylrest(e) mit 1 bis 8, im Alkylteil ist beziehungsweise sind, [ein] solche[r] 1 bis 4 Kohlenstoffatom(en) im Alkylteil, und/oder, soweit er beziehungsweise sie [ein] 1 oder mehr gleiche oder verschiedene Heteroatom(e), bestehend aus Stickstoff, Sauerstoff und/oder Schwefel, aufweisende[r] Heteroarylrest(e) ist beziehungsweise sind, der beziehungsweise die gegebenenfalls durch 1 oder mehr der aufgeführten Substituenten substituiert sein kann beziehungsweise können.

10. Eburnancarbonsäurederivate nach Anspruch 1 bis 9, dadurch gekennzeichnet, daß der beziehungsweise die Heteroarylrest(e), durch welche[n] der beziehungsweise die aliphatische(n) und/oder aromatische(n) Kohlenwasserstoffrest(e), für den beziehungsweise die $R^1$ oder $R^2$ und/oder $R^3$ stehen kann beziehungsweise können, beziehungsweise der beziehungsweise die cycloaliphatische(n) Rest(e), für den beziehungsweise die $R^1$ und/oder $R^3$ stehen kann beziehungsweise können, beziehungsweise der Heteroarylrest, für den $R^1$ stehen kann, substituiert sein kann beziehungsweise können, [ein] Pyrrolyl-, Furyl-, Thienyl-, Pyridyl-, Pyranyl-, Pyrazolyl-, Imidazolyl-, Pyrimidinyl-, Indolyl- und/oder Chinolylrest(e) ist beziehungsweise sind.

11. Die Eburnancarbonsäurederivate gemäß Anspruch 1:

11-(Benzoylamino)-apovincaminsäure,
9-(Benzoylamino)-apovincaminsäure,
11-(Thien-2'-ylcarbonylamino)-apovincaminsäure,
11-(Amino)-apovincaminsäureäthylester,
9-(Amino)-apovincaminsäureäthylester und sein Hydrochlorid,
11-(Methylsulfonylamino)-apovincaminsäureäthyl-ester und sein Hydrochlorid,
9-(Methylsulfonylamino)-apovincaminsäureäthylester und sein Hydrochlorid,
11-(Amino)-vincaminsäureäthylester,
9-(Amino)-vincaminsäureäthylester,
11-[4'-(Nitro)-benzoylamino]-apovincaminsäuremethylester,
9-[4'-(Nitro)-benzoylamino]-apovincaminsäuremethylester,
11-[2'-(Brom)-benzoylamino]-apovincaminsäuremethylester und sein Hydrochlorid,
9-[2'-(Brom)-benzoylamino]-apovincaminsäuremethylester und sein Hydrochlorid,
11-[2'-(Fluor)-benzoylamino]-vincamin,
9-[2'-(Fluor)-Benzoylamino]-vincamin,
11-[3',4',5'-Tri-(methoxy)-benzoylamino]-vincamin,
9-[3',4',5'-Tri-(methoxy)-benzoylamino]-vincamin,
11-(Pivaloylamino)-vincamin,
9-(Pivaloylamino)-vincamin,
9-(Benzoylamino)-vincamin,
11-(Benzoylamino)-vincamin,
11-(Thien-2'-ylcarbonylamino)-vincamin,
9-(Acetamido)-apovincamin,
11-(Acetamido)-apovincamin,
9-(Acetamido)-apovincaminsäureäthylester und sein Hydrochlorid,
11-(Acetamido)-apovincaminsäureäthylester,
9-(Acetamido)-apovincaminsäureisobutylester,
11-(Acetamido)-apovincaminsäureisobutylester und sein Hydrochlorid,
11-[2',3',4'-Tri-(methoxy)-benzoylamino]-apovincamin,

11-[3',4',5'-Tri-(methoxy)-benzoylamino]-apovincamin,
9-[3',4',5'-(Tri-(methoxy)-benzoylamino]-apovincamin,
11-(Pivaloylamino)-apovincamin,
9-(Benzoylamino)-vincaminsäureäthylester,
11-(Benzoylamino)-vincaminsäureäthylester,
9-(Benzoylamino)-apovincaminsäureäthylester,
11-(Benzoylamino)-apovincaminsäureäthylester und sein Hydrochlorid,
11-[Acetamido]-vincaminsäure-[2'-(hydroxy)-äthyl]-ester,
9-[Acetamido]-vincaminsäure-[2'-(hydroxy)-äthyl]-ester,
11-(Nicotinoylamino)-apovincamin,
9-(Nicotinoylamino)-apovincamin,
11-[2'-(Fluor)-benzoylamino]-apovincamin,
9-[2'-(Fluor)-benzoylamino]-apovincamin,
11-(Thien-2'-ylcarbonylamino)-apovincamin,
9-(Thien-2'-ylcarbonylamino)-apovincamin,
9-(Thien-2'-ylcarbonylamino)-apovincaminsäureäthylester
11-(Thien-2'-ylcarbonylamino)-apovincaminsäureäthylester und sein Hydrochlorid,
11-(Benzoylamino)-apovincamin,
9-(Benzoylamino)-apovincamin,
11-[Benzoylamino]-apovincaminsäure-[2'-(hydroxy)-äthyl]-ester,
9-[Benzoylamino]-apovincaminsäure-[2'-(hydroxy)-äthyl]-ester,
11-[Benzoylamino]-apovincaminsäure-[2'-(methyl)-propyl]-ester und sein Hydrochlorid,
9-[Benzoylamino]-apovincaminsäure-[2'-(methyl)-propyl]-ester und sein Hydrochlorid,
11-(Amino)-vincaminsäure,
9-(Amino)-vincaminsäure,
11-[2'-(Fluor)-benzoylamino]-vincaminsäure,
9-[2'-(Fluor)-benzoylamino]-vincaminsäure,
11-(Cyclohexylcarbonylamino)-vincamin,
11-[3'-(Brom)-propionylamino]-apovincamin,
11-(Cyclohexylcarbonylamino)-apovincamin,
9-(Pivaloylamino)-apovincamin,
9-(Cyclohexylcarbonylamino)-vincamin,
9-[3'-(Brom)-propionylamino]-apovincamin und
9-(Cyclohexylcarbonylamino)-apovincamin
sowie ihre optisch aktiven Isomere.

12. Verfahren zur Herstellung der Eburnancarbonsäurederivate nach Anspruch 1 bis 11 beziehungsweise von 11-(Acetamido)-vincamin beziehungsweise 9- beziehungsweise 11-(Amino)-apovincamin, dadurch gekennzeichnet, daß man

a) racemische oder optisch aktive Eburnancarbonsäurederivate der allgemeinen Formel

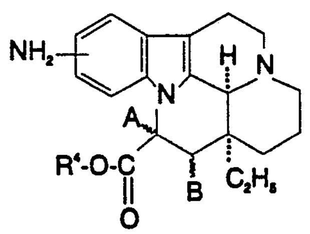

IV,

worin

$R^4$ für einen Methylrest steht oder, falls A und B zusammen eine chemische Bindung darstellen, auch Wasserstoff bedeuten kann,

und die Aminogruppe sich in der 9- oder 11-Stellung befindet, oder deren Salze, gegebenenfalls nach einer Hydrolyse, acyliert ausgenommen das Acetylieren, im Falle daß $R^4$ für einen Methylrest steht, A eine Hydroxygruppe bedeutet und B Wasserstoff darstellt, und

α) gegebenenfalls die erhaltenen Eburnancarbonsäurederivate der allgemeinen Formel I, bei welchen R für einen Acylamidorest der allgemeinen Formel II oder einen Sulfonylamidorest der allgemeinen Formel III, wobei $R^1$ und $R^2$ die in den Ansprüchen 1 bis 10 angegebenen Bedeutungen haben, steht, A und B die im Anspruch 1 angegebenen Bedeutungen haben und $R^3$ die Bedeutung von $R^4$ hat, beziehungsweise ihre Salze verestert beziehungsweise umestert und/oder

β) gegebenenfalls die erhaltenen Eburnancarbonsäurederivate der allgemeinen Formel I, bei welchen R für einen Acylamidorest der allgemeinen Formel II oder einen Sulfonylamidorest der allgemeinen Formel III, wobei $R^1$ und $R^2$ die in den Ansprüchen 1 bis 10 angegebenen Bedeutungen haben, steht, A für eine Hydroxygruppe steht, B Wasserstoff bedeutet und $R^3$ von Wasserstoff verschieden ist, beziehungsweise ihre Salze hydrolysiert und/oder

γ) gegebenenfalls die erhaltenen Eburnancarbonsäurederivate der allgemeinen Formel I, bei welchen R und $R^3$ die in den Ansprüchen 1 bis 10 angegebenen Bedeutungen haben, A für eine Hydroxygruppe steht und B Wasserstoff bedeutet, beziehungsweise ihre Salze dehydratisiert oder

b) racemische oder optisch aktive Eburnancarbonsäurederivate der allgemeinen Formel

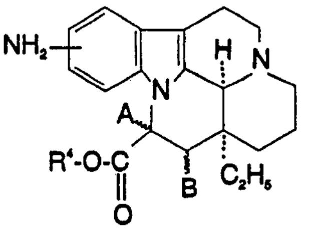

IV,

worin
$R^4$ für einen Methylrest steht oder, falls A und B zusammen eine chemische Bindung darstellen, auch Wasserstoff bedeuten kann, und die Aminogruppe sich in der 9- oder 11-Stellung befindet, oder deren Salze verestert beziehungsweise umestert und

α) gegebenenfalls die erhaltenen Eburnancarbonsäurederivate der allgemeinen Formel I, bei welchen R für eine Aminogruppe steht, A eine Hydroxygruppe bedeutet, B ein Wasserstoffatom darstellt und $R^3$ von Wasserstoff verschieden ist, beziehungsweise ihre Salze hydrolysiert und/oder

β) gegebenenfalls die erhaltenen Eburnancarbonsäurederivate der allgemeinen Formel I, bei welchen R und $R^3$ die in den Ansprüchen 1 bis 10 angegebenen Bedeutungen haben, A für eine Hydroxygruppe steht und B Wasserstoff bedeutet, beziehungsweise ihre Salze dehydratisiert und/oder

γ) gegebenenfalls die erhaltenen Eburnancarbonsäurederivate der allgemeinen Formel I, bei welchen R für eine Aminogruppe steht und $R^3$, A und B die in den Ansprüchen 1 bis 7, 9 oder 10 angegebenen Bedeutungen haben, beziehungsweise ihre Salze acyliert oder

c) racemische oder optisch aktive 9- oder 11-(Nitro)-eburnancarbonsäurederivate der allgemeinen Formel

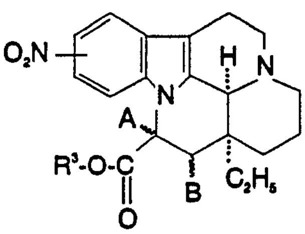

V,

worin $R^3$, A und B die in den Ansprüchen 1 bis 7, 9 oder 10 angegebenen Bedeutungen haben und die Nitrogruppe in der 9- oder 11-Stellung ist, reduziert und gegebenenfalls die Umsetzungen nach a) α) und/oder α) β) beziehungsweise b) α) und/oder a) γ) beziehungsweise b) β) und/oder b) γ) durchführt,

sowie gegebenenfalls die erhaltenen Eburnancarbonsäurederivate der allgemeinen Formel I, in Säureadditionssalze oder quaternäre Salze überführt und/oder gegebenenfalls die erhaltenen Salze der Eburnancarbonsäurederivate der allgemeinen Formel I in die freien Eburnancarbonsäurederivate der allgemeinen Formel I oder in andere Säureadditionssalze beziehungsweise quaternäre Salze überführt und/oder gegebenenfalls eine Spaltung der erhaltenen racemischen Eburnancarbonsäurederivate der allgemeinen Formel I beziehungsweise von deren Säureadditionssalzen beziehungsweise quaternären Salzen beziehungsweise eine Racemisierung der entsprechenden optisch aktiven Verbindungen in Stereoisomere vornimmt.

13. Arzneimittel, gekennzeichnet durch einen Gehalt an 1 oder mehr Verbindung(en) nach Anspruch 1 bis 11 und/oder 11-(Acetamido)-vincamin als Wirkstoff(en) zweckmäßigerweise zusammen mit 1 oder mehr üblichen Träger- und/oder Hilfsstoff(en).

**Patentansprüche** für den Vertragsstaat: AT

1. Verfahren zur Herstellung von Eburnancarbonsäurederivaten der folgenden allgemeinen Formel I bzw. von 11-(Acetamido)-vincamin bzw. 9- bzw. 11-(Amino)-apovincamin

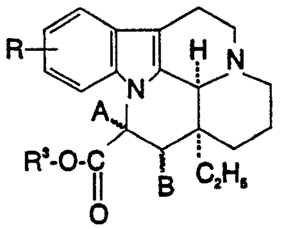

I,

worin

R für eine Aminogruppe oder einen Acylamidorest der allgemeinen Formel

H O<br>
| ||<br>
$-N-C-R^1$

II,

in welchletzterer

$R^1$ Wasserstoff oder einen aliphatischen Kohlenwasserstoffrest mit 1 bis 10 Kohlenstoffatom(en), cycloaliphatischen Kohlenwasserstoffrest mit 3 bis 8 Kohlenstoffatomen, aromatischen Kohlenwasserstoffrest mit 6 bis 14 Kohlenstoffatomen oder einen 1 oder mehr gleiche oder verschiedene aus Stickstoff, Sauerstoff und/oder Schwefel bestehende Heteroatome aufweisenden 5- bis 7-gliedrigen Heteroarylrest, gegebenenfalls zusätzlich zu diesen Gliedern mit 4 zur Bildung eines Benzolrings ankondensierten Gliedern bedeutet, wobei die vorstehend genannten Reste mit einem oder mehreren gleichen oder verschiedenen Substituenten substituiert sein können, bestehend aus Halogenatomen, primären, sekundären und tertiären Aminogruppen, Hydroxygruppen, Arylresten mit 6 bis 14 Kohlenstoffatomen, Alkyl-, Alkoxy- und Alkylthioresten mit 1 bis 8 Kohlenstoffatomen, Trifluormethylresten, Carboxylgruppen, Alkoxycarbonylresten mit 1 bis 8 Kohlenstoffatomen im Alkylteil, Thio-, Sulfinyl-, Sulfonyl-, Nitro-Keto-, Aldehyd- und Cyangruppen und 1 oder mehr aus Stickstoff, Sauerstoff und/oder Schwefel als Heteroatome aufweisende 5- bis 7-gliedrige Heteroarylresten, gegebenenfalls zusätzlich zu diesen Gliedern mit 4 zur Bildung eines Benzolrings ankondensierten Gliedern, vor allem Pyrrolyl-, Furyl-, Thienyl-, Pyridyl-, Pyranyl-, Pyrazolyl-, Imidazolyl-, Pyrimidinyl-, Indolyl- und Chinolylresten, die gegebenenfalls durch 1 oder mehr der aufgeführten Substituenten substituiert sein können, oder einen Sulfonamidorest der allgemeinen Formel

H<br>
|<br>
$-N-S-R^2$<br>
$O_2$

III,

in welchletzterer

$R^2$ einen aliphatischen Kohlenwasserstoffrest mit 1 bis 10 Kohlenstoffatom(en) oder einen aromatischen Kohlenwasserstoffrest mit 6 bis 14 Kohlenstoffatomen bedeutet, wobei diese Reste durch einen oder mehrere gleiche oder verschiedene Substituenten, wie sie im Zusammenhang mit $R^1$ aufgeführt sind, substituiert sein können, steht,

$R^3$ Wasserstoff oder einen aliphatischen Kohlenwasserstoffrest mit 1 bis 10 Kohlenstoffatom(en), cycloaliphatischen Kohlenwasserstoffrest mit 3 bis 8 Kohlenstoffatomen oder aromatischen Kohlenwasserstoffrest mit 6 bis 14 Kohlenstoffatomen bedeutet, wobei diese Reste mit 1 oder mehr gleichen oder verschiedenen Substituenten, wie sie im Zusammenhang mit $R^1$ definiert worden sind, substituiert sein können, und

A für eine Hydroxygruppe steht und

B Wasserstoff bedeutet oder

A und B zusammen eine chemische Bindung darstellen und

R in der 9- oder 11-Stellung ist, mit den weiteren Maßgaben, daß

a) im Falle daß

R für eine Aminogruppe steht,

$R^3$ von einem nicht substituierten Methylrest verschieden ist, und

b) im Falle daß

R für einen in der 9- oder 11-Stellung befindlichen Acylamidorest der allgemeinen Formel II,

bei welchem

$R^1$ einen Methylrest bedeutet,

steht,

A eine Hydroxygruppe bedeutet und

B Wasserstoff darstellt,

$R^3$ von einem nicht substituierten Methylrest verschieden ist,

sowie ihre optisch aktiven Isomere und Salze, dadurch gekennzeichnet, daß man

a) racemische oder optisch aktive Eburnancarbonsäurederivate der allgemeinen Formel

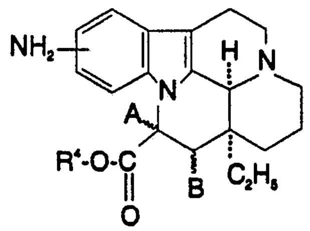

IV,

worin

$R^4$ für einen Methylrest steht oder, falls A und B zusammen eine chemische Bindung darstellen, auch Wasserstoff bedeuten kann, und die Aminogruppe sich in der 9- oder 11-Stellung befindet,

oder deren Salze, gegebenenfalls nach einer Hydrolyse, acyliert ausgenommen das Acetylieren, im Falle daß $R^4$ für einen Methylrest steht, A eine Hydroxygruppe bedeutet und B Wasserstoff darstellt, und

α) gegebenenfalls die erhaltenen Eburnancarbonsäurederivate der allgemeinen Formel I, bei welchen R für einen Acylamidorest der allgemeinen Formel II oder einen Sulfonylamidorest der allgemeinen Formel III, wobei $R^1$ und $R^2$ die vorstehend angegebenen Bedeutungen haben, steht, A und B die vorstehend angegebenen Bedeutungen haben und $R^3$ die Bedeutung von $R^4$ hat, beziehungsweise ihre Salze verestert beziehungsweise umestert und/oder

β) gegebenenfalls die erhaltenen Eburnancarbonsäurederivate der allgemeinen Formel I, bei welchen R für einen Acylamidorest der allgemeinen Formel II oder einen Sulfonylamidorest der allgemeinen Formel III, wobei $R^1$ und $R^2$ die vorstehend angegebenen Bedeutungen haben, steht, A für eine Hydroxygruppe steht, B Wasserstoff bedeutet und $R^3$ von Wasserstoff verschieden ist, beziehungsweise ihre Salze hydrolysiert und/oder

γ) gegebenenfalls die erhaltenen Eburnancarbonsäurederivate der allgemeinen Formel I, bei welchen R und $R^3$ die vorstehend angegebenen Bedeutungen haben, A für eine Hydroxygruppe steht und B Wasserstoff bedeutet, beziehungsweise ihre Salze dehydratisiert oder

b) racemische oder optisch aktive Eburnancarbonsäurederivate der allgemeinen Formel

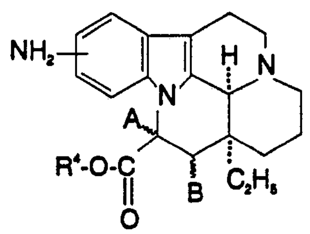

IV,

worin

$R^4$ für einen Methylrest steht oder, falls A und B zusammen eine chemische Bindung darstellen, auch Wasserstoff bedeuten kann, und die Aminogruppe sich in der 9- oder 11-Stellung befindet,

oder deren Salze verestert beziehungsweise umestert und

α) gegebenenfalls die erhaltenen Eburnancarbonsäurederivate der allgemeinen Formel I, bei welchen R für eine Aminogruppe steht, A eine Hydroxygruppe bedeutet, B ein Wasserstoffatom darstellt und $R^3$ von Wasserstoff verschieden ist, beziehungsweise ihre Salze hydrolysiert und/oder

β) gegebenenfalls die erhaltenen Eburnancarbonsäurederivate der allgemeinen Formel I, bei welchen R und $R^3$ die vorstehend angegebenen Bedeutungen beben, A für eine Hydroxygruppe steht und B Wasserstoff bedeutet, beziehungsweise ihre Salze dehydratisiert und/oder

γ) gegebenenfalls die erhaltenen Eburnancarbonsäurederivate der allgemeinen Formel I, bei welchen R für eine Aminogruppe steht und $R^3$, A und B die vorstehend angegebenen Bedeutungen haben, beziehungsweise ihre Salze acyliert oder

c) racemische oder optisch aktive 9- oder 11-(Nitro)-eburnancarbonsäurederivate der allgemeinen Formel

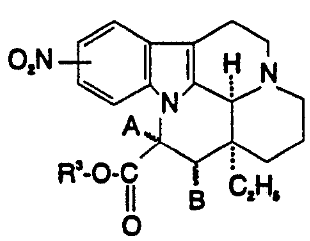

V,

worin $R^3$, A und B die vorstehend angegebenen Bedeutungen haben und die Nitrogruppe in der 9- oder 11-Stellung ist, reduziert und gegebenenfalls die Umsetzungen nach a) α) und/oder α) β) beziehungsweise b) α) und/oder a) γ) beziehungsweise b) β) und/oder b) γ) durchführt,

sowie gegebenenfalls die

erhaltenen Eburnancarbonsäurederivate der allgemeinen Formel I, in Säureadditionssalze oder quaternäre Salze überführt und/oder
gegebenenfalls die erhaltenen Salze der Eburnancarbonsäurederivate der allgemeinen Formel I in die freien Eburnancarbonsäurederivate der allgemeinen Formel I oder in andere Säureadditionssalze beziehungsweise quaternäre Salze überführt und/oder gegebenenfalls eine Spaltung der erhaltenen racemischen Eburnancarbonsäurederivate der allgemeinen Formel I beziehungsweise von deren Säureadditionssalzen beziehungsweise quaternären Salzen beziehungsweise eine Racemisierung der entsprechenden optisch aktiven Verbindungen in Stereoisomere vornimmt.

2. Verfahren nach Anspruch 1, dadurch gekennzeichnet, daß solche Eburnancarbonsäurederivate der allgemeinen Formel I hergestellt werden, bei denen der bzw. die aliphatische(n) Kohlenwasserstoffrest(e), für welche[n] $R^1$ oder $R^2$ und/oder $R^3$ stehen kann beziehungsweise können, [ein] Alkylrest(e), und/oder [ein] Alkenyl- und/oder Alkinylrest(e) ist beziehungsweise sind.

3. Verfahren nach Anspruch 1 oder 2, dadurch gekennzeichnet, daß solche Eburnancarbonsäurederivate der Formel I hergestellt werden, bei denen der bzw. die aliphatische(n) Kohlenwasserstoffrest(e), für welche[n] $R^1$ oder $R^2$ und/oder $R^3$ stehen kann beziehungsweise können, [ein] solche[r] mit 1 bis 8 Kohlenstoffatomen und der beziehungsweise die Alkenyl- und/oder Alkinylrest(e) [ein] solche[r] mit 2 bis 4 Kohlenstoffatomen ist beziehungsweise sind.

4. Verfahren nach Anspruch 1 bis 3, dadurch gekennzeichnet, daß solche Eburnancarbonsäurederivate der Formel I hergestellt werden, bei denen der bzw. die cycloaliphatische(n) Kohlenwasserstoffrest(e), für welche[n] $R^1$ und/oder $R^3$ stehen kann beziehungsweise können, [ein] Cycloalkylrest(e) ist beziehungsweise sind.

5. Verfahren nach Anspruch 1 bis 3, dadurch gekennzeichnet, daß solche Eburnancarbonsäurederivate der Formel I hergestellt werden, bei denen der bzw. die Cycloalkylrest(e) für welche[n] $R^1$ und/oder $R^3$ stehen kann beziehungsweise können, [ein] solche[r] mit 5 bis 7 Kohlenstoffatomen ist beziehungsweise sind.

6. Verfahren nach Anspruch 1 bis 5, dadurch gekennzeichnet, daß solche Eburnancarbonsäurederivate der Formel I hergestellt werden, bei denen der bzw. die aromatische(n) Kohlenwasserstoffrest(e), für welche[n] $R^1$ oder $R^2$ und/oder $R^3$ stehen kann beziehungsweise können, monocyclisch, nämlich [ein] Phenylrest(e), und/oder polycyclisch nicht kondensiert und/oder polycyclisch kondensiert ist beziehungsweise sind.

7. Verfahren nach Anspruch 1 bis 6, dadurch gekennzeichnet, daß solche Eburnancarbonsäurederivate der Formel I hergestellt werden, bei denen der bzw. die aromatischen Kohlenwasserstoffrest(e), für welche[n] $R^1$ oder $R^2$ und/oder $R^3$ stehen kann beziehungsweise können, soweit er beziehungsweise sie polycyclisch ist beziehungsweise sind, [ein] Diphenylrest(e) und/oder Naphthylrest(e) ist beziehungsweise sind.

8. Verfahren nach Anspruch 1 bis 7, dadurch gekennzeichnet, daß solche Eburnancarbonsäurederivate der Formel I hergestellt werden, bei denen der Heteroarylrest, für welchen $R^1$ stehen kann, ein Pyrrolyl-, Furyl-, Thienyl-, Pyridyl-, Pyranyl-, Pyrazolyl-, Imidazolyl-, Pyrimidinyl-, Indolyl- oder Chinolylrest ist.

9. Verfahren nach Anspruch 1 bis 8, dadurch gekennzeichnet, daß solche Eburnancarbonsäurederivate der Formel I hergestellt werden, bei denen der bzw. die Substituent(en), durch welche[n] der beziehungsweise die aliphatische(n) und/oder aromatische(n) Kohlenwasserstoffrest(e), für den beziehungsweise die $R^1$ oder $R^2$ und/oder $R^3$ stehen kann beziehungsweise können, beziehungsweise der beziehungsweise die cycloaliphatische(n) Rest(e), für den beziehungsweise die $R^1$ und/oder $R^3$ stehen kann beziehungsweise können, beziehungsweise der Heteroarylrest, für den $R^1$ stehen kann, substituiert sein kann beziehungsweise können, soweit er beziehungsweise sie [ein] Halogenatom(e) ist beziehungsweise sind, [ein] Fluor-, Chlor- und/oder Bromatom(e), soweit er beziehungsweise sie [eine] primäre, sekundäre und/oder tertiäre Aminogruppe(n) ist beziehungsweise sind, [eine] Dialkylaminogruppe(n) mit 1 bis 10 Kohlenstoffatom(en) in jedem Alkylteil, soweit er beziehungsweise sie [ein] Arylrest(e) mit 6 bis 14 Kohlenstoffatomen ist beziehungsweise sind, [ein] Phenyl-, Diphenyl- oder Naphthylrest(e), soweit er beziehungsweise sie [ein] Alkyl-, Alkoxy- und/oder Alkylthiorest(e) mit [je] 1 bis 8 Kohlenstoffatom(en), ist beziehungsweise sind [ein] solche[r] mit 1 bis 4 Kohlenstoffatom(en), soweit er beziehungsweise sie [ein] Alkoxycarbonylrest(e) mit 1 bis 8, im Alkylteil ist beziehungsweise sind, [ein] solche[r] 1 bis 4 Kohlenstoffatom(en) im Alkylteil, und/oder, soweit er beziehungsweise sie [ein] 1 oder mehr gleiche oder verschiedene Heteroatom(e), bestehend aus Stickstoff, Sauerstoff und/oder Schwefel, aufweisende[r] Heteroarylrest(e) ist beziehungsweise sind, der beziehungsweise die gegebenenfalls durch 1 oder mehr der aufgeführten Substituenten substituiert sein kann beziehungsweise können.

10. Verfahren nach Anspruch 1 bis 9, dadurch gekennzeichnet, daß solche Eburnancarbonsäurederivate der Formel I hergestellt werden, bei denen der bzw. die Heteroarylrest(e), durch welche[n] der beziehungsweise die aliphatische(n) und/oder aromatische(n) Kohlenwasserstoffrest(e), für den beziehungsweise die $R^1$ oder $R^2$ und/oder $R^3$ stellen kann beziehungsweise können, beziehungsweise der beziehungsweise die cycloaliphatische(n) Rest(e), für den beziehungsweise die $R^1$ und/oder $R^3$ stehen kann beziehungsweise können, beziehungsweise der Heteroarylrest, für den $R^1$ stehen kann, substituiert sein kann beziehungsweise können, [ein] Pyrrolyl-, Furyl-, Thienyl-, Pyridyl-, Pyranyl-, Pyrazolyl-, Imidazolyl-, Pyrimidinyl-, Indolyl- und/oder Chinolylrest(e) ist beziehungsweise sind.

11. Verfahren nach Anspruch 1, dadurch gekennzeichnet, daß
11-(Benzoylamino)-apovincaminsäure,
9-(Benzoylamino)-apovincaminsäure, 11-(Thien-2'-ylcarbonylamino)-apovincaminsäure,
11-(Amino)-apovincaminsäureäthylester,
9-(Amino)-apovincaminsäureäthylester und sein Hydrochlorid,
11-(Methylsulfonylamino)-apovincaminsäureäthylester und sein Hydrochlorid,
9-(Methylsulfonylamino)-apovincaminsäureäthylester und sein Hydrochlorid,
11-(Amino)-vincaminsäureäthylester,
9-(Amino)-vincaminsäureäthylester,
11-[4'-(Nitro)-benzoylamino]-apovincaminsäuremethylester,
9-[4'-(Nitro)-benzoylamino]-apovincaminsäuremethylester,
11-[2'-(Brom)-benzoylamino]-apovincaminsäuremethylester und sein Hydrochlorid,
9-[2'-(Brom)-benzoylamino]-apovincaminsäuremethylester und sein Hydrochlorid,
11-[2'-(Fluor)-benzoylamino]-vincamin,
9-[2'-(Fluor)-benzoylamino]-vincamin,
11-[3',4',5'-Tri-(methoxy)-benzoylamino]-vincamin,
9-[3',4',5'-Tri-(methoxy)-benzoylamino]-vincamin,
11-(Pivaloylamino)-vincamin,
9-(Pivaloylamino)-vincamin,
9-(Benzoylamino)-vincamin,
11-(Benzoylamino)-vincamin,
11-(Thien-2'-ylcarbonylamino)-vincamin,
9-(Acetamido)-apovincamin,
11-(Acetamido)-apovincamin,
9-(Acetamido)-apovincaminsäureäthylester und sein Hydrochlorid,
11-(Acetamido)-apovincaminsäureäthylester,
9-(Acetamido)-apovincaminsäureisobutylester,
11-(Acetamido)-apovincaminsäureisobutylester und sein Hydrochlorid,
11-[2',3',4'-Tri-(methoxy)-benzoylamino]-apovincamin,
11-[3',4',5'-Tri-(methoxy)-benzoylamino]-apovincamin,
9-[3',4',5'-(Tri-(methoxy)-benzoylamino]-apovincamin,
11-(Pivaloylamino)-apovincamin,
9-(Benzoylamino)-vincaminsäureäthylester,
11-(Benzoylamino)-vincaminsäureäthylester,
9-(Benzoylamino)-apovincaminsäureäthylester,
11-(Benzoylamino)-apovincaminsäureäthylester und sein Hydrochlorid,
11-[Acetamido]-vincaminsäure-[2'-(hydroxy)-äthyl]-ester,
9-[Acetamido]-vincaminsäure-[2'-(hydroxy)-äthyl]-ester,
11-(Nicotinoylamino)-apovincamin,
9-(Nicotinoylamino)-apovincamin,
11-[2'-(Fluor)-benzoylamino]-apovincamin,
9-[2'-(Fluor)-benzoylamino]-apovincamin,
11-(Thien-2'-ylcarbonylamino )-apovincamin,
9-(Thien-2'-ylcarbonylamino)-apovincamin,
9-(Thien-2'-ylcarbonylamino)-apovincaminsäureäthylester
11-(Thien-2'-ylcarbonylamino )-apovincaminsäureäthylester und sein Hydrochlorid,
11-(Benzoylamino)-apovincamin,
9-(Benzoylamino)-apovincamin,
11-[Benzoylamino]-apovincaminsäure-[2'-(hydroxy)-äthyl]-ester,
9-[Benzoylamino]-apovincaminsäure-[2'-(hydroxy)-äthyl]-ester,
11-[Benzoylamino]-apovincaminsäure-[2'-(methyl)-propyl]-ester und sein Hydrochlorid,
9-[Benzoylamino]-apovincaminsäure-[2'-(methyl)-propyl]-ester und sein Hydrochlorid,
11-(Amino)-vincaminsäure,
9-(Amino)-vincaminsäure,
11-[2'-(Fluor)-benzoylamino]-vincaminsäure,
9-[2'-(Fluor)-benzoylamino]-vincaminsäure,
11-(Cyclohexylcarbonylamino)-vincamin,
11-[3'-(Brom)-propionylamino]-apovincamin,
11-(Cyclohexylcarbonylamino)-apovincamin,
9-(Pivaloylamino)-apovincamin,
9-(Cyclohexylcarbonylamino)-vincamin,
9-[3'-(Brom)-propionylamino]-apovincamin und
9-(Cyclohexylcarbonylamino)-apovincamin
sowie ihre optisch aktiven Isomere hergestellt werden.

**Claims** for the Contracting States: BE, CH, DE, FR, GB, IT, LI, NL, SE

1. Eburnanecarboxylic acid derivatives which have at position 9 or 11 an optionally substituted amino group and are of the general formula

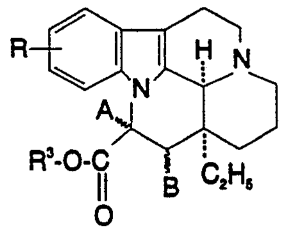

I

wherein

R stands for an amino group or an acylamino residue of the general formula

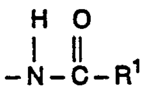

II

in which $R^1$ is hydrogen or an aliphatic hydrocarbon residue having 1 to 10 carbon atom(s), a cycloaliphatic hydrocarbon residue having 3 to 8 carbon atoms, an aromatic hydrocarbon residue having 6 to 14 carbon atoms or a 5- to 7-membered heteroaryl residue having one or more identical or different heteroatoms consisting of nitrogen, oxygen and/or sulphur, and optionally having in addition to said members 4 members which have been fused on to form a benzene ring, wherein the above-mentioned residues are optionally substituted with one or more identical or different substituents consisting of halogen atoms, primary, secondary and tertiary amino groups, hydroxy groups, aryl residues having 6 to 14 carbon atoms, trifluoromethyl residues, carboxyl groups, alkoxy-carbonyl residues having 1 to 8 carbon atoms in the alkyl moiety, thio, sulphinyl, sulphonyl, nitro, keto, aldehyde and cyano groups and 1 or more 5- to 7-membered heteroaryl residues containing nitrogen, oxygen and/or sulphur as heteroatoms and having optionally in addition to said members 4 members which have been fused on to form a benzene ring, particularly pyrrolyl, furyl, thienyl, pyridyl, pyranyl, pyrazolyl, imidazolyl, pyrimidinyl, indolyl and quinolyl residues, which may optionally be substituted with 1 or more of the listed substituents, or a sulphonamido residue of the general formula

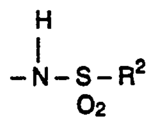

III

in which $R^2$ is an aliphatic hydrocarbon residue having 1 to 10 carbon atom(s) or an aromatic hydrocarbon residue having 6 to 14 carbon atoms, wherein said residues may be substituted with one or more of the substituents listed in connection with $R^1$,

$R^3$ is hydrogen or an aliphatic hydrocarbon residue having 1 to 10 carbon atom(s), a cycloaliphatic hydrocarbon residue having 3 to 8 carbon atoms or an aromatic hydrocarbon residue having 6 to 14 carbon atoms, which residues may be substituted with 1 or more identical or different ones of the substituents listed in connection with $R^1$,

A stands for a hydroxy group,

B means hydrogen or

A and B together represent a chemical bond and

R is in position 9 or 11,

provided that

a) if R stands for an amino group, then $R^3$ is different from an unsubstituted methyl residue and

b) if R stands for an acylamido residue which is in position 9 or 11 and has the general formula II, in which $R^1$ is a methyl residue,

A represents a hydroxy group and

B represents hydrogen, then

$R^3$ is different from an unsubstituted methyl residue, and their optically active isomers and salts.

2. Eburnanecarboxylic acid derivatives according to claim 1, characterized in that the aliphatic hydrocarbon residue(s) which may be represented by $R^1$ or $R^2$ and/or $R^3$ is or are (an) alkyl residue(s) and/or (an) alkenyl and/or alkinyl residue(s).

3. Eburnanecarboxylic acid derivatives according to claim 1 or 2, characterized in that the aliphatic hydrocarbon residue(s) which may be represented by $R^1$ or $R^2$ and/or $R^3$ is or are such residue(s) having 1 to 8 carbon atoms and the alkenyl and/or alkinyl residue(s) is or are such residue(s) having 2 to 4 carbon atoms.

4. Eburnanecarboxylic acid derivatives according to claims 1 to 3, characterized in that the cycloaliphatic hydrocarbon residue(s) which may be represented by $R^1$ and/or $R^3$ is or are (a) cycloalkyl residue(s).

5. Eburnanecarboxylic acid derivatives according to claims 1 to 3, characterized in that the cycloalkyl which may be represented by $R^1$ and/or $R^3$ is or are such residue(s) having 5 to 7 carbon atoms.

6. Eburnanecarboxylic acid derivatives according to claims 1 to 5, characterized in that the aromatic hydrocarbon residue(s) which may be represented by $R^1$ or $R^2$ and/or $R^3$ is or are monocyclic, namely (a) phenyl residue(s), and/or is or are polycyclic and uncondensed and/or polycyclic and condensed.

7. Eburnanecarboxylic acid derivatives according to claims 1 to 6, characterized in that that one or more aromatic hydrocarbon radical(s) which may be represented by $R^1$ or $R^2$ and/or $R^3$ and which is or are polycyclic is or are (a) diphenyl residue(s) and/or naphthyl residue(s).

8. Eburnanecarboxylic acid derivatives according to claims 1 to 7, characterized in that the heteroaryl residue which may be represented by $R^1$ is a pyrrolyl, furyl, thienyl, pyridyl, pyranyl, pyrazolyl, imidazolyl, pyriminyl, indolyl or quinolyle residue.

9. Eburnanecarboxylic acid derivatives according to claims 1 to 8, characterized in that the substituent(s) with which the aliphatic and/or aromatic hydrocarbon residue(s) which may be represented by $R^1$ or $R^2$ and/or $R^3$ and/or the cycloaliphatic residue(s) which may be represented by $R^1$ and/or $R^3$ and/or the heteroaryl residue which may be represented by $R^1$ is or are (a) fluorine, chlorine and/or bromine atom(s) or is or are (a) dialkylamino group(s) having 1 to 10 carbon atoms in each alkyl moiety if it is or they are (a) primary, secondary and/or tertiary amino group(s) or is or are (a) phenyl, diphenyl or naphthyl residue(s) if it is or they are (an) aryl residue(s) having 6 to 14 carbon atoms or is or (a) residue(s) having 1 to 4 carbon atom(s) if it is or they are (an) alkyl, alkoxy and/or alkylthio residue(s) having 1 to 8 carbon atoms each or a residue having in an alkyl moiety 1 to 4 carbon atom(s) if it is or they are (an) alkoxycarbonyl residue(s) having 1 to 8 carbon atoms in its alkyl moiety, and/or said residue(s) may consist of (a) heteroaryl residue(s) which contain(s) 1 or more identical or different heteroatom(s) consisting of nitrogen, oxygen and/or sulphur and in that case may be substituted with 1 or more of the listed substituents.

10. Eburnanecarboxylic acid derivatives according to claims 1 to 9, characterized in that the heteroaryl residue(s) with which the aliphatic and/or aromatic hydrocarbon residue(s) which may be represented by $R^1$ or $R^2$ and/or $R^3$ and or the cycloaliphatic residue(s) which may be represented by $R^1$ and/or $R^3$ and/or the heteroaryl residue which may be represented by $R^1$ may be substituted is or are (a) pyrrolyl, furyl, thienyl, pyridyl, pyranyl, pyrazolyl, imidazolyl, pyrimidinyl, indolyl and/or quinolyl residue(s).

11. The following eburnanecarboxylic acid derivatives according to claim 1:
11-(benzoylamino)-apovincamic acid,
9-(benzoylamino)-apovincamic acid,
11-(thiene-2'-ylcarbonylamino)-apovincamic acid,
11-(amino)-apovincamic acid ethyl ester,
9-(amino)-apovincamic acid ethyl ester and its hydrochloride,
11-(methylsulphonylamino)-apovincamic acid ethyl ester and its hydrochloride,
9-(methylsulphonylamino)-apovincamic acid ethyl ester and its hydrochloride,
11-(amino)-vincamic acid ethyl ester,
9-(amino)-vincamic acid ethyl ester,
11-[4'-(nitro)-benzoylamino]-apovincamic acid methyl ester,
9-[4'-(nitro)-benzoylamino]-apovincamic acid methyl ester,
11-[2'-(bromo)-benzoylamino-apovincamic acid methyl ester and its hydrochloride,
9-[2'-(bromo)-benzoylamino]-apovincamic acid methyl ester and its hydrochloride,
11-[2'-(fluoro)-benzoylamino]-vincamine,
9-[2'-(fluoro)-benzoylamino]-vincamine,
11-[3',4',5'-tri-(methoxy)-benzoylamino]-vincamine,
9-[3',4',5'-tri-(methoxy)-benzoylamino]-vincamine,
11-(pivaloylamino)-vincamine,
9-(pivaloylamino)-vincamine,
9-(benzoylamino)-vincamine,
11-(benzoylamino)-vincamine,
11-(thiene-2'-ylcarbonylamino)-vincamine,
9-(acetamido)-apovincamine,
11-(acetamido)-apovincamine,
9-(acetamido)-apovincamic acid ethyl ester and its hydrochloride,
11-(acetamido)-apovincamic acid ethyl ester,
9-(acetamido)-apovincamic acid isobutyl ester,
11-(acetamido)-apovincamic acid isobutyl ester and its hydrochloride,

11-[2',3',4'-tri-(methoxy)-benzoylamino]-apovincamine,
11-[3',4',5'-tri-(methoxy)-benzoylamino]-apovincamine,
9-[3',4',5'-tri-(methoxy)-benzoylamino]-apovincamine,
11-(pivaloylamino)-apovincamine,
9-(benzoylamino)-vincamic acid ethyl ester,
11-(benzoylamino)-vincamic acid ethyl ester,
9-(benzoylamino)-apovincamic acid ethyl ester,
9-(benzoylamino)
11-(benzoylamino)-apovincamic acid ethyl ester and its hydrochloride,
11-[acetamido]-vincamic acid-[2'-(hydroxy)-ethyl] ester,
9-[acetamido-vincamic acid-[2'-(hydroxy)-ethyl] ester,
11-(nicotinoylamino)-apovincamine,
9-(nicotinoylamino)-apovincamine,
11-[2'-(fluoro)-benzoylamino] -apovincamine,
9-[2'-(fluoro)-benzoylamino] -apovincamine,
11-(thiene-2'-ylcarbonylamino)-apovincamine,
9-(thiene-2'-ylcarbonylamino)-apovincamine,
9-(thiene-2'-ylcarbonylamino)-apovincamic acid ethyl ester,
11-(thiene-2'-ylcarbonylamino)-apovincamic acid and its hydrochloride,
11-(benzoylamino)-apovincamine,
9-(benzoylamino)-apovincamine,
11-[benzoylamino]-apovincamic acid-[2'-(hydroxy)-ethyl] ester,
9-[benzoylamino]-apovincamic acid-[2'-(hydroxy)-ethyl] ester,
11-[benzoylamino]-apovincamic acid-[2'-(methyl)-propyl] ester and its hydrochloride,
9-[benzoylamino]-apovincamic acid-[2'-(methyl)-propyl] ester and its hydrochloride,
11-(amino)-vincamic acid,
9-(amino)-vincamic acid,
11-[2'-(fluoro)-benzoylamino]-vincamic acid,
9-[2'-(fluoro)-benzoylamino]-vincamic acid,
11-(cyclohexylcarbonylamino)-vincamine,
11-[3'-(bromo)-propionylamino]-apovincamine,
11-(cyclohexylcarbonylamino)-apovincamine,
9-(pivaloylamino)-apovincamine,
9-(cyclohexylcarbonylamino)-vincamine,
9-[3'-(bromo)-propionylamino]-apovincamine and
9-(cyclohexylcarbonylamino)-apovincamine and their optically active isomers.

12. A process of producing the eburnanecarboxylic acid derivatives according to claims 1 to 11 and/or of 11-(acetamido)-vincamine and/or of 9- and/or 11-(amino)-apovincamine,
characterized in that
a) racemic or optically active eburnanecarboxylic acid derivatives of the general formula

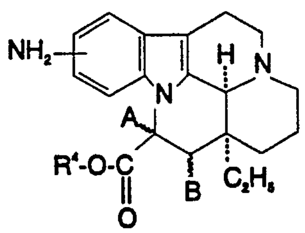

IV,

in which $R^4$ stands for a methyl residue or, if A and B together represent a chemical bond, may also represent hydrogen and the amino group is in position 9 or 11,
or their salts, optionally after a hydrolysis, are acylated except acetylation in the case that $R^4$ stands for a methyl residue, A represents a hydroxy group and B represents hydrogen, and
α) the resulting eburnanecarboxylic acid derivatives of the general formula I in which R stands for an acylamido residue of the general formula II or a sulphonylamido residue of the general formula III in which $R^1$ and $R^2$ have the meanings stated in claims 1 to 10, A and B have the meanings stated in claim 1 and $R^3$ has the meaning of $R^4$, or the salts thereof are optionally esterified or transesterified and/or
β) the resulting eburnanecarboxylic acid derivatives of the general formula I in which R stands for an acylamido residue of the general formula II or for a sulphonamido residue of the general formula III in which $R^1$ and $R^2$ have the meanings stated in claims 1 to 10, A stands for a hydroxy group, B represents hydrogen and $R^3$ is different from hydrogen, or the salts thereof are optionally hydrolyzed, and/or
γ) the resulting eburnanecarboxylic acid derivatives of the general formula I in which R and $R^3$ have the

meanings stated in claims 1 to 10, A stands for a hydroxy group and B represents hydrogen, or their salts are optionally dehydrated or

b) racemic or optically active eburnanecarboxylic acid derivatives having the general formula

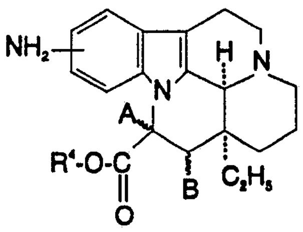

IV,

in which $R^4$ stands for a methyl residue or, if A and B together represent a chemical bond, may also represent hydrogen and the amino group is in position 9 or 11,

or their salts are esterified or transesterified and

α) the resulting eburnanecarboxylic acid derivatives of the general formula I, in which R stands for an amino group, A stands for a hydroxy group, B represents hydrogen and $R^3$ is different from hydrogen, or the salts thereof are optionally hydrolyzed, and/or

β) the resulting eburnanecarboxylic acid derivatives of the general formula I in which R and $R^3$ have the meanings stated in claims 1 to 10, A stands for a hydroxy group and B represents hydrogen, or their salts are optionally dehydrated and/or

γ) the resulting eburnanecarboxylic acid derivatives of the general formula I, in which R stands for an amino group and $R^3$, A and B have the meanings stated in claims 1 to 7, 9 or 10 or stands for an unsubstituted methyl residue, or their salts are optionally acylated or

c) racemic or optically active 9- or 11- (nitro)-eburnanecarboxylic acid derivatives of the general formula

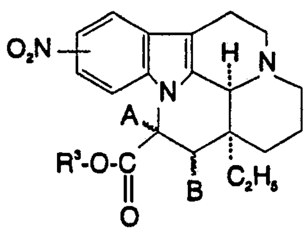

V,

in which $R^3$, A and B have the meanings stated in claims 1 to 7, 9 or 10 and the nitro group is in position 9 or 11 are reduced and the reactions according to a) α) and/or α) β) and/or b) α) and/or a) γ) and/or b) β) and/or b) γ) are optionally carried out and the resulting eburnanecarboxylic acid derivatives of the general formula I are optionally converted to acid addition salts or quaternary salts and/or the resulting salts of the eburnanecarboxylic acid derivatives of the general formula I are optionally converted to the free eburnanecarboxylic acid derivatives of the general formula I or to other acid addition salts and/or quaternary salts and/or the resulting racemic eburnanecarboxylic acid derivatives of the general formula I and/or their acid addition salts and/or quaternary salts are optionally split and/or the corresponding optically active compounds are optionally racemized to stereoisomers.

13. Medicaments, characterized by a content of 1 or more compound(s) according to claims 1 to 11 and/or 11-(acetamido)-vincamine as active ingredient(s), suitably together with 1 or more conventional carrier(s) and/or adjuvant(s).

**Claims** for the Contracting State: AT

1. A process of producing eburnanecarboxylic acid derivatives which have at position 9 or 11 an optionally substituted amino group and are of the general formula

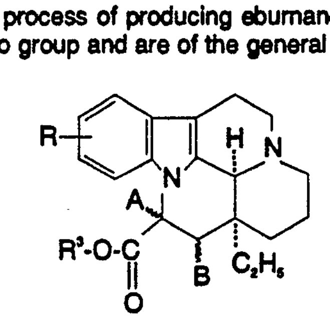

I,

wherein

R stands for an amino group or an acylamino residue of the general formula

```
  H  O
  |  ||
-N - C - R¹
```
II

in which $R^1$ is hydrogen or an aliphatic hydrocarbon residue having 1 to 10 carbon atom(s), a cycloaliphatic hydrocarbon residue having 3 to 8 carbon atoms, an aromatic hydrocarbon residue having 6 to 14 carbon atoms or a 5- to 7-membered heteroaryl residue having one or more identical or different heteroatoms consisting of nitrogen, oxygen and/or sulphur, and optionally having in addition to said members 4 members which have been fused on to form a benzene ring, wherein the above-mentioned residues are optionally substituted with one or more identical or different substituents consisting of halogen atoms, primary, secondary and tertiary amino groups, hydroxy groups, aryl residues having 6 to 14 carbon atoms, trifluoromethyl residues, carboxyl groups, alkoxy-carbonyl residues having 1 to 8 carbon atoms in the alkyl moiety, thio, sulphinyl, sulphonyl, nitro, keto, aldehyde and cyano groups and 1 or more 5- to 7-membered heteroaryl residues containing nitrogen, oxygen and/or sulphur as heteroatoms and having optionally in addition to said members 4 members which have been fused on to form a benzene ring, particularly pyrrolyl, furyl, thienyl, pyridyl, pyranyl, pyrazolyl, imidazolyl, pyrimidinyl, indolyl and quinolyl residues, which may optionally be substituted with 1 or more of the listed substituents, or a sulphonamido residue of the general formula

```
  H
  |
-N - S - R²
     O2
```
III

in which $R^2$ is an aliphatic hydrocarbon residue having 1 to 10 carbon atom(s) or an aromatic hydrocarbon residue having 6 to 14 carbon atoms, wherein said residues may be substituted with one or more of the substituents listed in connection with $R^1$,

$R^3$ is hydrogen or an aliphatic hydrocarbon residue having 1 to 10 carbon atom(s), a cycloaliphatic hydrocarbon residue having 3 to 8 carbon atoms or an aromatic hydrocarbon residue having 6 to 14 carbon atoms, which residues may be substituted with 1 or more identical or different ones of the substituents listed in connection with $R^1$,

A stands for a hydroxy group,

R means hydrogen or

A and B together represent a chemical bond and

R is in position 9 or 11,

provided that

a) if R stands for an amino group, then $R^3$ is different from an unsubstituted methyl residue and

b) if R stands for an acylamido residue which is in position 9 or 11 and has the general formula II, in which $R^1$ is a methyl residue,

A represents a hydroxy group and

B represents hydrogen, then

$R^3$ is different from an unsubstituted methyl residue,

and their optically active isomers and salts, characterised in that

a) racemic or optically active eburnanecarboxylic acid derivatives of the general formula

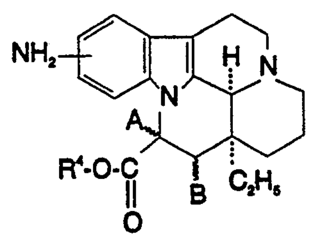

IV,

in which $R^4$ stands for a methyl residue or, if A and B together represent a chemical bond, may also represent hydrogen and the amino group is in position 9 or 11,

or their salts, optionally after a hydrolysis, are acylated except acetylation in the case that $R^4$ stands for a methyl residue, A represents a hydroxy group and B represents hydrogen, and

α) the resulting eburnanecarboxylic acid derivatives of the general formula I in which R stands for an acylamido residue of the general formula II or a sulphonylamido residue of the general formula III in which $R^1$ and $R^2$ have the meaning stated in claims 1 to 10, A and B have the meanings stated in claim 1 and $R^3$ has the meaning of $R^4$, or the salts thereof are optionally esterified or transesterified and/or

β) the resulting eburnanecarboxylic acid derivatives of the general formula I in which R stands for an acylamido residue of the general formula II or far a sulphonamido residue of the general formula III in which $R^1$

and $R^2$ have the meanings stated in claims 1 to 10, A stands for a hydroxy group, B represents hydrogen and $R^3$ is different from hydrogen, or the salts thereof are optionally hydrolyzed, and/or

γ) the resulting eburnanecarboxylic acid derivatives of the general formula I in which R and $R^3$ have the meanings stated in claims 1 to 10, A stands for a hydroxy group and B represents hydrogen, or their salts are optionally dehydrated or

b) racemic or optically active eburnanecarboxylic acid derivatives having the general formula

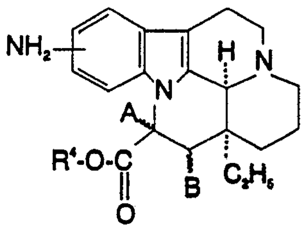

IV

in which $R^4$ stands for a methyl residue or, if A and B together represent a chemical bond, may also represent hydrogen and the amino group is in position 9 or 11,

or their salts are esterified or transesterified and

α) the resulting eburnanecarboxylic acid derivatives of the general formula I, in which R stands for an amino group,

A stands for a hydroxy group, a represents hydrogen and $R^3$ is different from hydrogen, or the salts thereof are optionally hydrolyzed, and/or

β) the resulting eburnanecarboxylic acid derivatives of the general formula I in which R and $R^3$ have the meanings stated in claims 1 to 10, A stands for a hydroxy group and a represents hydrogen, or their salts are optionally dehydrated and/or

γ) the resulting eburnanecarboxylic acid derivatives of the general formula I, in which R stands for an amino group and $R^3$, A and B have the meanings stated in claims 1 to 7, 9 or 10 or stands for an unsubstituted methyl residue, or their salts are optionally acylated or

c) racemic or optically active 9- or 11- (nitro)-eburnanecarboxylic acid derivatives of the general formula

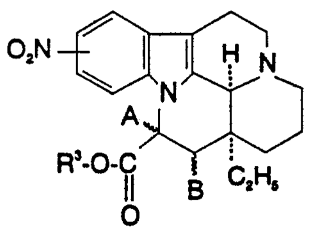

V,

in which $R^3$, A and B have the meanings stated in claims 1 to 7 9 or 10 and the nitro group is in position 9 or 11 are reduced and the reactions according to a) α) and/or α) β) and/or b) α) and/or a) γ) and/or b) β) and/or b) γ) are optionally carried out and the resulting eburnanecarboxylic acid derivatives of the general formula I are optionally converted to acid addition salts or quaternary salts and/or the resulting salts of the eburnanecarboxylic acid derivatives of the general formula I are optionally converted to the free eburnanecarboxylic acid derivatives of the general formula I or to other acid addition salts and/or quaternary salts and/or the resulting racemic eburnanecarboxylic acid derivatives of the general formula I and/or their acid addition salts and/or quaternary salts optionally split and/or the corresponding optically active compounds are optionally racemized to stereoisomers.

2. A process according to claim 1, characterized in that those eburnanecarboxylic acid derivatives of the general formula I are prepared in which the aliphatic hydrocarbon residue(s) which may be represented by $R^1$ or $R^2$ and/or $R^3$ is or are (an) alkyl residue(s) and/or (an) alkenyl and/or alkinyl residue(s).

3. A process according to claim 1 or 2, characterized in that those eburnanecarboxylic acid derivatives of the formula I are prepared in which the aliphatic hydrocarbon residue(s) which may be represented by $R^1$ or $R^2$ and/or $R^3$ is or are such residue(s) having 1 to 8 carbon atoms and the alkenyl and/or alkinyl residue(s) is or are such residue(s) having 2 to 4 carbon atoms.

4. A process according to claims 1 to 3, characterized in that those eburnanecarboxylic acid derivatives of the formula I are prepared in which the cycloaliphatic hydrocarbon residue(s) which may be represented by $R^1$ and/or $R^3$ is or are (a) cycloalkyl residue(s).

5. A process according to claims 1 to 3, characterized in that those eburnanecarboxylic acid derivatives of the formula I

are prepared in which the cycloalkyl residue(s) which may be represented by $R^1$ and/or $R^3$ is or are such residue(s) having 5 to 7 carbon atoms.

6. A process according to claims 1 to 5, characterized in that those eburnanecarboxylic acid derivatives of the formula I are prepared in which the aromatic hydrocarbon residue(s) which may be represented by $R^1$ or $R^2$ and/or $R^3$ is or are monocyclic, namely (a) phenyl residue(s), and/or is or are polycyclic and uncondensed and/or polycyclic and condensed.

7. A process according to claims 1 to 6, characterized in that those eburnanecarboxylic acid derivatives of the formula I are prepared in which one or more aromatic hydrocarbon radical(s) which may be represented by $R^1$ or $R^2$ and/or $R^3$ and which is or are polycyclic is or are (a) diphenyl residue(s) and/or naphthyl residue(s).

8. A process according to claims 1 to 7, characterized in that those eburnanecarboxylic acid derivatives of the formula I are prepared in which the heteroaryl residue which may be represented by $R^1$ is a pyrrolyl, furyl, thienyl, pyridyl, pyranyl, pyrazolyl, imidazolyl, pyriminyl, indolyl or quinolyle residue.

9. A process according to claims 1 to 8, characterized in that those eburnanecarboxylic acid derivatives of the formula I are prepared in which the substituent(s) with which the aliphatic and/or aromatic hydrocarbon residue(s) which may be represented by $R^1$ or $R^2$ and/or $R^3$ and/or the cyclo aliphatic residue(s) which may be represented by $R^1$ and/or $R^3$ and/or the heteroaryl residue which may be represented by $R^1$ is or are (a) fluorine, chlorine and/or bromine atom(s) or is or are (a) dialkylamino group(s) having 1 to 10 carbon atoms in each alkyl moiety if it is or they are (a) primary, secondary and/or tertiary amino group(s) or is or are (a) phenyl, diphenyl or naphthyl residue(s) if it is or they are (an) aryl residue(s) having 6 to 14 carbon atoms or is or (a) residue(s) having 1 to 4 carbon atom(s) if it is or they are (an) alkyl, alkoxy and/or alkylthio residue(s) having 1 to 8 carbon atoms each or a residue having in an alkyl moiety 1 to 4 carbon atom(s) if it is or they are (an) alkoxycarbonyl residue(s) having 1 to 8 carbon atoms in its alkyl moiety, and/or said residue(s) may consist of (a) heteroaryl residue(s) which contain(s) 1 or more identical or different heteroatom(s) consisting of nitrogen, oxygen and/or sulphur and in that case may be substituted with 1 or more of the listed substituents.

10. A process according to claims 1 to 9, characterized in that those eburnanecarboxylic acid derivatives of the formula I are prepared in which the heteroaryl residue(s) with which the aliphatic and/or aromatic hydrocarbon residue(s) which may be represented by $R^1$ or $R^2$ and/or $R^3$ and or the cycloaliphatic residue(s) which may be represented by $R^1$ and/or $R^3$ and/or the heteroaryl residue which may be represented by $R^1$ may be substituted is or are (a) pyrrolyl, furyl, thienyl, pyridyl, pyranyl, pyrazolyl, imidazolyl, pyrimidinyl, indolyl and/or quinolyl residue(s).

11. A process according to claim 1, characterized in that
11-(benzoylamino)-apovincamic acid,
9-(benzoylamino)-apovincamic acid,
11-(thiene-2'-ylcarbonylamino)-apovincamic acid,
11-(amino)-apovincamic acid ethyl ester,
9-(amino)-apovincamic acid ethyl ester and its hydrochloride,
11-(methylsulphonylamino)-apovincamic acid ethyl ester and its hydrochloride,
9-(methylsulphonylamino)-apovincamic acid ethyl ester and its hydrochloride,
11-(amino)-vincamic acid ethyl ester,
9-(amino)-vincamic acid ethyl ester,
11-[4'-(nitro)-benzoylamino]-apovincamic acid methyl ester,
9-[4'-(nitro)-benzoylamino]-apovincamic acid methyl ester,
11-[2'-(bromo)-benzoylamino-apovincamic acid methyl ester and its hydrochloride,
9-[2'-(bromo)-benzoylamino]-apovincamic acid methyl ester and its hydrochloride,
11-[2'-(fluoro)-benzoylamino]-vincamine,
9-[2'-(fluoro)-benzoylamino]-vincamine,
11-[3',4',5'-tri-(methoxy)-benzoylamino]-vincamine,
9-[3',4',5'-tri-(methoxy)-benzoylamino]-vincamine,
11-(pivaloylamino)-vincamine,
9-(pivaloylamino)-vincamine,
9-(benzoylamino)-vincamine,
11-(benzoylamino)-vincamine,
11-(thiene-2'-ylcarbonylamino)-vincamine,
9-(acetamido)-apovincamine,
11-(acetamido)-apovincamine,
9-(acetamido)-apovincamic acid ethyl ester and its hydrochloride,
11-(acetamido)-apovincamic acid ethyl ester,
9-(acetamido)-apovincamic acid isobutyl ester,
11-(acetamido)-apovincamic acid isobutyl ester and its hydrochloride,
11-[2',3',4'-tri-(methoxy)-benzoylamino]-apovincamine,

11-[3',4',5'-tri-(methoxy)-benzoylamino]-apovincamine,
9-[3',4',5'-tri-(methoxy)-benzoylamino]-apovincamine,
11-(pivaloylamino)-apovincamine,
9-(benzoylamino)-vincamic acid ethyl ester,
11-(benzoylamino)-vincamic acid ethyl ester,
9-(benzoylamino)-apovincamic acid ethyl ester,
9-(benzoylamino)
11-(benzoylamino)-apovincamic acid ethyl ester and its hydrochloride,
11-[acetamido]-vincamic acid-[2'-(hydroxy)-ethyl] ester,
9-[acetamido-vincamic acid-[2'-(hydroxy)-ethyl] ester,
11-(nicotinoylamino)-apovincamine,
9-(nicotinoylamino)-apovincamine,
11-[2'-(fluoro)-benzoylamino]-apovincamine,
9-[2'-(fluoro)-benzoylamino]-apovincamine,
11-(thiene-2'-ylcarbonylamino)-apovincamine,
9-(thiene-2'-ylcarbonylamino)-apovincamine,
9-(thiene-2'-ylcarbonylamino)-apovincamic acid ethyl ester,
11-(thiene-2'-ylcarbonylamino)-apovincamic acid and its hydrochloride,
11-(benzoylamino)-apovincamine,
9-(benzoylamino)-apovincamine,
11-[benzoylamino]-apovincamic acid-[2'-(hydroxy)-ethyl] ester,
9-[benzoylamino]-apovincamic acid-[2'-(hydroxy)-ethyl] ester,
11-[benzoylamino]-apovincamic acid-[2'-(methyl)-propyl] ester and its hydrochloride,
9-[benzoylamino]-apovincamic acid-[2'-(methyl)-propyl] ester and its hydrochloride,
11-(amino)-vincamic acid,
9-(amino)-vincamic acid,
11-[2'-(fluoro)-benzoylamino]-vincamic acid,
9-[2'-(fluoro)-benzoylamino]-vincamic acid,
11-(cyclohexylcarbonylamino)-vincamine,
11-[3'-(bromo)-propionylamino]-apovincamine,
11-(cyclohexylcarbonylamino)-apovincamine,
9-(pivaloylamino)-apovincamine,
9-(cyclohexylcarbonylamino)-vincamine,
9-[3'-(bromo)-propionylamino]-apovincamine and
9-(cyclohexylcarbonylamino)-apovincamine
and their optically active isomers are prepared.

**Revendications** pour les Etats Contractants: BE, CH, DE, FR, GB, IT, LI, NL, SE

1. Dérivés de l'acide éburnanecarboxylique ayant, sur la position 9 ou sur la position 11, un groupe amino qui est éventuellement substitué, de formule générale

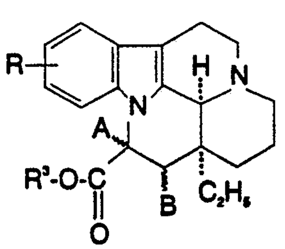

I,

dans laquelle R désigne un groupe amino ou un radical acylamido de formule générale

H O
| ||
-N-C-$R^1$

II,

dans cette dernière $R^1$ signifiant un atome d'hydrogène ou un radical hydrocarbure aliphatique ayant 1 à 10 atomes de carbone, un radical hydrocarbure cycloaliphatique ayant 3 à 8 atomes de carbone, un radical hydrocarbure aromatique ayant 6 à 14 atomes de carbone ou un radical hétéroaryle ayant 5 à 7 chaînons comportant un ou plusieurs hétéroatomes, identiques ou différents, constitués par l'azote, l'oxygène et/ou le soufre, et comportant éventuellement condensés d'une manière supplémentaire à ces chaînons, 4 chaînons pour la formation d'un noyau benzène, les radicaux précités pouvant être substitués par un ou plusieurs substituants identiques ou différents, constitués par des atomes

d'halogène, des groupes amino primaire, secondaire et tertiaire, des groupes hydroxy, des radicaux aryle ayant 6 à 14 atomes de carbone, des radicaux alcoyle, alcoxy et alcoylthio ayant 1 à 8 atomes de carbone, des radicaux trifluorométhyle, des radicaux carboxyle, des radicaux alcoxycarbonyle ayant 1 à 8 atomes de carbone dans la portion alcoyle, des groupes thio, sulfinyle, sulfonyle, nitro, céto, aldéhyde et cyano et des radicaux hétéroaryle ayant 5 à 7 chaînons comportant un ou plusieurs hétéroatomes constitués par l'azote, l'oxygène et/ou le soufre, comportant éventuellement condensés d'une manière supplémentaire à ces chaînons, 4 chaînons pour la formation d'un noyau benzène, surtout des radicaux pyrrolyle, furyle, thiényle, pyridyle, pyrannyle, pyrazolyle, imidazolyle, pyrimidinyle, indolyle et quinoléyle, qui peuvent être éventuellement substitués par un ou plusieurs des substituants indiqués,

ou un radical sulfonamido de formule générale

$$-\overset{\displaystyle H \atop |}{N}-\underset{O_2}{S}-R^2 \qquad \text{III},$$

dans cette dernière $R^2$ signifiant un radical hydrocarbure aliphatique ayant 1 à 10 atomes de carbone ou un radical hydrocarbure aromatique ayant 6 à 14 atomes de carbone, ces radicaux pouvant être substitués par un ou plusieurs substituants identiques ou différents, tels que mentionnés en rapport avec $R^1$,

$R^3$ signifie un atome d'hydrogène ou un radical hydrocarbure aliphatique ayant 1 à 10 atomes de carbone, un radical hydrocarbure cycloaliphatique ayant 3 à 8 atomes de carbone ou un radical hydrocarbure aromatique ayant 6 à 14 atomes de carbone, ces radicaux pouvant être substitués par un ou plusieurs substituants identiques ou différents tels qu'ils ont été définis en rapport avec $R^1$, et

A désigne un groupe hydroxyle, et

B signifie un atome d'hydrogène ou A et B représentent conjointement une liaison chimique, et

R est en position 9 ou 11,

avec les conditions supplémentaires que

a) dans le cas où R désigne un groupe amino, $R^3$ soit différent d'un radical méthyle non substitué, et

b) dans le cas où R désigne un radical acylamido, se trouvant en position 9 ou 11, de formule générale II, dans laquelle $R^1$ signifie un radical méthyle, A signifie un groupe hydroxy et B représente l'hydrogène, $R^3$ soit différent d'un radical méthyle non substitué, ainsi que leurs isomères optiquement actifs et leurs sels.

2. Dérivés de l'acide éburnanecarboxylique selon la revendication 1, caractérisés en ce que le ou les radicaux hydrocarbure aliphatique, que peut ou peuvent désigner $R^1$ ou $R^2$ et/ou $R^3$, est ou sont un ou des radicaux alcoyle et/ou un ou des radicaux alcényle et/ou alcynyle.

3. Dérivés de l'acide éburnanecarboxylique selon la revendication 1 ou 2, caractérisés en ce que le ou les radicaux hydrocarbure aliphatique, que peut ou peuvent désigner $R^1$ ou $R^2$ et/ou $R^3$, est ou sont un tel ou de tels radicaux ayant 1 à 8 atomes de carbone et le ou les radicaux alcényle et/ou alcynyle est ou sont un ou de tels radicaux ayant 2 à 4 atomes de carbone.

4. Dérivés de l'acide éburnanecarboxylique selon l'une des revendications 1 à 3, caractérisés en ce que le ou les radicaux hydrocarbure cycloaliphatique, que peut ou peuvent désigner $R^1$ et/ou $R^3$, est ou sont un ou des radicaux cycloalcoyle.

5. Dérivés de l'acide éburnanecarboxylique selon l'une des revendications 1 à 3, caractérisés en ce que le ou les radicaux cycloalcoyle, que peut ou peuvent désigner $R^1$ et/ou $R^3$, est ou sont un ou de tels radicaux ayant 5 à 7 atomes de carbone.

6. Dérivés de l'acide éburnanecarboxylique selon l'une des revendications 1 à 5, caractérisés en ce que le ou les radicaux hydrocarbure aromatique, que peut ou peuvent désigner $R^1$ ou $R^2$ et/ou $R^3$, est ou sont monocycliques, à savoir un ou des radicaux phényle, et/ou polycycliquement non condensés et/ou polycycliquement condensés.

7. Dérivés de l'acide éburnanecarboxylique selon l'une des revendications 1 à 6, caractérisés en ce que le ou les radicaux hydrocarbure aromatique, que peut ou peuvent désigner $R^1$ ou $R^2$ et/ou $R^3$, est ou sont, dans la mesure où il est ou ils sont polycycliques, un ou des radicaux diphényle et/ou un ou des radicaux naphtyle.

8. Dérivés de l'acide éburnanecarboxylique selon l'une des revendications 1 à 7, caractérisés en ce que le radical hétéroaryle, que peut représenter $R^1$, est un radical pyrrolyle, furyle, thiényle, pyridyle, pyrannyle, pyrazolyle, imidazolyle, pyrimidinyle, indolyle ou quinoléyle.

9. Dérivés de l'acide éburnanecarboxylique selon l'une des revendications 1 à 8, caractérisés en ce que le ou les substituants par lequel ou lesquels peut ou peuvent être substitués le ou les radicaux hydrocarbure aliphatique et/ou aromatique, que peut ou peuvent désigner $R^1$ ou $R^2$ et/ou $R^3$, ou le ou les radicaux cycloaliphatiques, que peut ou peuvent désigner $R^1$ et/ou $R^3$, ou le radical hétéroaryle, que peut désigner $R^1$, est ou sont, dans la mesure où il est ou ils sont un ou des atomes d'halogène, un ou des atomes de fluor, chlore et/ou brome, dans la mesure où il est ou ils

sont un ou des groupes amino primaire, secondaire et/ou tertiaire, un ou des groupes dialcoylamino ayant 1 à 10 atomes de carbone dans chaque portion alcoyle, dans la mesure où il est ou ils sont un ou des radicaux aryle ayant 6 à 14 atomes de carbone, un ou des radicaux phényle, diphényle ou naphtyle, dans la mesure où il est ou ils sont un ou des radicaux alcoyle, alcoxy et/ou alcoylthio ayant chacun 1 à 8 atomes de carbone, un ou de tels radicaux ayant 1 à 4 atomes de carbone, dans la mesure où il est ou ils sont un ou des radicaux alcoxycarbonyle ayant 1 à 8 atomes de carbone dans la portion alcoyle, un ou de tels radicaux ayant 1 à 4 atomes de carbone dans la portion alcoyle, et/ou, dans la mesure où il est ou ils sont un ou des radicaux hétéroaryle comportant un ou plusieurs hétéroatomes identiques ou différents constitués par l'azote, l'oxygène et/ou le soufre, un ou de tels radicaux qui peut ou peuvent être éventuellement substitués par un ou plusieurs des substituants mentionnés.

10. Dérivés de l'acide éburnanecarboxylique selon l'une des revendications 1 à 9, caractérisés en ce que le ou les radicaux hétéroaryle par lequel ou lesquels peut ou peuvent être substitués le ou les radicaux hydrocarbure aliphatique et/ou aromatique, que peut ou peuvent désigner $R^1$ ou $R^2$ et/ou $R^3$, ou le ou les radicaux cycloaliphatiques, que peut ou peuvent désigner $R^1$ et/ou $R^3$, ou le radical hétéroaryle, que peut désigner $R^1$, est ou sont un ou des radicaux pyrrolyle, furyle, thiényle, pyridyle, pyrannyle, pyrazolyle, imidazolyle, pyrimidinyle, indolyle et/ou quinoléyle.

11. Les dérivés de l'acide éburnanecarboxylique selon la revendication 1:
acide 11-(benzoylamino)-apovincaminique,
acide 9-(benzoylamino)-apovincaminique,
acide 11-(thién-2'-ylcarbonylamino)-apovincaminique,
11-(amino)-apovincaminoate d'éthyle,
9-(amino)-apovincaminoate d'éthyle et son chlorhydrate,
11-(méthylsulfonylamino)-apovincaminoate d'éthyle et son chlorhydrate,
9-(méthylsulfonylamino)-apovincaminoate d'éthyle et son chlorhydrate,
11-(amino)-vincaminoate d'éthyle,
9-(amino)-vincaminoate d'éthyle,
11-[4'-(nitro)-benzoylamino]-apovincaminoate de méthyle,
9-[4'-(nitro)-benzoylamino]-apovincaminoate de méthyle,
11-[2'-(bromo)-benzoylamino]-apovincaminoate de méthyle et son chlorhydrate,
9-[2'-(bromo)-benzoylamino]-apovincaminoate de méthyle et son chlorhydrate,
11-[2'-(fluoro)-benzoylamino]-vincamine,
11-[3',4',5'-tri-(méthoxy)-benzoylamino]-vincamine,
9-[3',4',5'-tri-(méthoxy)-benzoylamino]-vincamine,
11-(pivaloylamino)-vincamine,
9-(pivaloylamino)-vincamine,
9-benzoylamino-vincamine,
11-benzoylamino-vincamine,
11-(thién-2'-ylcarbonylamino)-vincamine,
9-(acétamido)-apovincamine,
11-(acétamido)-apovincamine,
9-(acétamido)-apovincaminoate d'éthyle et son chlorhydrate,
11-(acétamido)-apovincaminoate d'éthyle,
9-(acétamido)-apovincaminoate d'isobutyle,
11-(acétamido)-apovincaminoate d'isobutyle et son chlorhydrate,
11-[2',3',4'-tri-(méthoxy)-benzoylamino]-apovincamine,
11-[3',4',5'-tri-(méthoxy)-benzoylamino]-apovincamine,
9-[3',4',5'-tri-(méthoxy)-benzoylamino]-apovincamine,
11-(pivaloylamino)-apovincamine,
9-(benzoylamino)-vincaminoate d'éthyle,
11-(benzoylamino)-vincaminoate d'éthyle,
9-(benzoylamino)-apovincaminoate d'éthyle,
11-(benzoylamino)-apovincaminoate d'éthyle et son chlorhydrate,
11-[acétamido]-vincaminoate de 2'-(hydroxy)-éthyle,
9-[acétamido]-vincaminoate de 2'-(hydroxy)-éthyle,
11-(nicotinoylamino)-apovincamine,
9-(nicotinoylamino)-apovincamine,
11-[2'-(fluoro)-benzoylamino]-apovincamine,
9-[2'-(fluoro)-benzoylamino]-apovincamine,
11-(thién-2'-ylcarbonylamino)-apovincamine,
9-(thién-2'-ylcarbonylamino)-apovincamine,
9-(thién-2'-ylcarbonylamino)-apovincaminoate d'éthyle,
11-(thién-2'-ylcarbonylamino)-apovincaminoate d'éthyle et son chlorhydrate,
11-(benzoylamino)-apovincamine,
9-(benzoylamino)-apovincamine,
11-[benzoylamino]-apovincaminoate de 2'-(hydroxy)-éthyle,

9-[benzoylamino]-apovincaminoate de 2'-(hydroxy)-éthyle,
11-[benzoylamino]-apovincaminoate de 2'-(méthyl)-propyle et son chlorhydrate,
9-[benzoylamino]-apovincaminoate de 2'-(méthyl)-propyle et son chlorhydrate,
acide 11-(amino)-vincaminique,
acide 9-(amino)-vincaminique,
acide 11-[2'-(fluoro)-benzoylamino]-vincaminique,
acide 9-[2'-(fluoro)-benzoylamino]-vincaminique,
11-(cyclohexylcarbonylamino)-vincamine,
11-[3'-(bromo)-propionylamino]-apovincamine,
11-(cyclohexylcarbonylamino)-apovincamine,
9-(pivaloylamino)-apovincamine,
9-(cyclohexylcarbonylamino)-vincamine,
9-[3'-(bromo)-propionylamino]-apovincamine et
9-cyclohexylcarbonylamino)-apovincamine,
ainsi que leurs isomères optiquement actifs.

12. Procédé pour la préparation des dérivés de l'acide éburnanecarboxylique selon les revendications 1 à 11 et/ou de la 11-(acétamido)-vincamine et/ou de la 9- et/ou 11-(amino)-apovincamine, caractérisé ce que:

a) on acyle, éventuellement après une hydrolyse, les dérivés racémiques ou optiquement actifs de l'acide éburnanecarboxylique de formule générale

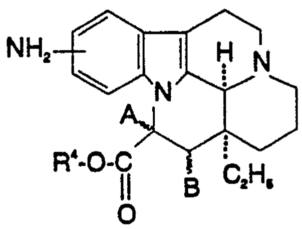

IV,

dans laquelle $R^4$ désigne un radical méthyle ou, dans le cas où A et B représentent conjointement une liaison chimique, peut également signifier un atome d'hydrogène, et le groupe amino se trouve en position 9 ou 11, ou leurs sels, excepté l'acétylation dans le cas où $R^4$ désigne un radical méthyle, A signifie un groupe hydroxy et B représente l'hydrogène, et α) éventuellement on estérifie ou transestérifie les dérivés de l'acide éburnanecarboxylique de formule générale I obtenus, dans lesquels R désigne un radical acylamido de formule générale II ou un radical sulfonylamido de formule générale III, $R^1$ et $R^2$ étant définis comme spécifié dans les revendications 1 à 10, A et B sont définis comme spécifié dans la revendication 1 et $R^3$ a la signification de $R^4$, et/ou leurs sels, et/ou

β) éventuellement on hydrolyse les dérivés de l'acide éburnanecarboxylique de formule générale I obtenus, dans lesquels R désigne un radical acylamido de formule générale II ou un radical sulfonylamido de formule générale III, $R^1$ et $R^2$ étant définis comme spécifié dans les revendications 1 à 10, A désigne un groupe hydroxy, B signifie de l'hydrogène et $R^3$ est différent de l'hydrogène, et/ou leurs sels, et/ou

γ) éventuellement on déshydrate les dérivés de l'acide éburnanecarboxylique de formule générale I obtenus, dans lesquels R et $R^3$ sont définis comme spécifié dans les revendications 1 à 10, A désigne un groupe hydroxy et B signifie l'hydrogène, et/ou leurs sels ou b) on estérifie ou transestérifie les dérivés racémiques ou optiquement actifs de l'acide éburnanecarboxylique de formule générale

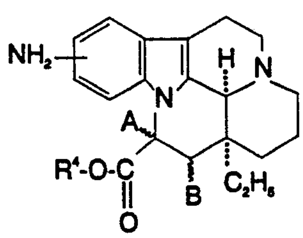

IV,

dans laquelle $R^4$ désigne un radical méthyle ou, dans le cas où A et B représentent conjointement une liaison chimique, peut également signifier un atome d'hydrogène, et le groupe amino se trouve en position 9 ou 11, ou leurs sels, et

α) éventuellement on hydrolyse les dérivés de l'acide éburnanecarboxylique de formule générale I obtenus, dans lesquels R désigne un groupe amino, A signifie un groupe hydroxy, B représente un atome d'hydrogène et $R^3$ est différent de l'hydrogène, et/ou leurs sels, et/ou

β) éventuellement on déshydrate les dérivés de l'acide éburnanecarboxylique de formule générale I obtenus, dans lesquels R et $R^3$ sont définis comme spécifié dans les revendications 1 à 10, A désigne un groupe hy-

droxy et B signifie l'hydrogène, et/ou leurs sels, et/ou

γ) éventuellement on acyle les dérivés de l'acide éburnanecarboxylique de formule générale I obtenus, dans les quels R désigne un groupe amino et $R^3$, A et B sont définis comme spécifié dans les revendications 1 à 7, 9 ou 10 ou désigne un radical méthyle non substituée et/ou leurs sels, ou

c) on réduit les dérivés racémiques ou optiquement actifs de l'acide 9- ou 11-(nitro)-éburnanecarboxylique de formule générale

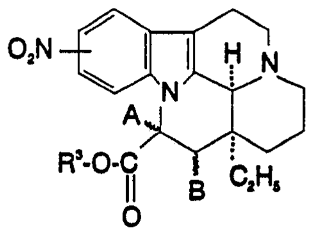

V,

dans laquelle $R^3$, A et B sont définis comme spécifié dans les revendications 1 à 7, 9 ou 10 et le groupe nitro est en position 9 ou 11, et éventuellement on effectue les réactions selon a) α) et/ou α) β) ou b) α) et/ou a) γ) ou b) β) et/ou b) γ), ainsi qu'éventuellement on convertit les dérivés de l'acide éburnanecarboxylique de formule générale I obtenus, en sels d'addition d'acides ou en sels quaternaires et/ou éventuellement on convertit les sels, obtenus, des dérivés de l'acide éburnanecarboxylique de formule générale I en dérivés de l'acide éburnanecarboxylique, libres, de formule générale I ou en d'autres sels d'addition d'acides ou en sels quaternaires, et/ou éventuellement on effectue un dédoublement des dérivés racémiques de l'acide éburnanecarboxylique de formule générale I obtenus ou de leurs sels d'addition d'acides ou sels quaternaires ou une racémisation des composés optiquement actifs correspondants en stéréo-isomères.

13. Médicaments, caractérisés par une teneur en un ou plusieurs composés selon les revendications 1 à 11 et/ou en 11-(acétamido)-vincamine en tant que substance active ou substances actives, convenablement conjointement avec un ou plusieurs véhicules et/au additifs courants.

**Revendications** pour l'Etat Contractant: AT

1. Procédé pour la préparation des dérivés de l'acide éburnanecarboxylique ayant, sur la position 9 ou sur la position 11, un groupe amino qui est éventuellement substitué, de formule générale

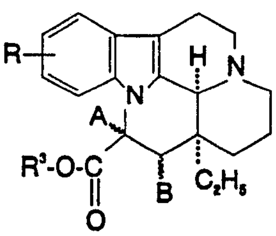

I,

dans laquelle R désigne un groupe amino ou un radical acylamido de formule générale

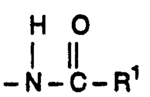

II,

dans cette dernière $R^1$ signifiant un atome d'hydrogène ou un radical hydrocarbure aliphatique ayant 1 à 10 atomes de carbone, un radical hydrocarbure cycloaliphatique ayant 3 à 8 atomes de carbone, un radical hydrocarbure aromatique ayant 6 à 14 atomes de carbone ou un radical hétéroaryle ayant 5 à 7 chaînons comportant un ou plusieurs hétéroatomes, identiques ou différents, constitués par l'azote, l'oxygène et/ou le soufre, et comportant éventuellement condensés d'une manière supplémentaire à ces chaînons, 4 chaînons pour la formation d'un noyau benzène, les radicaux précités pouvant être substitués par un ou plusieurs substituants identiques ou différents, constitués par des atomes d'halogène, des groupes amino primaire, secondaire et tertiaire, des groupes hydroxy, des radicaux aryle ayant 6 à 14 atomes de carbone, des radicaux alcoyle, alcoxy et alcoylthio ayant 1 à 8 atomes de carbone, des radicaux trifluorométhyle, des radicaux carboxyle, des radicaux alcoxycarbonyle ayant 1 à 8 atomes de carbone dans la portion alcoyle, des groupes thio, sulfinyle, sulfonyle, nitro, céto, aldéhyde et cyano et des radicaux hétéroaryle ayant 5 à 7 chaînons comportant un ou plusieurs hétéroatomes constitués par l'azote, l'oxygène et/ou le soufre, comportant éventuellement condensés d'une manière supplémentaire à ces chaînons, 4 chaînons pour la formation d'un noyau benzène,

surtout des radicaux pyrrolyle, furyle, thiényle, pyridyle, pyrannyle, pyrazolyle, imidazolyle, pyrimidinyle, indolyle et quinoléyle, qui peuvent être éventuellement substitués par un ou plusieurs des substituants indiqués,

ou un radical sulfonamido de formule générale

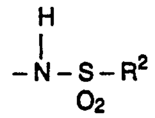

III,

dans cette dernière $R^2$ signifiant un radical hydrocarbure aliphatique ayant 1 à 10 atomes de carbone ou un radical hydrocarbure aromatique ayant 6 à 14 atomes de carbone, ces radicaux pouvant être substitués par un ou plusieurs substituants identiques ou différents, tels que mentionnés en rapport avec $R^1$,

$R^3$ signifie un atome d'hydrogène ou un radical hydrocarbure aliphatique ayant 1 à 10 atomes de carbone, un radical hydrocarbure cycloaliphatique ayant 3 à 8 atomes de carbone ou un radical hydrocarbure aromatique ayant 6 à 14 atomes de carbone, ces radicaux pouvant être substitués par un ou plusieurs substituants identiques ou différents tels qu'ils ont été définis en rapport avec $R^1$, et

A désigne un groupe hydroxyle, et

B signifie un atome d'hydrogène ou A et B représentent conjointement une liaison chimique, et

R est en position 9 ou 11,

avec les conditions supplémentaires que

a) dans le cas où R désigne un groupe amino, $R^3$ soit différent d'un radical méthyle non substitué, et

b) dans le cas où R désigne un radical acylamido, se trouvant en position 9 ou 11, de formule générale II, dans laquelle $R^1$ signifie un radical méthyle, A signifie un groupe hydroxy et B représente l'hydrogène, $R^3$ soit différent d'un radical méthyle non substitué, ainsi que leurs isomères optiquement actifs et leurs sels,

caractérisé en ce que:

a) on acyle, éventuellement après une hydrolyse, les dérivés racémiques ou optiquement actifs de l'acide éburnanecarboxylique de formule générale

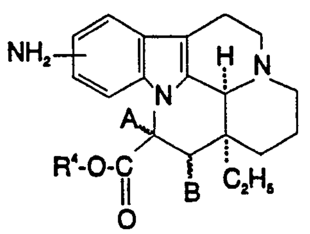

IV,

dans laquelle $R^4$ désigne un radical méthyle ou, dans le cas où A et B représentent conjointement une liaison chimique, peut également signifier un atome d'hydrogène, et le groupe amino se trouve en position 9 ou 11, ou leurs sels, excepté l'acétylation dans le cas où $R^4$ désigne un radical méthyle, A signifie un groupe hydroxy et B représente l'hydrogène, et

α) éventuellement on estérifie ou transestérifie les dérivés de l'acide éburnanecarboxylique de formule générale I obtenus, dans lesquels R désigne un radical acylamido de formule générale II ou un radical sulfonylamido de formule générale III, $R^1$ et $R^2$ étant définis comme spécifié dans les revendications 1 à 10, A et B sont définis comme spécifié dans la revendication 1 et $R^3$ a la signification de $R^4$ et/ou leurs sels, et/ou

β) éventuellement on hydrolyse les dérivés de l'acide éburnanecarboxylique de formule générale I obtenus, dans lesquels R désigne un radical acylamido de formule générale II ou un radical sulfonylamido de formule générale III, $R^1$ et $R^2$ étant définis comme spécifié dans les revendications 1 à 10, A désigne un groupe hydroxy, B signifie de l'hydrogène et $R^3$ est différent de l'hydrogène, et/ou leurs sels, et/ou

γ) éventuellement on déshydrate les dérivés de l'acide éburnanecarboxylique de formule générale I obtenus, dans lesquels R et $R^3$ sont définis comme spécifié dans les revendications 1 à 10, A désigne un groupe hydroxy et B signifie l'hydrogène, et/ou leurs sels ou

b) on estérifie ou transestérifie les dérivés racémiques ou optiquement actifs de l'acide éburnanecarboxylique de formule générale

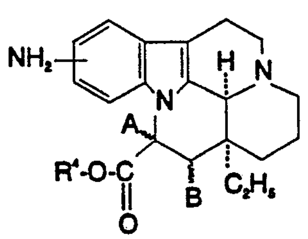

IV

dans laquelle $R^4$ désigne un radical méthyle ou, dans le cas où A et B représentent conjointement une liaison chimique, peut également signifier un atome d'hydrogène, et le groupe amino se trouve en position 9 ou 11, ou leurs sels, et

β) éventuellement on hydrolyse les dérivés de l'acide éburnanecarboxylique de formule générale I obtenus, dans lesquels R désigne un groupe amino, A signifie un groupe hydroxy, B représente un atome d'hydrogène et $R^3$ est différent de l'hydrogène, et/ou leurs sels, et/ou

β) éventuellement on déshydrate les dérivés de l'acide éburnanecarboxylique de formule générale I obtenus, dans lesquels R et $R^3$ sont définis comme spécifié dans les revendications 1 à 10, A désigne un groupe hydroxy et B signifie l'hydrogène, et/ou leurs sels, et/ou

γ) éventuellement on acyle les dérivés de l'acide éburnanecarboxylique de formule générale I obtenus, dans lesquels R désigne un groupe amino et $R^3$, A et B sont définis comme spécifié dans les revendications 1 à 7, 9 ou 10 ou désigne un radical méthyle non substitué, et/ou leurs sels, ou

c) on réduit les dérivés racémiques ou optiquement actifs de l'acide 9- ou 11-(nitro)-éburnanecarboxylique de formule générale

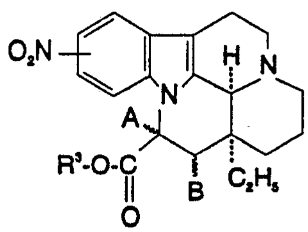

V

dans laquelle $R^3$, A et B sont définis comme spécifié dans les revendications 1 à 7, 9 ou 10 et le groupe nitro est en position 9 ou 11, et éventuellement on effectue les réactions selon a) α) et/ou α) β) ou b) α) et/ou a) γ) ou b) β) et/ou b) γ), ainsi qu'éventuellement on convertit les dérivés de l'acide éburnanecarboxylique de formule générale I obtenus, en sels d'addition d'acides ou en sels quaternaires et/ou éventuellement on convertit les sels, obtenus, des dérives de l'acide éburnanecarboxylique de formule générale I en dérivés de l'acide éburnanecarboxylique, libres, de formule générale I ou en d'autres sels d'addition d'acides ou en sels quaternaires, et/ou éventuellement on effectue un dédoublement des dérivés racémiques de l'acide éburnanecarboxylique de formule générale I obtenus ou de leurs sels d'addition d'acides ou sels quaternaires ou une racémisation des composés optiquement actifs correspondants en stéréo-isomères.

2. Procédé selon la revendication 1, caractérisé en ce que les dérivés de l'acide éburnanecarboxylique de formule générale I sont préparés dans lesquelles le ou les radicaux hydrocarbure aliphatique, que peut ou peuvent désigner $R^1$ ou $R^2$ et/ou $R^3$, est ou sont un ou des radicaux alcoyle et/ou un ou des radicaux alcényle et/ou alcynyle.

3. Procédé selon la revendication 1 ou 2, caractérisé en ce que les dérivés de l'acide éburnanecarboxylique de formule générale I sont préparés dans lesquelles le ou les radicaux hydrocarbure aliphatique, que peut ou peuvent désigner $R^1$ ou $R^2$ et/ou $R^3$, est ou sont un tel ou de tels radicaux ayant 1 à 8 atomes de carbone et le ou les radicaux alcényle et/ou alcynyle est ou sont un ou de tels radicaux ayant 2 à 4 atomes de carbone.

4. Procédé selon les revendications 1 à 3, caractérisé en ce que les dérivés de l'acide éburnanecarboxylique de formule générale I sont préparés dans lesquelles le ou les radicaux hydrocarbure cycloaliphatique, que peut ou peuvent désigner $R^1$ et/ou $R^3$, est ou sont un ou des radicaux cycloalcoyle.

5. Procédé selon les revendications 1 à 3, caractérisé en ce que les dérivés de l'acide éburnanecarboxylique de formule générale I sont préparés dans lesquelles le ou les radicaux cycloalcoyle, que peut ou peuvent désigner $R^1$ et/ou $R^3$, est ou sont un ou de tels radicaux ayant 5 à 7 atomes de carbone.

6. Procédé selon les revendications 1 à 5, caractérisé en ce que les dérivés de l'acide éburnanecarboxylique de formule générale I sont préparés dans lesquelles le ou les radicaux hydrocarbure aromatique, que peut ou peuvent désigner $R^1$ ou $R^2$ et/ou $R^3$, est ou sont monocycliques, à savoir un ou des radicaux phényle, et/ou polycycliquement non condensés et/ou polycycliquement condensés.

7. Procédé selon les revendications 1 à 6, caractérisé en ce que les dérivés de l'acide éburnanecarboxylique de formule générale I sont préparés dans lesquelles le ou les radicaux hydrocarbure aromatique, que peut ou peuvent désigner $R^1$ ou $R^2$ et/ou $R^3$, est ou sont, dans la mesure où il est ou ils sont polycycliques, un ou des radicaux diphényle et/ou un ou des radicaux naphtyle.

8. Procédé selon les revendications 1 à 7, caractérisé en ce que les dérivés de l'acide éburnanecarboxylique de formule générale I sont préparés dans lesquelles le radical hétéroaryle, que peut représenter $R^1$, est un radical pyrrolyle, furyle, thiényle, pyridyle, pyrannyle, pyrazolyle, imidazolyle, pyrimidinyle, indolyle ou quinoléyle.

9. Procédé selon les revendications 1 à 8, caractérisé en ce que les dérivés de l'acide éburnanecarboxylique de formule générale I sont préparés dans lesquelles le ou les substituants par lequel ou lesquels peut ou peuvent être substitués le ou les radicaux hydrocarbure aliphatique et/ou aromatique, que peut ou peuvent désigner $R^1$ ou $R^2$ et/ou $R^3$, ou le ou les radicaux cycloaliphatiques, que peut ou peuvent désigner $R^1$ et/ou $R^3$, ou le radical hétéroaryle, que peut désigner $R^1$, est ou sont, dans la mesure où il est ou ils sont un ou des atomes d'halogène, un ou des atomes de fluor, chlore et/ou brome, dans la mesure où il est ou ils sont un ou des groupes amino primaire, secondaire et/ou tertiaire, un ou des groupes dialcoylamino ayant 1 à 10 atomes de carbone dans chaque portion alcoyle, dans la mesure où il est ou ils sont un ou des radicaux aryle ayant 6 à 14 atomes de carbone, un ou des radicaux phényle, diphényle ou naphtyle, dans la mesure où il est ou ils sont un ou des radicaux alcoyle, alcoxy et/ou alcoylthio ayant chacun 1 à 8 atomes de carbone, un ou de tels radicaux ayant 1 à 4 atomes de carbone, dans la mesure où il est ou ils sont un ou des radical alcoxycarbonyle ayant 1 à 8 atomes de carbone dans la portion alcoyle, un ou de tels radicaux ayant 1 à 4 atomes de carbone dans la portion alcoyle, et/ou, dans la mesure où il est ou ils sont un ou des radicaux hétéroaryle comportant un ou plusieurs hétéroatomes identiques ou différents constitués par l'azote, l'oxygène et/ou le soufre, un ou de tels radicaux qui peut ou peuvent être éventuellement substitués par un ou plusieurs des substituants mentionnés.

10. Procédé selon les revendications 1 à 9, caractérisé en ce que les dérivés de l'acide éburnanecarboxylique de formule générale I sont préparés dans lesquelles le ou les radicaux hétéroaryle par lequel ou lesquels peut ou peuvent être substitués le ou les radicaux hydrocarbure aliphatique et/ou aromatique, que peut ou peuvent désigner $R^1$ ou $R^2$ et/ou $R^3$, ou le ou les radicaux cycloaliphatiques, que peut ou peuvent désigner $R^1$ et/ou $R^3$, ou le radical hétéroaryle, que peut désigner $R^1$, est ou sont un ou des radicaux pyrrolyle, furyle, thiényle, pyridyle, pyrannyle, pyrazolyle, imidazolyle, pyrimidinyle, indolyle et/ou quinoléyle.

11. Procédé selon la revendication 1 caractérisé en ce que les dérivés de
l'acide 11-(benzoylamino)-apovincaminique,
acide 9-(benzoylamino)-apovincaminique,
acide 11-(thién-2'-ylcarbonylamino)-apovincaminique,
11-(amino)-apovincaminoate d'éthyle,
9-(amino)-apovincaminoate d'éthyle et son chlorhydrate,
11-(méthylsulfonylamino)-apovincaminoate d'éthyle et son chlorhydrate,
9-(méthylsulfonylamino)-apovincaminoate d'éthyle et son chlorhydrate,
11-(amino)-vincaminoate d'éthyle,
9-(amino)-vincaminoate d'éthyle,
11-[4'-(nitro)-benzoylamino]-apovincaminoate de méthyle,
9-[4'-(nitro)-benzoylamino]-apovincaminoate de méthyle,
11-[2'-(bromo)-benzoylamino]-apovincaminoate de méthyle et son chlorhydrate,
9-[2'-(bromo)-benzoylamino]-apovincaminoate de méthyle et son chlorhydrate,
11-[2'-(fluoro)-benzoylamino]-vincamine,
11-[3',4',5'-tri-(méthoxy)-benzoylamino]-vincamine,
9-[3',4',5'-tri-(méthoxy)-benzoylamino]-vincamine,
11-(pivaloylamino)-vincamine,
9-(pivaloylamino)-vincamine,
9-benzoylamino-vincamine,
11-benzoylamino-vincamine,
11-(thién-2'-ylcarbonylamino)-vincamine,
9-(acétamido)-apovincamine,
11-(acétamido)-apovincamine,
9-(acétamido)-apovincaminoate d'éthyle et son chlorhydrate,
11-(acétamido)-apovincaminoate d'éthyle,
9-(acétamido)-apovincaminoate d'isobutyle,
11-(acétamido)-apovincaminoate d'isobutyle et son chlorhydrate,
11-[2',3',4'-tri-(méthoxy)-benzoylamino]-apovincamine,
11-[3',4',5'-tri-(méthoxy)-benzoylamino]-apovincamine,
9-[3',4',5'-tri-(méthoxy)-benzoylamino]-apovincamine,
11-(pivaloylamino)-apovincamine,
9-(benzoylamino)-vincaminoate d'éthyle,
11-(benzoylamino)-vincaminoate d'éthyle,
9-(benzoylamino)-apovincaminoate d'éthyle,
11-(benzoylamino)-apovincaminoate d'éthyle et son chlorhydrate,
11-[acétamido]-vincaminoate de 2'-(hydroxy)-éthyle,
9-[acétamido]-vincaminoate de 2'-(hydroxy)-éthyle,
11-(nicotinoylamino)-apovincamine,
9-(nicotinoylamino)-apovincamine,
11-[2'-(fluoro)-benzoylamino]-apovincamine,
9-[2'-(fluoro)-benzoylamino]-apovincamine,

11-(thién-2'-ylcarbonylamino)-apovincamine,
9-(thién-2'-ylcarbonylamino)-apovincamine,
9-(thién-2'-ylcarbonylamino)-apovincaminoate d'éthyle,
11-(thién-2'-ylcarbonylamino)-apovincaminoate d'éthyle et son chlorhydrate,
11-(benzoylamino)-apovincamine,
9-(benzoylamino)-apovincamine,
11-[benzoylamino]-apovincaminoate de 2'-(hydroxy)-éthyle,
9-[benzoylamino]-apovincaminoate de 2'-(hydroxy)-éthyle,
11-[benzoylamino]-apovincaminoate de 2'-(méthyl)-propyle et son chlorhydrate,
9-[benzoylamino]-apovincaminoate de 2'-(méthyl)-propyle et son chlorhydrate,
acide 11-(amino)-vincaminique,
acide 9-(amino)-vincaminique,
acide 11-[2'-(fluoro)-benzoylamino]-vincaminique,
acide 9-[2'-(fluoro)-benzoylamino]-vincaminique,
11-(cyclohexylcarbonylamino)-vincamine,
11-[3'-(bromo)-propionylamino]-apovincamine,
11-(cyclohexylcarbonylamino)-apovincamine,
9-(pivaloylamino)-apovincamine,
9-(cyclohexylcarbonylamino)-vincamine,
9-[3'-(bromo)-propionylamino]-apovincamine et
9-cyclohexylcarbonylamino)-apovincamine,
ainsi que leurs isomères optiquement actifs sont préparés.